# EUROPEAN PATENT APPLICATION

(11) **EP 4 596 534 A1**
(43) Date of publication of application: **06.08.2025**
(21) Application number: 24154998.9
(22) Date of filing: 31.01.2024
(51) Int. Cl.: C07D 209/44, C07D 217/08, C07D 401/12, C07D 405/12, C07D 409/12, A61P 17/06, A61P 39/06, A61K 31/472, A61K 31/4725, A61P 25/28, A61P 29/00, A61P 35/00

(54) **NONCOVALENT SMALL-MOLECULE INHIBITORS OF THE KEAP1-NRF2 INTERACTION**

(71) Applicant: University of Copenhagen, 1165 Copenhagen K (DK); Danmarks Tekniske Universitet, 2800 Kongens Lyngby (DK)
(72) Inventor: Qin, Yuting, 1165 Copenhagen K (DK); Tran, Kim Tai, 2800 Kongens Lyngby (DK); Narayanan, Dilip, 1165 Copenhagen K (DK); Mukminova, Elina, 1165 Copenhagen K (DK); Bach, Anders, 1165 Copenhagen K (DK)
(74) Representative: Høiberg P/S

(57) **Abstract**

The present invention relates to noncovalent Keap1-Nrf2 small-molecule inhibitors and their use as medicaments .

## Description

### Technical field

The present invention relates to noncovalent Keap1-Nrf2 small-molecule inhibitors and their use as medicaments.

### Background

Reactive oxygen species (ROS) generated in low levels during cellular homeostasis are used for redox signalling events and controlled by endogenous antioxidants and neutralizing enzymes. Several disease conditions, however, lead to ROS amounts exceeding the capacity of these endogenous defence molecules, which can result in oxidative stress and inflammation.^{1.2}

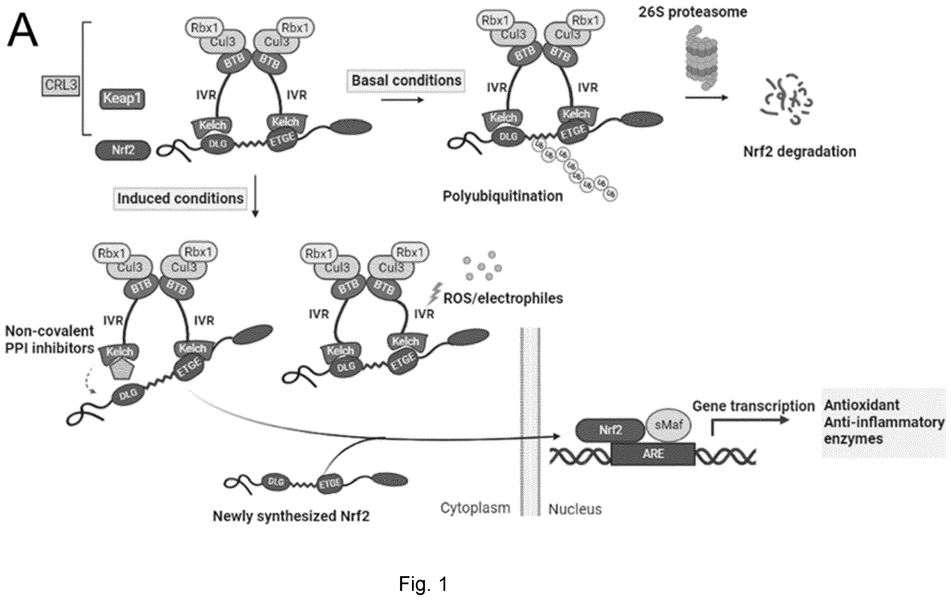

The protein-protein interaction (PPI) between the transcription factor nuclear erythroid-related factor 2 (Nrf2) and the Kelch domain of Kelch-like ECH-associated protein 1 (Keap1) is central in the cellular adaptive response to fluctuating levels of ROS and exogenous electrophiles. Keap1 is a substrate adaptor protein and component of the cullin 3 E3 ubiquitin ligase complex that recognizes and ubiquitinates Nrf2, marking it for proteasomal degradation.³ However, when ROS levels increase, key sensor cysteine residues on the broad complex, tramtrack, and bric-à-brac (BTB) domain and intervening region (IVR) of Keap1 are modified, which leads to a conformational change in Keap1 that prevents Cullin 3-mediated ubiquitination.^{4, 5} This results in cytosolic accumulation of Nrf2 followed by translocation into the nucleus, where Nrf2 forms a transcription factor complex that induces expression of detoxifying antioxidant enzymes, such as NAD(P)H:quinone oxidoreductase 1 (NQO1), heme oxygenase 1 (HO-1), and glutathione reductase, and suppresses pro-inflammatory genes (**Figure 1A**).⁶

Pharmacological inhibition of the Keap1-Nrf2 PPI has emerged as a promising therapeutic strategy to alleviate oxidative stress and reduce inflammation in many diseases,⁶⁻⁹ for example CNS injuries (cerebral ischemia,¹⁰ intracerebral haemorrhage,¹¹ ischemic stroke,¹²⁻¹⁴ and traumatic brain injury¹⁵), retinal ischemia-reperfusion (I/R) injury,¹⁶ neurodegenerative disorders (e.g. Alzheimer's disease,^{17, 18} Parkinson's disease, and multiple sclerosis),^{6, 19} lung diseases (e.g. chronic obstructive pulmonary disease,²⁰ pulmonary hypertension,⁶ and acute lung inflammation^{21, 22}), metabolic and and/or inflammatory liver conditions (such as non-alcoholic steatohepatitis, NASH)^{23, 24}, chronic kidney disease (CKD;²⁵ including Alport syndrome,^{6, 26}) skin diseases (e.g. atopic dermatitis and psoriasis²⁷), rheumatoid arthritis,²⁸ ulcerative colitis,²⁹ acetaminophen-induced hepatotoxicity,³⁰ and some cancer types.⁶ Thus, Keap1 is a promising drug target for a diverse set of pathologies.

Electrophilic Keap1-Nrf2 inhibitors, which covalently bind the sensor cysteines of Keap1, have been thoroughly explored and are often effective in enhancing the antioxidant defence response.⁶ A prominent example is dimethyl fumarate,³¹ which is clinically used against multiple sclerosis and psoriasis. However, unspecific reactivity of covalent binders can lead to toxicity and uncertainties about the mode of action.^{32, 33} Thus, noncovalent reversible Keap1-Nrf2 PPI inhibitors comprise an alternative and more attractive strategy. These bind the Keap1 Kelch domain and displace Nrf2 either partly or fully from Keap1, hence allowing Nrf2 to escape proteasomal degradation and initiate the expression Nrf2-controlled genes.^{4,5} Such inhibitors could provide specific, less toxic, and potent chemical probes and drug leads.^{4, 6, 7, 34}

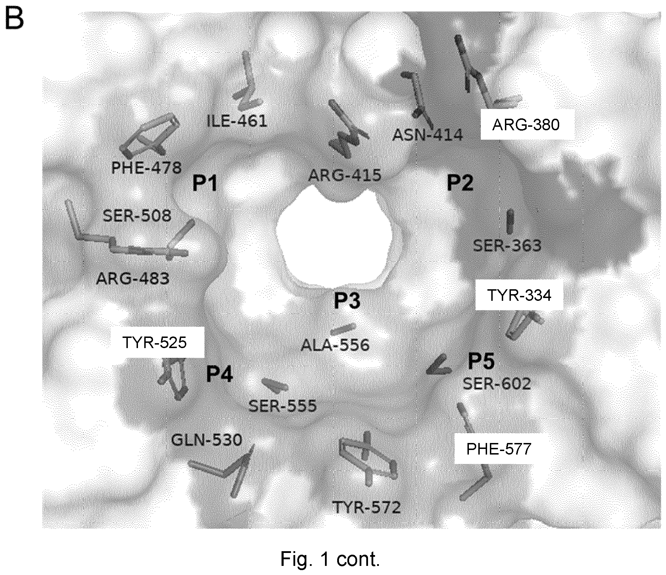

Today, there exist several small-molecule noncovalent reversible Keap1-Nrf2 inhibitors. However, they often have suboptimal properties, such as low specificity, low affinity, low solubility, mutagenic activity, low metabolic stability, low membrane permeability, or low bioavailability, and/or high clearance.³⁵⁻³⁷ Also, their size (molecular weight and total polar surface area), number of hydrogen bond donors, and inclusion of carboxylic acids prevent brain permeability and thus limit their use as chemical probes for investigating CNS diseases.⁴ These properties are likely a result of the nature of the Keap1 Kelch binding pocket, as, to obtain high affinity, it appears that both the polar arginine-containing subpockets (P1 and/or P2) and the hydrophobic subpockets (P4 and/or P5) need to be occupied (**Figure 1B**), generally leading to large and charged compounds.^{4,} 6, 38

Fragment-based drug discovery (FBDD) has in recent years become a state-of-the-art strategy for the development of high-quality chemical probes and clinical candidates.^{39, 40} FBDD has been used to make three series of noncovalent Keap1-Nrf2 inhibitors.^{20, 36, 41} The principle of FBDD is to screen for small substructures (fragments) of drug-like compounds with molecular weights of 100-300 Daltons and few functional and hydrogen-bonding groups and use these as starting points for further optimization.⁴² This contrasts with conventional high-throughput screening (HTS) where larger molecules of 250-600 Daltons are tested. The advantage of FBDD is that small fragments can fit easier into protein binding pockets, whereby they contain higher binding energies relative to their size, compared to a typical and larger HTS hit that often form low-quality interactions and therefore is difficult to optimize. Chemical growing or linking of the fragment hits, preferably guided by X-ray crystallographic data, can convert the fragments into highly optimized and potent molecules. By making sure that the molecule does not grow excessively large or lipophilic during the fragment-to-lead optimization process, molecules with more drug-like properties (e.g. good solubility, metabolic stability, membrane permeability, absorption, pharmacokinetic properties etc.) are achieved.

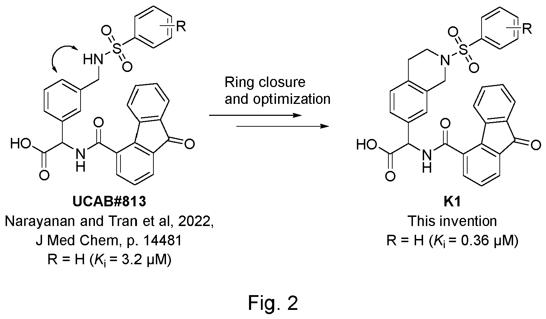

Previously,³⁷ we screened 2500 fragments using orthogonal assays - fluorescence polarization (FP), thermal shift assay (TSA), and surface plasmon resonance (SPR) - and validated the hits by saturation transfer difference (STD) NMR leading to 28 high-priority hits. Thirteen co-structures showed fragments binding mainly in the P4 and P5 subpockets of Keap1's Kelch domain. Three fluorenone-based fragments featuring a novel binding mode were optimized by structure-based drug discovery, which resulted in high-affinity (*K*ᵢ > 280 nM), and cell-active noncovalent small-molecule Keap1-Nrf2 PPI inhibitors (**Figure 2**).³⁷

### Summary

Keap1 is a promising drug target for a diverse set of pathologies, where oxidative stress and inflammation play key roles. The present invention entails a series of novel noncovalent small-molecule inhibitors of the Keap1-Nrf2 interaction developed by a conformational restriction strategy of the fluorenone-based compounds previously identified by FBDD. From the previous non-cyclic compound **UCAB**#813, a new series of cyclic tetrahydroisoquinoline (THIQ)-based Keap1 inhibitors was developed leading to an initial 10-fold improved affinity (**Figure 2**). Further optimization gave several novel compounds able to inhibit the Keap1-Nrf2 interaction with very high affinity (*K*ᵢ > 13 nM).

In one main aspect, the present disclosure relates to a compound of formula (I): 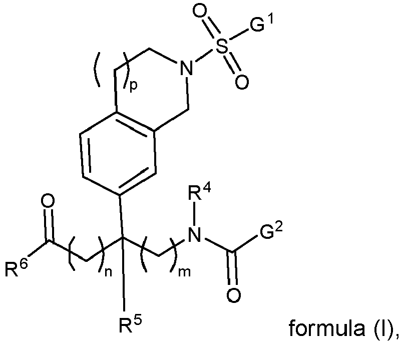 or a pharmaceutically acceptable salt thereof, wherein
G¹ is according to 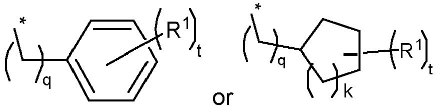
R' is in each instance individually selected from -H, -F, -Cl, -Br, -I, -CN, -O-(C₁-C₃ alkyl), -CF₃, -OCF₃, -N(R^{1a})(R^{1b}), C₁-C₃ alkyl, -C(=O)O-(C₁-C₃ alkyl), or two R' groups are linked together to form a carbocycle or heterocycle;
R^{1a} and R^{1b} are each independently a C₁-C₃ alkyl;
G² is according to 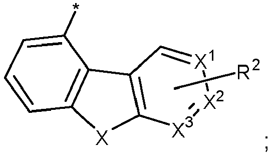
R² is H, -F, -Cl, -CH₃, or -O-CH₃;
X¹, X², X³ are each individually selected from CH, or N; provided that at most only one of X¹, X² and X³ is N;
X is O, S, 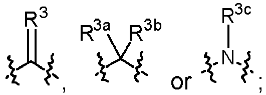
R³ is (=O), or (=S);
R^{3a} is H, halogen, -O-(C₁-C₃ alkyl), -O-CF₃, -S-(C₁ alkyl) or -C₁-C₃ alkyl; R^{3b} is H,
-OH, -O-(C₁-C₃ alkyl), -O-CF₃, halogen, -S-(C₁ alkyl), or -C₁-C₃ alkyl; or R^{3a} and R^{3b} are linked together to form a carbocycle or heterocycle;
R^{3c} is H, C₁-C₃ alkyl, or -CF₃;
R⁴ is H, or C₁ alkyl;
R⁵ is H, or F;
R⁶ is -OH, -O-C₁-C₁₀ alkyl, -O-C₃-C₁₀ cycloalkyl, a heterocycle, or a -O-C₅-C₁₂ aryl;
n, m, p, and q are integers each individually and independently selected from 0 or 1;
k is an integer selected from 0 to 2
t is an integer selected from 0 to 4.

As shown in the examples, compounds according to formula (I) inhibit the interaction between Nrf2 and Keap1.

In another aspect, the present disclosure provides for a composition comprising a compound according as describe herein, or a pharmaceutically acceptable salt thereof, and a pharmaceutically acceptable carrier or excipient.

In yet another aspect, the present invention relates to a compound or composition as described herein for use as a medicament.

### Description of Drawings

Figure 1: The Keap1-Nrf2 PPI.
   A) Molecular mechanisms of the Keap1-Nrf2 pathway showing the effect of ROS, electrophiles and non-covalent PPI inhibitors.
   B) X-ray crystal structure of the Neh2-binding pocket (P1-P5) of the Keap1 Kelch domain (modified from PDB ID 6ZF8). The five subpockets are labelled P1-5.
Figure 2: The conformational restriction design strategy leading to the invention.
Figure 3: X-ray crystal structure of compound K2 binding in the Neh2-binding pocket of the Keap1 Kelch domain.

### Definitions

The term "alkyl" as used herein refers to a linear or branched hydrocarbon moiety wherein hydrogen atom has been removed. For example, a C₁-C₅ alkyl refers to a linear or branched hydrocarbon moiety having 1 to 5 carbons. Examples of alkyl groups are for example but not limited to methyl, ethyl, propyl, butyl, pentyl, propyl, isopropyl, tert-butyl, sec-butyl, etc.

As used herein the term "cycloalkyl" or "carbocycle" refers to a monocyclic or polycyclic system. For example, a C₃-C₁₀ cycloalkyl or carbocycle, refers to a monocyclic or polycyclic system having a total of 3 to 10 ring atoms. Examples of cycloalkyl or carbocycles are for example but not limited to cyclopropyl, cyclobutyl, cyclopentyl, cyclohexyl, cycloheptyl etc. The carbocycles or cycloalkyl groups used herein can optionally contain one or more unsaturations or substituents, for example but not limited to 1-cyclobutenyl, 1-cyclopentenyl, 1,3-cyclopentadienly, 1-methylbutyl, 1-methylpentyl, 1-isopropylcyclobutyl, etc.

The term "heterocycle", refers to a monocyclic or polycyclic system, as defined for carbocycles, wherein at least one of the ring atoms are heteroatom(s). For example, a heterocycle may be a thiazolidinone.

As used herein "heteroatom", particularly as a ring heteroatom, refers to sulfur, oxygen and nitrogen.

The terms "substituents" or "substituted" as used herein, alone or in combination, refer to groups which may be used to replace hydrogen. The substituted molecule may itself be further substituted in some embodiments of the invention. As referred here a "substituent derived from" refer to a group of atoms derived from a specific molecule or formula at any position of said molecule or formula. In some embodiments, a substituent derived from a molecule is the corresponding molecule wherein a hydrogen atom has been removed. For example, a substituent derived from CH₄ may be -CH₃.

The term "aromatic" or "aryl" refers to a cyclic or polycyclic moiety having a conjugated unsaturated (4η+2)π electron system (where n is a positive integer), sometimes referred to as a delocalized π electron system.

The term "heteroaromatic" or "heteroaryl" as used herein, alone or in combination, refers to an aromatic ring or an aromatic polycyclic system where at least one of the ring atoms are heteroatom(s). For example, heteroaryl or heteroaromatic refers to systems containing from 5 to 12 ring atoms an where one or more ring atoms are individually and independently selected from N, S, or O.

As used herein, the position marked with * in a structure refers to the attachment point.

### Detailed description

### A compound

In one main aspect, the present disclosure relates to a compound of formula (I): 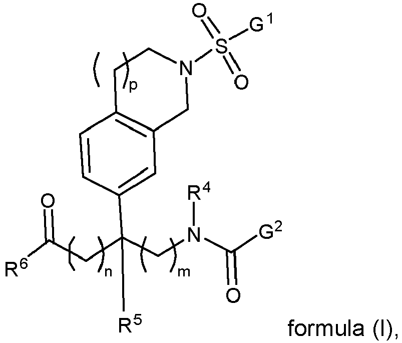 or a pharmaceutically acceptable salt thereof, wherein
G¹ is according to 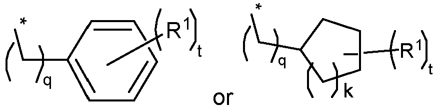
R' is in each instance individually selected from -H, -F, -Cl, -Br, -I,
-CN, -O-(C₁-C₃ alkyl), -CF₃, -OCF₃, -N(R^{1a})(R^{1b}), C₁-C₃ alkyl, -C(=O)O-(C₁-C₃ alkyl), or two R' groups are linked together to form a carbocycle or heterocycle;
R^{1a} and R^{1b} are each independently a C₁-C₃ alkyl;
G² is according to 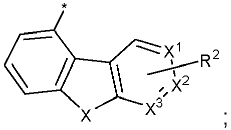
R² is H, -F, -Cl, -CH₃, or -O-CH₃;
X¹, X², X³ are each individually selected from CH, or N; provided that at most only one of X¹, X² and X³ is N;
X is O, S, 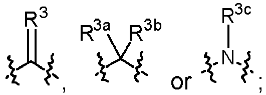
R³ is (=O), or (=S);
R^{3a} is H, halogen, -O-(C₁-C₃ alkyl), -O-CF₃, -S-(C₁ alkyl) or -C₁-C₃ alkyl; R^{3b} is H, - OH, -O-(C₁-C₃ alkyl), -O-CF₃, halogen, -S-(C₁ alkyl), or -C₁-C₃ alkyl; or R^{3a} and R^{3b} are linked together to form a carbocycle or heterocycle;
R^{3c} is H, C₁-C₃ alkyl, or -CF₃;
R⁴ is H, or C₁ alkyl;
R⁵ is H, or F;
R⁶ is -OH, -O-C₁-C₁₀ alkyl, -O-C₃-C₁₀ cycloalkyl, a heterocycle, or a -O-C₅-C₁₂ aryl;
n, m, p, and q are integers each individually and independently selected from 0 or 1;
k is an integer selected from 0 to 2
t is an integer selected from 0 to 4.

In some embodiments, t is 0 to 2. In one embodiment, t is 0. In one embodiment, t is 1. In one embodiment, t is 2.

In one embodiment, G¹ is according to any one of:

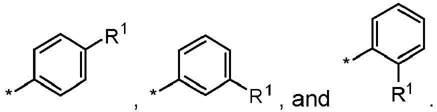

In one embodiment, G¹ is according to any one of:

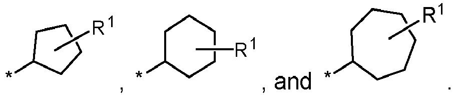

In one embodiment, R¹ is H.

In one embodiment, R¹ is not H.

In one embodiment, R¹ is -OCH₃.

In one embodiment, R¹ is -CH₃.

In one embodiment, R¹ is -N(CH₃)₂.

In one embodiment, R¹ is -O-(C₁₋C₃ alkyl). In one embodiment, R¹ is -O-(C₁ alkyl). In one embodiment, R¹ is -O-(C₂ alkyl). In one embodiment, R¹ is -O-(C₃ alkyl). In one embodiment, R¹ is -O(CF₃).

In one embodiment, R¹ is -F. In one embodiment, R¹ is -Cl. In one embodiment, R¹ is - Br. In one embodiment, R¹ is -I.

In one embodiment, R¹ is -CN.

In one embodiment, R¹ is -CF₃.

In one embodiment, R¹ is -C₁-C₃ alkyl. In one embodiment, wherein R¹ is -C₁ alkyl. In one embodiment, R¹ is -C₂ alkyl. In one embodiment, wherein R¹ is -C₃ alkyl.

In one embodiment, R¹ is -C(=O)O-(C₁-C₃ alkyl). In one embodiment, R¹ is -C(=O)O-(C₁ alkyl). In one embodiment, R¹ is -C(=O)O-(C₂ alkyl). In one embodiment, R¹ is -C(=O)O-(C₃ alkyl).

In one embodiment, wherein R¹ is -N(R^{1a})(R^{1b}), R^{1a} is C₁ alkyl, C₂ alkyl or C₃ alkyl and R^{1b} is C₁ alkyl, C₂ alkyl or C₃ alkyl.

In one embodiment, when t is 2, each instance of R¹ may be at any position of the ring in G¹. For example, in one embodiment, G¹ is according to any one of: 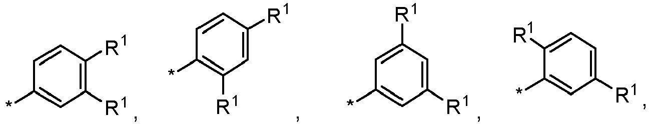 and 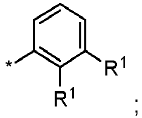 wherein each instance of R¹ is as described herein above.

In one embodiment, G¹ is according to any one of: 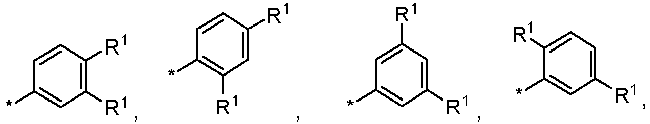 and 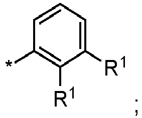 wherein each instance of R' is individually and independently selected from, -F, -Cl, -Br, -I, -CN, -O-(C₁-C₃ alkyl), -CF₃, -OCF₃, -N(R^{1a})(R^{1b}), C₁-C₃ alkyl, -C(=O)O-(C₁-C₃ alkyl), or two R' groups are linked together to form a carbocycle or heterocycle.

In one embodiment, t is 2 and each instance of R' is individually selected from -F, -CN, -O(CH₃), or -C(=O)O-(C₁-C₃ alkyl).

In some embodiments, two R¹ groups are linked together to form a carbocycle or heterocycle. In said embodiments, each R¹ is individually selected from -H, -O-(C₁-C₃ alkyl), -N(R^{1a})(R^{1b}), C₁-C₃ alkyl, -C(=O)O-(C₁-C₃ alkyl), wherein one hydrogen is removed to form a diradical. For example, when R' is C₁ alkyl and linked to another R¹ group, the C₁ alkyl is understood to be C₁ alkanediyl (-CH₂-).

In one embodiment, t is 2 and the two instances of R¹ are linked together to form a carbocycle or heterocycle. In one embodiment, t is 2 and two instances of R¹ are linked together to form a carbocycle. In one embodiment, t is 2 and two instances of R' are linked together to form a heterocycle.

Preferably, when two instances R' are linked together to form a carbocycle or heterocycle as described herein, said two instances of R' are adjacent to each other and the carbocycle or heterocycle is formed together with the intervening atoms. In some embodiments, when two instances R' are linked together to form a carbocycle or heterocycle as described herein, G¹ is according to: 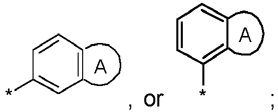 wherein A is a carbocycle or heterocycle. For example, A forms a 5-member or a 6-member carbocycle or heterocycle. In one embodiment, A forms a 6-member heterocycle with 2 heteroatoms in the cycle.

In one embodiment, G¹ is selected from any one of the groups shown in **Table A1:**

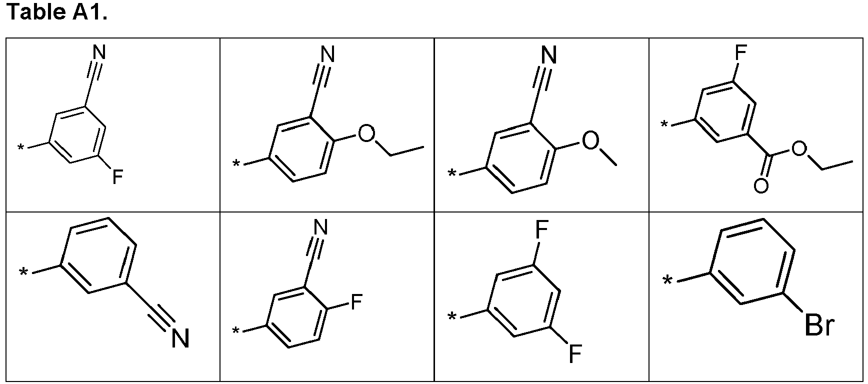

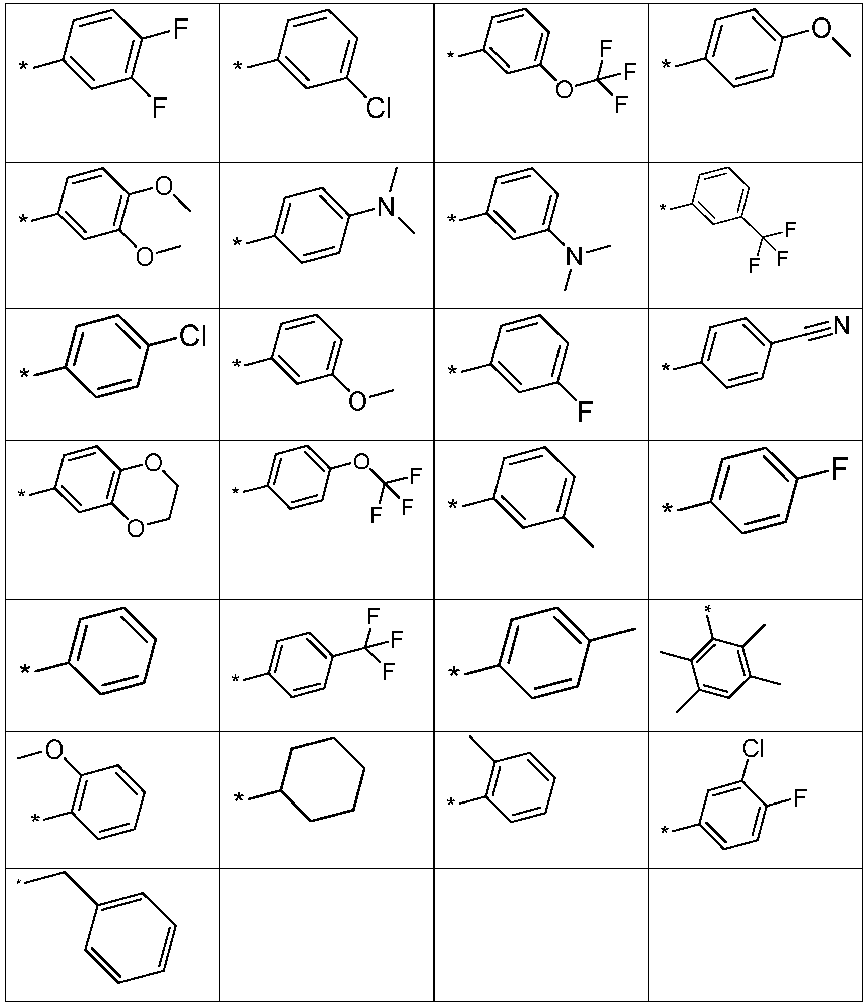

In one embodiment, G¹ is according to any one of the groups shown in **Table A2.**

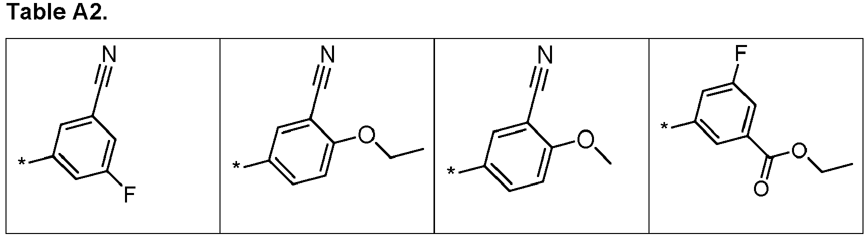

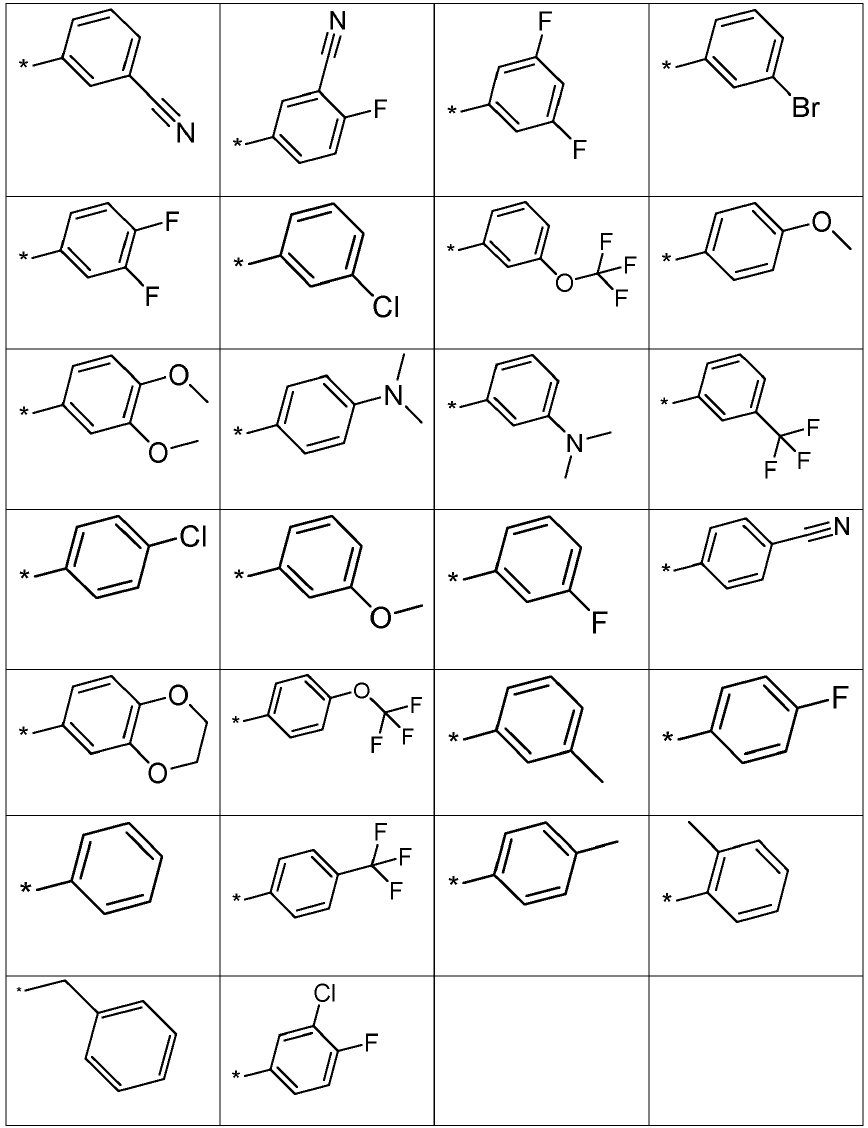

In one embodiment, G¹ is according to any one of the groups shown in G¹ is according to any one of the groups shown in **Table A3.**

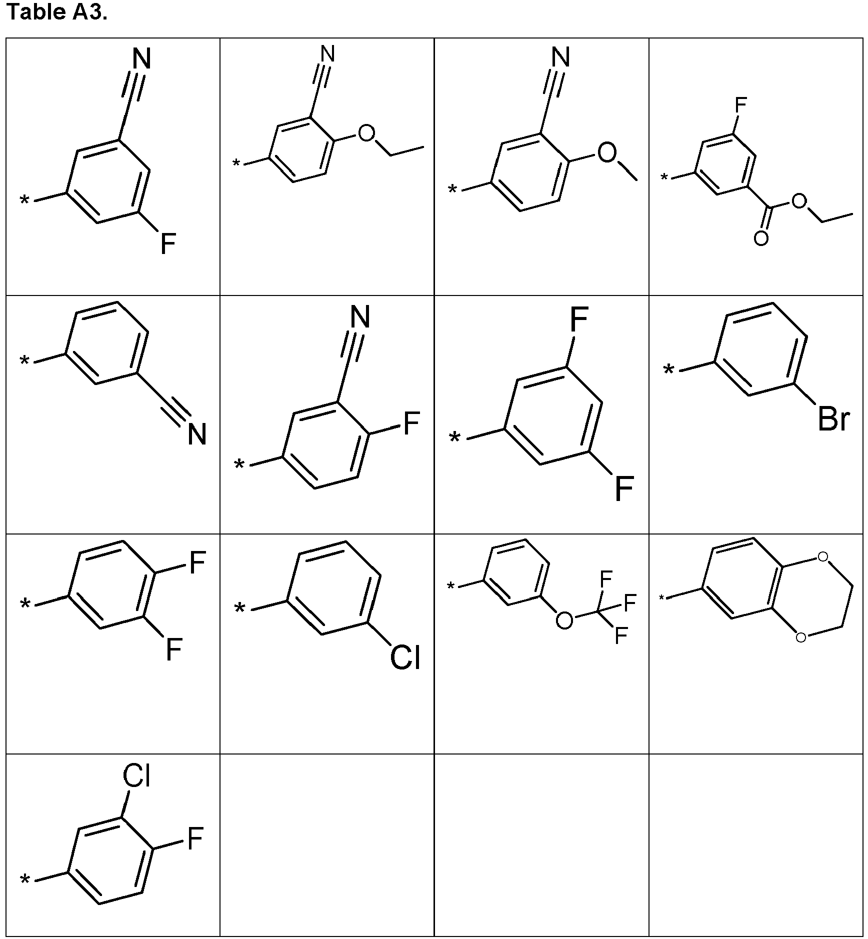

In one embodiment, t is 2 and each instance of R' is individually selected from -F, -CN, -O(CH₃), -O-(CH₂-CH₃), or -C(=O)O-(C₁-C₃ alkyl).

In one embodiment, t is 1 and R' is -F, -CN, -O-(CF₃), or -Br or -Cl.

In one embodiment, at least on instance of R' is -F, -CN, -O(CH₃), or -C(=O)O-(C₁-C₃ alkyl), -Br or -Cl.

In one embodiment, R² is H.

In one embodiment, R² is -F, -Cl, -CH₃, or -O-CH₃

In one embodiment, one of X¹, X² or X³ is N, such as X¹ is N, X² is N or X³ is N. In one embodiment, such as X¹ is N; X² is CH and X³ is CH; or X¹ is CH; X² is N and X³ is CH; or X¹ is CH; X² is CH and X³ is N.

In one embodiment, X¹, X² and X³ are CH.

In one embodiment, wherein G² is 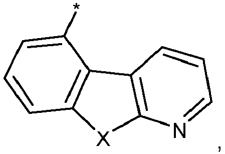 wherein X is as defined herein.

In one embodiment, G² is 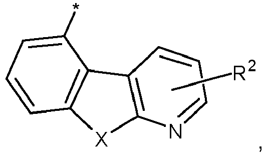 wherein R² is as defined herein.

In one embodiment, G² is any one of: 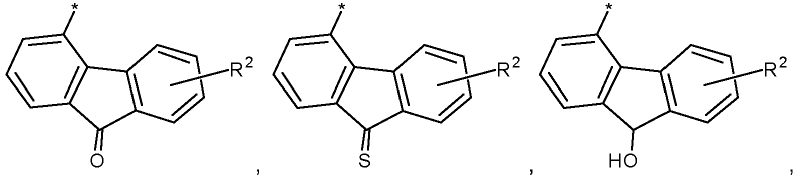 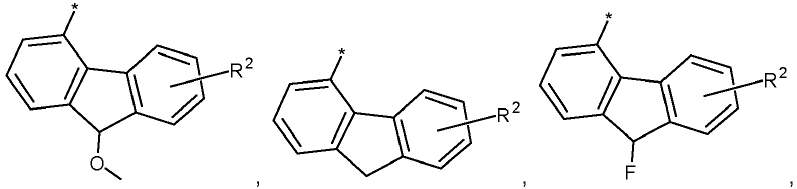 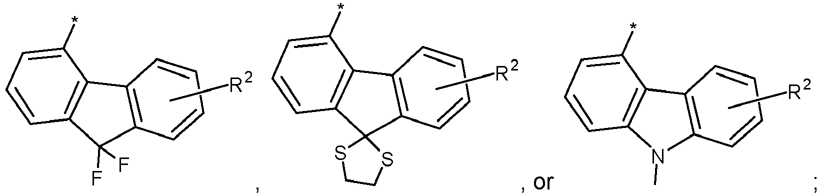 wherein R² is as defined herein.

In one embodiment, X is O. In one embodiment, X is S.

In one embodiment, X is 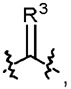 wherein R³ is (=O). In one embodiment, X is 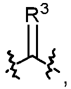 wherein R³ is or (=S).

In one embodiment, X is 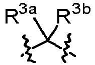 wherein R^{3a} is H, and R^{3b} is H, -OH, -O-(C₁-C₃ alkyl), -O-CF₃, halogen, -S-(C₁ alkyl), or -C₁-C₃ alkyl.

In one embodiment, X is 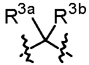 wherein R^{3a} is halogen, and R^{3b} is H, -OH, -O-(C₁-C₃ alkyl), -O-CF₃, halogen, -S-(C₁ alkyl), or -C₁-C₃ alkyl.

In one embodiment, X is 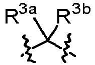 wherein R^{3a} is -O-(C₁ alkyl) or -S-(C₁ alkyl); and R^{3b} is H, -OH, -O-(C₁-C₃ alkyl), -O-CF₃, halogen, -S-(C₁ alkyl), or -C₁-C₃ alkyl.

In one embodiment, wherein X is 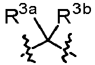 wherein are R^{3a} and R^{3b} are linked together to form a carbocycle or heterocycle.

In one embodiment, X is 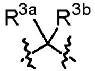 wherein are R^{3a} and R^{3b} are linked together to form a carbocycle.

In one embodiment, X is 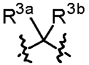 wherein are R^{3a} and R^{3b} are linked together to form a heterocycle.

In one embodiment, X is 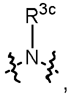 wherein R^{3c} is H, C₁-C₃ alkyl, or -CF₃.

In one embodiment, X is 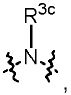 wherein R^{3c} C₁-C₃ alkyl, such as C₁ alkyl, C₂ alkyl, or C₃ alkyl.

In one embodiment, X is selected from any one of the group consisting of:

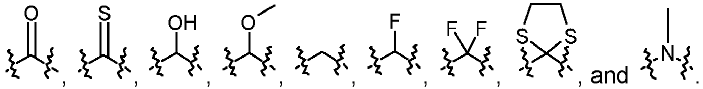

In one embodiment, X is 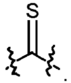 In one embodiment, X is

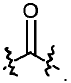

In one embodiment, X is

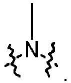

In one embodiment, X is

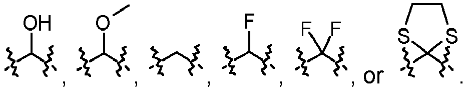

In one embodiment, X is

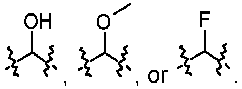

In one embodiment, X is

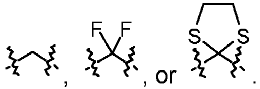

In one embodiment, X is 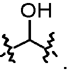 In one embodiment, X is 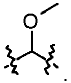 In one embodiment, X is 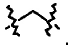 In one embodiment, X is 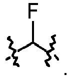 In one embodiment, X is

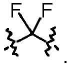

In one embodiment, X is

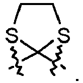

In one embodiment, G² is any one of the group consisting of:

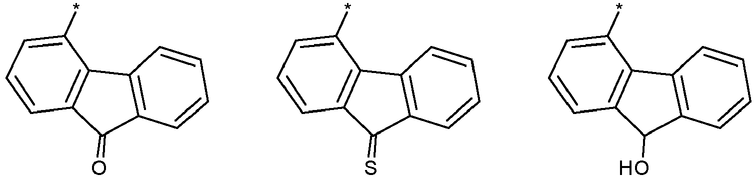

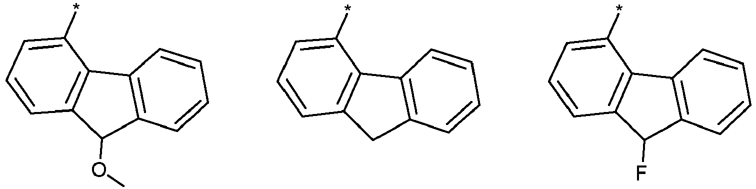

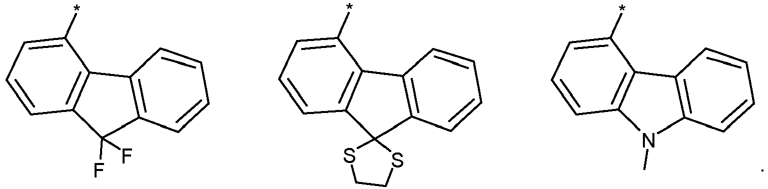

In one embodiment, R⁴ is H. In one embodiment, R⁴ is C₁ alkyl.

In one embodiment, R⁵ is H. In one embodiment, R⁵ is F.

In one embodiment, n is 0. In one embodiment, m is 0. In one embodiment, q is 0. In one embodiment, p is 0.

In one embodiment, n is 1. In one embodiment, m is 1. In one embodiment, q is 1. In one embodiment, p is 1.

In one embodiment, n, m, and q are 0 and p is 1.

In one embodiment, n is 1, m is 1; p is 1 and q is 0.

In one embodiment, n, m, p and q are 0. In one embodiment, n, m, p and q are 1.

In one embodiment, n and p are 1; and m and q are 0.

In one embodiment, n and m are 0; and p and q are 1.

In one embodiment, R⁶ is -OH.

In one embodiment, the compound is according to any one of the compounds shown in Table B, or a pharmaceutically acceptable salt thereof.

**Table B.**

| | | | |
|---|---|---|---|
| K1 | 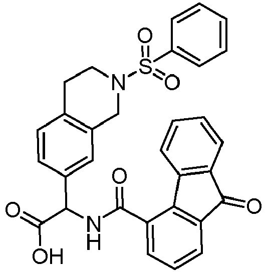 | K28 | 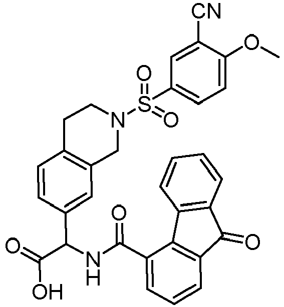 |
| K2 | 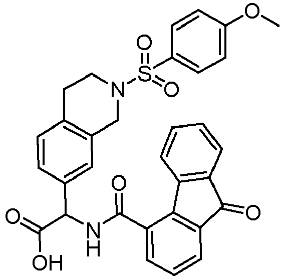 | K29 | 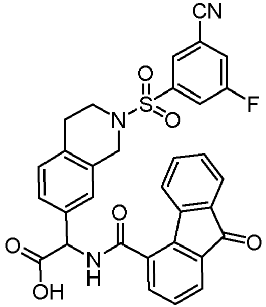 |
| K3 | 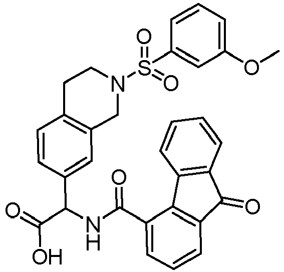 | K30 | 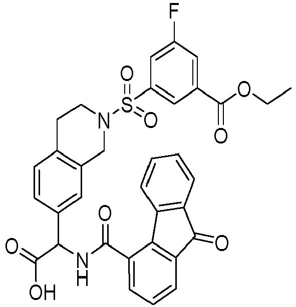 |
| K4 | 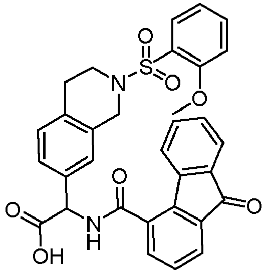 | K31 | 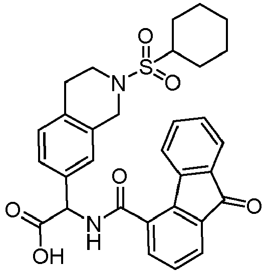 |
| K5 | 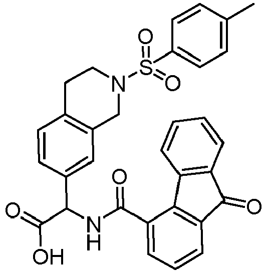 | K32 | 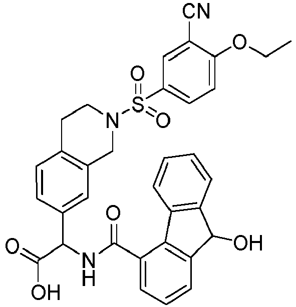 |
| K6 | 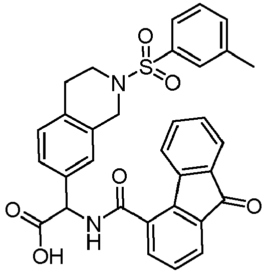 | K33 | 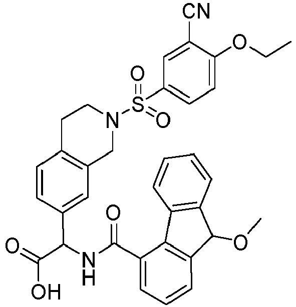 |
| K7 | 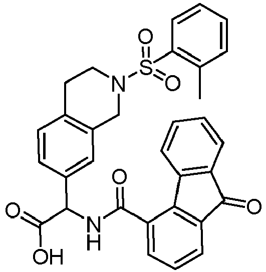 | K34 | 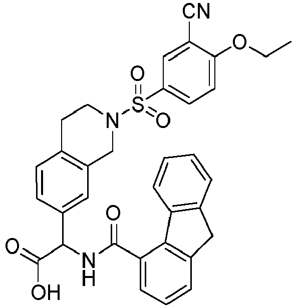 |
| K8 | 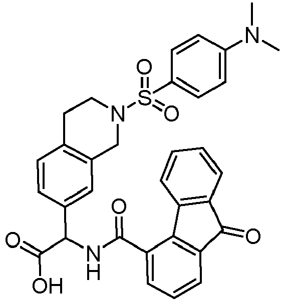 | K35 | 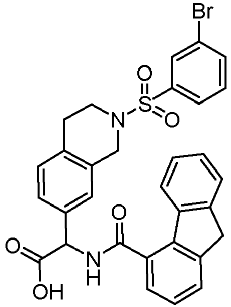 |
| K9 | 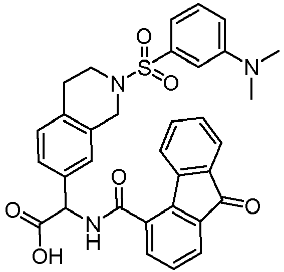 | K36 | 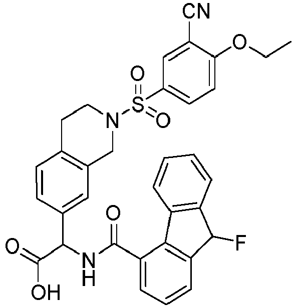 |
| K10 | 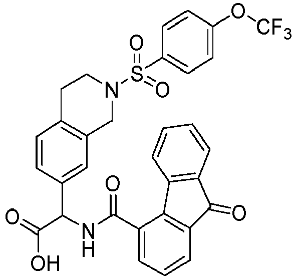 | K37 | 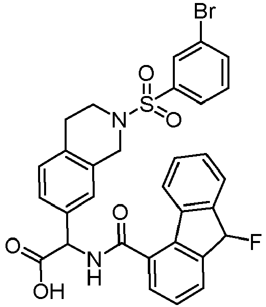 |
| K11 | 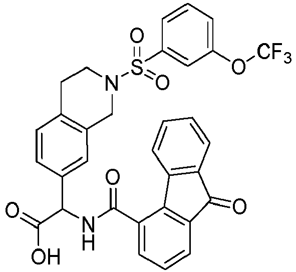 | K38 | 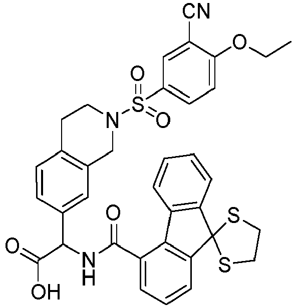 |
| K12 | 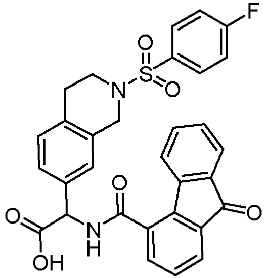 | K39 | 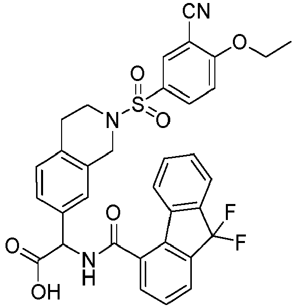 |
| K13 | 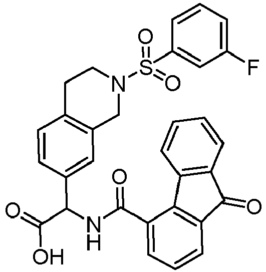 | K40 | 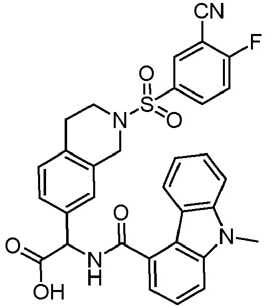 |
| K14 | 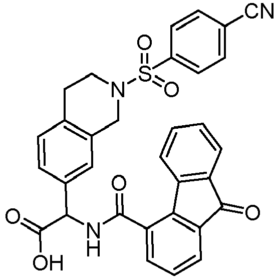 | K41 | 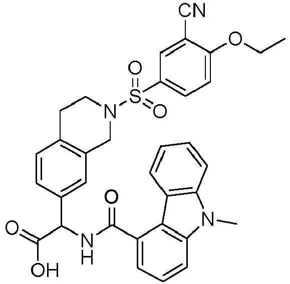 |
| K15 | 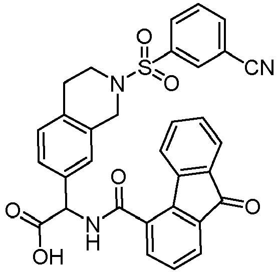 | K42 | 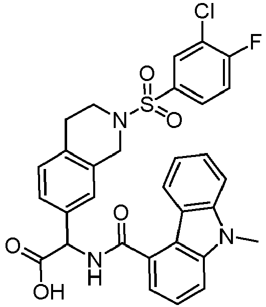 |
| K16 | 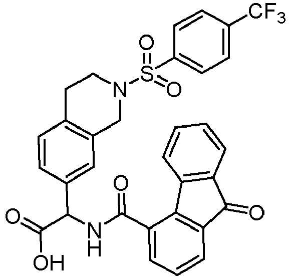 | K43 | 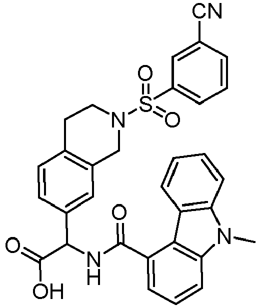 |
| K17 | 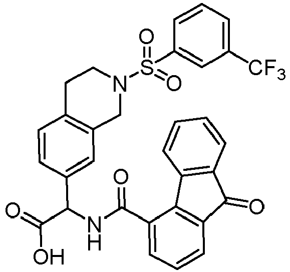 | K44 | 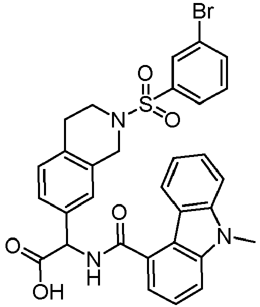 |
| K18 | 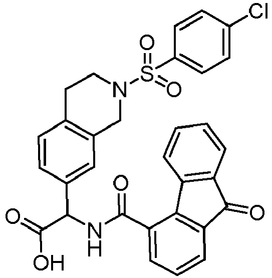 | K45 | 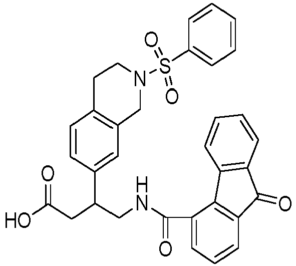 |
| K19 | 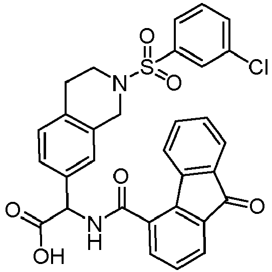 | K46 | 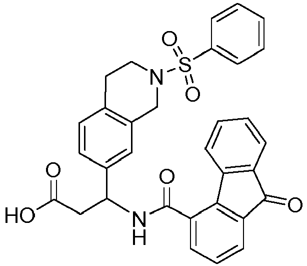 |
| K20 | 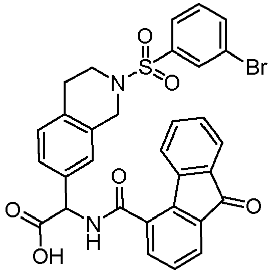 | K47 | 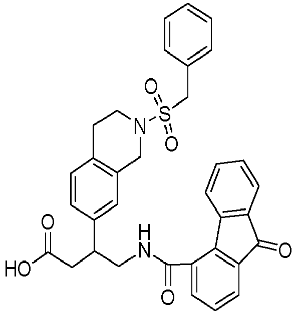 |
| K21 | 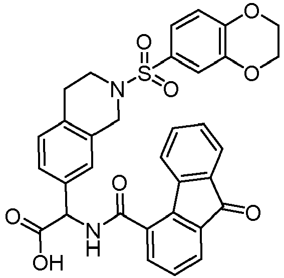 | K48 | 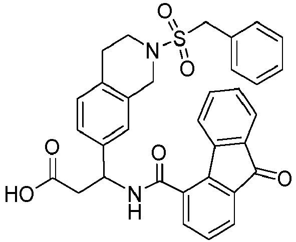 |
| K22 | 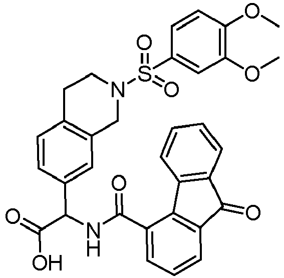 | K49 | 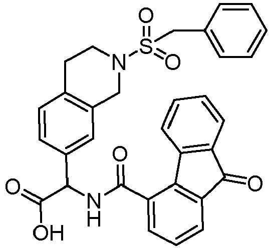 |
| K23 | 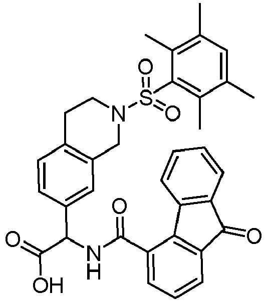 | K50 | 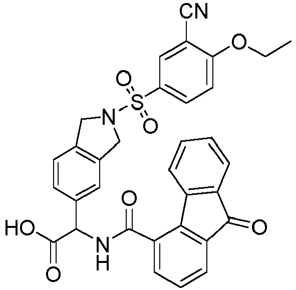 |
| K24 | 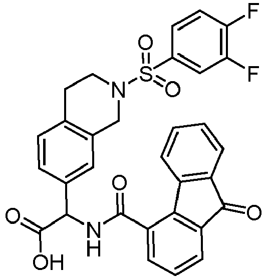 | K51 | 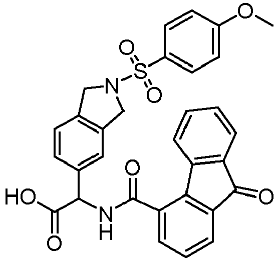 |
| K25 | 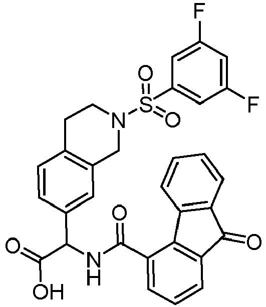 | K52 | 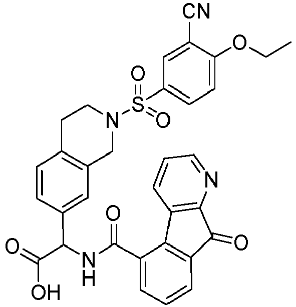 |
| K26 | 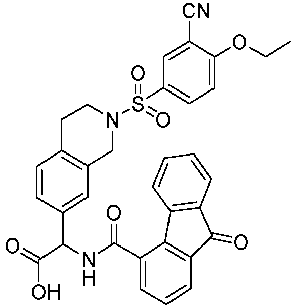 | K53 | 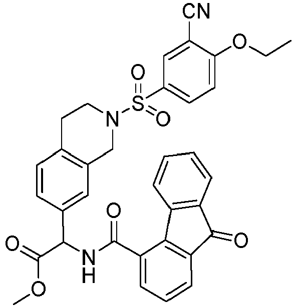 |
| K27 | 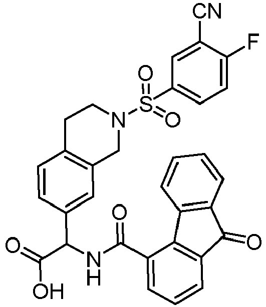 | K54 | 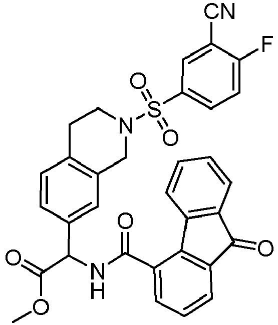 |

In one embodiment, the compound is 2-(9-oxo-9H-fluorene-4-carboxamido)-2-(2-(phenylsulfonyl)-1,2,3,4-tetrahydroisoquinolin-7-yl)acetic acid , or a pharmaceutically acceptable salt thereof.

In one embodiment, the compound is 2-(2-((4-methoxyphenyl)sulfonyl)-1,2,3,4-tetrahydroisoquinolin-7-yl)-2-(9-oxo-9H-fluorene-4-carboxamido)acetic acid , or a pharmaceutically acceptable salt thereof.

In one embodiment, the compound is 2-(2-((3-methoxyphenyl)sulfonyl)-1,2,3,4-tetrahydroisoquinolin-7-yl)-2-(9-oxo-9H-fluorene-4-carboxamido)acetic acid , or a pharmaceutically acceptable salt thereof.

In one embodiment, the compound is 2-(2-((2-methoxyphenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(9-oxo-9H-fluorene-4-carboxamido)-2-(2-tosyl-1,2,3,4-tetrahydroisoquinolin-7-yl)acetic acid, or a pharmaceutically acceptable salt thereof.

In one embodiment, the compound is 2-(9-oxo-9H-fluorene-4-carboxamido)-2-(2-(m-tolylsulfonyl)-1,2,3,4-tetrahydroisoquinolin-7-yl)acetic acid, or a pharmaceutically acceptable salt thereof.

In one embodiment, the compound is 2-(9-oxo-9H-fluorene-4-carboxamido)-2-(2-(o-tolylsulfonyl)-1,2,3,4-tetrahydroisoquinolin-7-yl)acetic acid, or a pharmaceutically acceptable salt thereof.

In one embodiment, the compound is 2-(2-((4-(dimethylamino)phenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-(dimethylamino)phenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(9-oxo-9H-fluorene-4-carboxamido)-2-(2-((4-(trifluoromethoxy)phenyl)sulfonyl)-1,2,3,4-tetrahydroisoquinolin-7-yl)acetic acid, or a pharmaceutically acceptable salt thereof.

In one embodiment, the compound is 2-(9-oxo-9H-fluorene-4-carboxamido)-2-(2-((3-(trifluoromethoxy)phenyl)sulfonyl)-1,2,3,4-tetrahydroisoquinolin-7-yl)acetic acid, or a pharmaceutically acceptable salt thereof.

In one embodiment, the compound is 2-(2-((4-fluorophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-fluorophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((4-cyanophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-cyanophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(9-oxo-9H-fluorene-4-carboxamido)-2-(2-((4-(trifluoromethyl)phenyl)sulfonyl)-1,2,3,4-tetrahydroisoquinolin-7-yl)acetic acid, or a pharmaceutically acceptable salt thereof.

In one embodiment, the compound is 2-(9-oxo-9H-fluorene-4-carboxamido)-2-(2-((3-(trifluoromethyl)phenyl)sulfonyl)-1,2,3,4-tetrahydroisoquinolin-7-yl)acetic acid, or a pharmaceutically acceptable salt thereof.

In one embodiment, the compound is 2-(2-((4-chlorophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-chlorophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-bromophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((2,3-dihydrobenzo[b][1,4]dioxin-6-yl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3,4-dimethoxyphenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(9-oxo-9H-fluorene-4-carboxamido)-2-(2-((2,3,5,6-tetramethylphenyl)sulfonyl)-1,2,3,4-tetrahydroisoquinolin-7-yl)acetic acid, or a pharmaceutically acceptable salt thereof.

In one embodiment, the compound is 2-(2-((3,4-difluorophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3,5-difluorophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-cyano-4-ethoxyphenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-cyano-4-fluorophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-cyano-4-methoxyphenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-cyano-5-fluorophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-(ethoxycarbonyl)-5-fluorophenyl)sulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-(cyclohexylsulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-cyano-4-ethoxyphenyl)sulfonyl)-1,2,3,4-tetrahydroisoquinolin-7-yl)-2-(9-hydroxy-9H-fluorene-4-carboxamido)acetic acid, or a pharmaceutically acceptable salt thereof.

In one embodiment, the compound is 2-(2-((3-cyano-4-ethoxyphenyl)sulfonyl)-1,2,3,4-tetrahydroisoquinolin-7-yl)-2-(9-methoxy-9H-fluorene-4-carboxamido)acetic acid, or a pharmaceutically acceptable salt thereof.

In one embodiment, the compound is 2-(2-((3-cyano-4-ethoxyphenyl)sulfonyl)-1,2,3,4-tetrahydroisoquinolin-7-yl)-2-(9H-fluorene-4-carboxamido)acetic acid, or a pharmaceutically acceptable salt thereof.

In one embodiment, the compound is 2-(2-((3-bromophenyl)sulfonyl)-1,2,3,4-tetrahydroisoquinolin-7-yl)-2-(9H-fluorene-4-carboxamido)acetic acid, or a pharmaceutically acceptable salt thereof.

In one embodiment, the compound is 2-(2-((3-cyano-4-ethoxyphenyl)sulfonyl)-1,2,3,4-tetrahydroisoquinolin-7-yl)-2-(9-fluoro-9H-fluorene-4-carboxamido)acetic acid, or a pharmaceutically acceptable salt thereof.

In one embodiment, the compound is 2-(2-((3-bromophenyl)sulfonyl)-1,2,3,4-tetrahydroisoquinolin-7-yl)-2-(9-fluoro-9H-fluorene-4-carboxamido)acetic acid, or a pharmaceutically acceptable salt thereof.

In one embodiment, the compound is 2-(2-((3-cyano-4-ethoxyphenyl)sulfonyl)-1,2,3,4-tetrahydroisoquinolin-7-yl)-2-(spiro[fluorene-9,2'-[1,3]dithiolane]-4-carboxamido)acetic acid, or a pharmaceutically acceptable salt thereof.

In one embodiment, the compound is 2-(2-((3-cyano-4-ethoxyphenyl)sulfonyl)-1,2,3,4-tetrahydroisoquinolin-7-yl)-2-(9,9-difluoro-9H-fluorene-4-carboxamido)acetic acid, or a pharmaceutically acceptable salt thereof.

In one embodiment, the compound is 2-(2-((3-cyano-4-fluorophenyl)sulfonyl)-1,2,3,4-tetrahydroisoquinolin-7-yl)-2-(9-methyl-9H-carbazole-4-carboxamido)acetic acid, or a pharmaceutically acceptable salt thereof.

In one embodiment, the compound is 2-(2-((3-cyanophenyl)sulfonyl)-1,2,3,4-tetrahydroisoquinolin-7-yl)-2-(9-methyl-9H-carbazole-4-carboxamido)acetic acid, or a pharmaceutically acceptable salt thereof.

In one embodiment, the compound is 2-(2-((3-cyano-4-ethoxyphenyl)sulfonyl)-1,2,3,4-tetrahydroisoquinolin-7-yl)-2-(9-methyl-9H-carbazole-4-carboxamido)acetic acid, or a pharmaceutically acceptable salt thereof.

In one embodiment, the compound is 2-(2-((3-chloro-4-fluorophenyl)sulfonyl)-1,2,3,4-tetrahydroisoquinolin-7-yl)-2-(9-methyl-9H-carbazole-4-carboxamido)acetic acid, or a pharmaceutically acceptable salt thereof.

In one embodiment, the compound is 2-(2-((3-bromophenyl)sulfonyl)-1,2,3,4-tetrahydroisoquinolin-7-yl)-2-(9-methyl-9H-carbazole-4-carboxamido)acetic acid, or a pharmaceutically acceptable salt thereof.

In one embodiment, the compound is 4-(9-oxo-9H-fluorene-4-carboxamido)-3-(2-(phenylsulfonyl)-1,2,3,4-tetrahydroisoquinolin-7-yl)butanoic acid, or a pharmaceutically acceptable salt thereof.

In one embodiment, the compound is 3-(9-oxo-9H-fluorene-4-carboxamido)-3-(2-(phenylsulfonyl)-1,2,3,4-tetrahydroisoquinolin-7-yl)propanoic acid, or a pharmaceutically acceptable salt thereof.

In one embodiment, the compound is 3-(2-(benzylsulfonyl)-1,2,3,4-tetrahydroisoquinolin-7-yl)-4-(9-oxo-9H-fluorene-4-carboxamido)butanoic acid, or a pharmaceutically acceptable salt thereof.

In one embodiment, the compound is 3-(2-(benzylsulfonyl)-1,2,3,4-tetrahydroisoquinolin-7-yl)-3-(9-oxo-9H-fluorene-4-carboxamido)propanoic acid, or a pharmaceutically acceptable salt thereof.

In one embodiment, the compound is 2-(2-(benzylsulfonyl)-1,2,3,4-tetrahydroisoquinolin-7-yl)-2-(9-oxo-9H-fluorene-4-carboxamido)acetic acid, or a pharmaceutically acceptable salt thereof.

In one embodiment, the compound is 2-(2-((3-cyano-4-ethoxyphenyl)sulfonyl)isoindolin-5-yl)-2-(9-oxo-9H-fluorene-4-carboxamido)acetic acid, or a pharmaceutically acceptable salt thereof.

In one embodiment, the compound is 2-(2-((4-methoxyphenyl)sulfonyl)isoindolin-5-yl)-2-(9-oxo-9H-fluorene-4-carboxamido)acetic acid, or a pharmaceutically acceptable salt thereof.

In one embodiment, the compound is 2-(2-((3-cyano-4-ethoxyphenyl)sulfonyl)-1,2,3,4-tetrahydroisoquinolin-7-yl)-2-(9-oxo-9H-indeno[2,1-b]pyridine-5-carboxamido)acetic acid, or a pharmaceutically acceptable salt thereof.

In one embodiment, the compound is Methyl 2-(2-((3-cyano-4-ethoxyphenyl)sulfonyl)-1,2,3,4-tetrahydroisoquinolin-7-yl)-2-(9-oxo-9H-fluorene-4-carboxamido)acetate, or a pharmaceutically acceptable salt thereof.

In one embodiment, the compound is Methyl 2-(2-((3-cyano-4-fluorophenyl)sulfonyl)-1,2,3,4-tetrahydroisoquinolin-7-yl)-2-(9-oxo-9H-fluorene-4-carboxamido)acetate, or a pharmaceutically acceptable salt thereof.

### Pharmaceutically acceptable salts

The compound of the invention may be provided in any form suitable for the intended administration, including pharmaceutically (i.e. physiologically) acceptable salts. Such salts may be formed by procedures well known and described in the art.

### Prodrugs

In one aspect, the present invention relates to a prodrug of a compound of Keap1-Nrf2 small-molecule inhibitor as described herein.

As used herein "prodrug" refers to a compound which is converted to a therapeutically active compound after administration, and the term should be interpreted as broadly herein as is generally understood in the art. In one embodiment, the conversion occurs by hydrolysis of an ester group or some other biologically labile group. Generally, but not necessarily, a prodrug is inactive or less active than the therapeutically active compound to which it is converted.

Particularly, prodrugs according to the present disclosure refer to ester prodrugs wherein wherein R⁶ in formula (I) is not -OH. Said prodrugs may convert to active compounds by hydrolysis of the corresponding group e.g. an ester group, and forming compounds wherein R⁶ in formula (I) is -OH. Ester prodrugs of the compounds disclosed herein are contemplated. While not intending to be limiting, any R⁶ group forming an ester may be contemplated.

Thus, in some embodiments, R⁶ is a -O-C₁-C₁₀ alkyl, -O-C₃-C₁₀ cycloalkyl, a heterocycle, or a -O-C₅-C₁₂ aryl.

In one embodiment, R⁶ is -O-C₁-C₁₀ alkyl. In one embodiment, R⁶ is -O-C₃-C₁₀ cycloalkyl. In one embodiment, R⁶ is a -O-C₅-C₁₂ aryl.

In one particular embodiment, R⁶ is a thiazolidinone group according to: 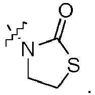 The thiazolidinone group acts as protective group which is sensitive to H₂O₂ and other reactive oxygen species (ROS) associated with inflammation.⁴³

### Pharmaceutical formulations

In one aspect, the present invention relates to a composition comprising the compound as disclosed herein. The composition according to the present disclosure may be used for treating, ameliorating and/or preventing a disease caused by formation of ROS. Thus, preferably, the compositions and compounds are pharmaceutically acceptable. In one embodiment the composition as described herein is in the form of a pharmaceutical formulation. In one embodiment, the composition as described herein further comprises a pharmaceutically acceptable carrier. In one aspect, the present invention concerns a composition comprising the compound as defined herein and a pharmaceutically acceptable carrier.

### Biological activity

The compounds of the present invention are capable of binding to the Keap1 Kelch domain and inhibiting the Keap1-Nrf2 interaction. In one embodiment, the compound is capable of reducing binding of Keap1 to Nrf2. In one aspect, the present invention relates to a method for reducing binding of Keap1 to Nrf2, said method comprising administering a compound or a composition as defined herein. In one embodiment, the compound has a Kᵢ value for inhibiting binding of Keap1 to Nrf2 of less than 500 µM, such as less than 100 µM, such as less than 50 µM, such as less than 25 µM, such as less than 10 µM, such as less than 5 µM, such as less than 1 µM, such as less than 0.5 µM, such as less than 0.1 µM, such as less than 0.01 µM. Said Kᵢ value may be determined using a fluorescence polarization (FP) competition assay.

The examples develop further how Ki may be determined using fluorescence polarization (FP) assays.

By inhibiting the Keap1-Nrf2 interaction, the cellular concentration of detoxifying and antioxidant enzymes are increased, and level of ROS is reduced. Thus, in one embodiment, the compound is capable of reducing the cellular concentration of ROS. In one aspect, the present invention relates to a method for reducing the cellular concentration of ROS, said method comprising administering a compound or composition as defined herein. In one aspect, the present invention relates to an in vitro method for reducing the cellular concentration of ROS, said method comprising contacting cells expressing Keap1 with a compound or composition as defined herein.

ROS are known to contribute to oxidative stress. Thus, by inhibiting the Keap1-Nrf2 interaction and thereby reducing the cellular concentration of ROS, the oxidative stress is reduced. In one embodiment, the compound is capable of reducing oxidative stress. In one aspect, the present invention relates to a method for reducing oxidative stress in a subject, said method comprising administering a compound or composition as defined herein to a subject in need thereof. In one aspect, the present invention relates to an in vitro method for reducing oxidative stress, said method comprising contacting cells expressing Keap1 with a compound or composition as defined herein.

Oxidative stress may trigger inflammation. In one aspect, the present invention relates to a compound or composition as defined herein for use in treating, ameliorating and/or preventing inflammation. In one aspect, the present invention relates to a method of treating, ameliorating and/or preventing inflammation, said method comprising administering a therapeutically effective dose of a compound or composition as defined herein to said subject in need thereof. In one aspect, the present invention relates to use of a compound or composition as defined herein in the manufacture of a medicament for treating, ameliorating and/or preventing inflammation.

In one aspect, the present invention relates to a method for inhibiting Keap1, said method comprising contacting cells expressing Keap1 with a compound or composition as defined herein.

### Medical use

In one aspect, the present invention relates to a compound or composition as defined herein for use as a medicament.

In one aspect, the present invention relates to a compound or composition as defined herein for use in treating, ameliorating and/or preventing a disease where ROS and/or inflammation is involved. For example, the disease may be caused by excessive ROS formation and/or by misplaced ROS. In one embodiment, the disease caused by formation of ROS is a disease in which inflammation plays a role in pathogenesis. In one embodiment, the disease caused by formation of ROS involves inflammation. In one embodiment, the disease caused by ROS is selected from the group consisting of Central Nervous System (CNS) diseases; lung diseases; autoimmune disorders; cancer; metabolic and/or inflammatory liver conditions; CKD, incl. Alport syndrome; skin diseases; retinal ischemia-reperfusion (I/R) injury; ulcerative colitis; and acetaminophen-induced hepatotoxicity.

In one aspect, the present invention relates to a compound or composition as defined herein for use in treating, ameliorating and/or preventing a disease selected from the group consisting of a CNS disease; a lung disease; an autoimmune disorder; cancer; a metabolic and/or inflammatory liver condition; CKD, incl. Alport syndrome; a skin disease; retinal ischemia-reperfusion (I/R) injury; ulcerative colitis; and acetaminophen-induced hepatotoxicity, in a subject.

In one embodiment, the disease is a CNS disease. In one embodiment, the CNS disease is selected from the group consisting of ischemic stroke, traumatic brain injury, cerebral ischemia, intracerebral haemorrhage, Alzheimer's disease, Parkinson's disease, and multiple sclerosis. In one embodiment, the CNS disorder is a CNS injury, such as selected from the group consisting of cerebral ischemia, intracerebral haemorrhage, ischemic stroke, and traumatic brain injury. In one embodiment, the CNS disorder is a neurodegenerative disorder, such as selected from the group consisting of Alzheimer's disease, Parkinson's disease, and multiple sclerosis.

In one embodiment, the disease is I/R injury.

In one embodiment, the disease is a lung disease. In one embodiment, the lung disease is selected from the group consisting of acute lung inflammation, for example mediated by viral infections; chronic obstructive pulmonary disease; and pulmonary hypertension.

In one embodiment, the disease is a metabolic and/or inflammatory liver condition. In one embodiment, the disease is non-alcoholic steatohepatitis (NASH).

In one embodiment, the disease is cancer.

In one embodiment, the disease is a CKD. In one embodiment, the disease is Alport syndrome.

In one embodiment, the disease is ulcerative colitis. In one embodiment, the disease is acetaminophen-induced hepatotoxicity.

In one embodiment, the disease is an autoimmune disorder. In one embodiment, the autoimmune disorder is rheumatoid arthritis.

In one embodiment, the disease is a skin disease. In one embodiment, the skin disease is atopic dermatitis or psoriasis.

The term "treatment" refers to the combating of a disease or disorder. "Treatment" or "treating," as used herein, includes any desirable effect on the symptoms or pathology of a disease or condition as described herein, and may include even minimal changes or improvements in one or more measurable markers of the disease or condition being treated. "Treatment" or "treating" does not necessarily indicate complete eradication or cure of the disease or condition, or associated symptoms thereof. In some embodiments, the term "treatment" encompasses amelioration and prevention.

The term "amelioration" refers to moderation in the severity of the symptoms of a disease or condition. Improvement in a patient's condition, or the activity of making an effort to correct, or at least make more acceptable, conditions that are difficult to endure related to patient's condition is considered "ameliorative" treatment.

The term "prevent" or "preventing" refers to precluding, averting, obviating, forestalling, stopping, or hindering something from happening, especially by advance action.

In one embodiment, the compound is administered at a therapeutically effective dose. A "therapeutically effective dose" refers to that amount of active ingredient, which ameliorates the symptoms or condition. In one embodiment, the compound or composition as defined herein is administered at a daily dosage of between 0.1 µg compound per kg and 1000 mg compound per kg bodyweight.

In one aspect, the present invention relates to a method of treating a disease caused by ROS in a subject, said method comprising administering a therapeutically effective dose of a compound or composition as defined herein to said subject in need thereof.

In one aspect, the present invention relates to use of a compound or composition as defined herein in the manufacture of a medicament for treatment of a disease caused by ROS.

In one embodiment, the subject is a mammal. In one embodiment, the subject is a human.

In one aspect, the present invention relates to a method of reducing inflammation, said method comprising administering a therapeutically effective amount of the compound or composition as defined herein to a subject in need thereof.

In one embodiment, the subject is a mammal. In one embodiment, the subject is a human. In one embodiment, the subject is suffering from a condition selected from the group consisting of a CNS disease; a lung disease; an autoimmune disorder; cancer; a metabolic and fibrotic kidney and liver condition; Alport syndrome; and a skin disease.

### Items

1. A compound according to formula (I): 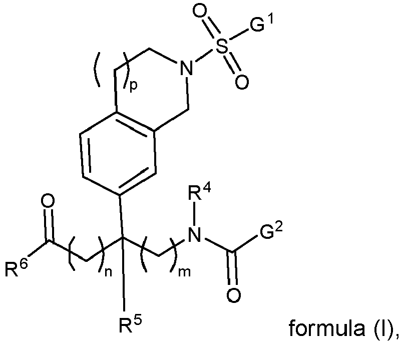 or a pharmaceutically acceptable salt thereof, wherein
   G¹ is according to 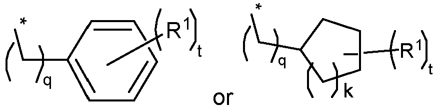
   R' is in each instance individually selected from -H, -F, -Cl, -Br, -I, -CN, -O-(C₁-C₃ alkyl), -CF₃, -OCF₃, -N(R^{1a})(R^{1b}),
   C₁-C₃ alkyl, -C(=O)O-(C₁-C₃ alkyl), or two R' groups are linked together to form a carbocycle or heterocycle;
   R^{1a} and R^{1b} are each independently a C₁-C₃ alkyl;
   G² is according to 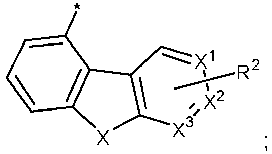
   R² is H, -F, -Cl, -CH₃, or -O-CH₃;
   X¹, X², X³ are each individually selected from CH, or N; provided that at most only one of X¹, X² and X³ is N;
   X is O, S, 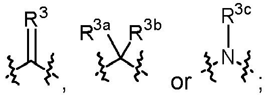
   R³ is (=O), or (=S);
   R^{3a} is H, halogen, -O-(C₁-C₃ alkyl), -O-CF₃, -S-(C₁ alkyl) or -C₁-C₃ alkyl; R^{3b} is H, -OH, -O-(C₁-C₃ alkyl), -O-CF₃, halogen, -S-(C₁ alkyl), or -C₁-C₃ alkyl; or R^{3a} and R^{3b} are linked together to form a carbocycle or heterocycle;
   R^{3c} is H, C₁-C₃ alkyl, or -CF₃;
   R⁴ is H, or C₁ alkyl;
   R⁵ is H, or F;
   R⁶ is -OH, -O-C₁-C₁₀ alkyl, -O-C₃-C₁₀ cycloalkyl, a heterocycle,or a -O-C₅-C₁₂ aryl;
   n, m, p, and q are integers each individually and independently selected from 0 or 1;
   k is an integer selected from 0 to 2
   t is an integer selected from 0 to 4.
2. The compound according to item 1, wherein t is 0 to 2.
3. The compound according to any one of the preceding items , wherein t is 0.
4. The compound according to any one of items 1 to 2, wherein t is 1.
5. The compound according to any one of items 1 to 2, wherein t is 2.
6. The compound according to any one of items 1 to 2 and 4 to 5, wherein G¹ is according to any one of: 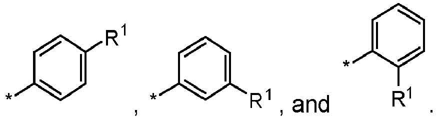
7. The compound according to any one of items 1 to 2 and 4 to 5, wherein G¹ is according to any one of: 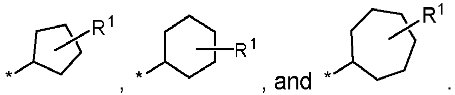
8. The compound according to any one of items 1 to 2 and 4 to 7, wherein R' is H.
9. The compound according to any one of items 1 to 2 and 4 to 7, wherein R' is not H.
10. The compound according to any one of items 1 to 2 and 4 to 7, wherein R' is -OCH₃.
11. The compound according to any one of items 1 to 2 and 4 to 7, wherein R' is -CH₃.
12. The compound according to any one of items 1 to 2 and 4 to 7, wherein R' is -N(CH₃)₂.
13. The compound according to any one of items 1 to 2 and 4 to 7, wherein R' is -O-(C₁₋C₃ alkyl).
14. The compound according to any one of items 1 to 2 and 4 to 7, wherein R' is -O-(C₁ alkyl).
15. The compound according to any one of items 1 to 2 and 4 to 7, wherein R' is -O-(C₂ alkyl).
16. The compound according to any one of items 1 to 2 and 4 to 7, wherein R' is -O-(C₃ alkyl).
17. The compound according to any one of items 1 to 2 and 4 to 7, wherein R' is -O(CF₃).
18. The compound according to any one of items 1 to 2 and 4 to 7, wherein R' is - F.
19. The compound according to any one of items 1 to 2 and 4 to 7, wherein R¹ is - Cl.
20. The compound according to any one of items 1 to 2 and 4 to 7, wherein R' is - Br.
21. The compound according to any one of items 1 to 2 and 4 to 7, wherein R' is -I.
22. The compound according to any one of items 1 to 2 and 4 to 7, wherein R' is -CN.
23. The compound according to any one of items 1 to 2 and 4 to 7, wherein R' is -CF₃.
24. The compound according to any one of items 1 to 2 and 4 to 7, wherein R' is -C₁-C₃ alkyl.
25. The compound according to any one of items 1 to 2 and 4 to 7, wherein R' is -C₁ alkyl.
26. The compound according to any one of items 1 to 2 and 4 to 7, wherein R' is -C₂ alkyl.
27. The compound according to any one of items 1 to 2 and 4 to 7, wherein R' is -C₃ alkyl.
28. The compound according to any one of items 1 to 2 and 4 to 7, wherein R' is -C(=O)O-(C₁-C₃ alkyl).
29. The compound according to any one of items 1 to 2 and 4 to 7, wherein R' is -C(=O)O-(C₁ alkyl).
30. The compound according to any one of items 1 to 2 and 4 to 7, wherein R' is -C(=O)O-(C₂ alkyl).
31. The compound according to any one of items 1 to 2 and 4 to 7, wherein R' is -C(=O)O-(C₃ alkyl).
32. The compound according to any one of items 1 to 2 and 4 to 7, wherein R' is -N(R^{1a})(R^{1b}), wherein R^{1a} is C₁ alkyl, C₂ alkyl or C₃ alkyl and R^{1b} is C₁ alkyl, C₂ alkyl or C₃ alkyl.
33. The compound according to any one items 1 to 2 and 5, wherein G¹ is according to any one of: 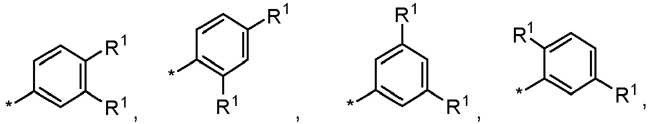 and 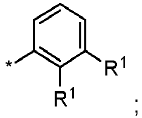 wherein each instance of R' is individually and independently selected from , -F, -Cl, -Br, -I, -CN, -O-(C₁-C₃ alkyl), -CF₃, -OCF₃, -N(R^{1a})(R^{1b}), C₁-C₃ alkyl, -C(=O)O-(C₁-C₃ alkyl), or two R' groups are linked together to form a carbocycle or heterocycle.
34. The compound according to any one of items 1 to 2, 5 and 33, wherein t is 2 and each instance of R' is individually selected from -F, -CN, -O(CH₃), or -C(=O)O-(C₁-C₃ alkyl).
35. The compound according to item 33, wherein each instance is individually selected according to any one of items 10 to 32.
36. The compound according to any one of items 1 to 2, 5 and 33, wherein t is 2 and the two instances of R' are linked together to form a carbocycle or heterocycle.
37. The compound according to any one of 1 to 2, 5 and 33, wherein t is 2 and two instances of R' are linked together to form a carbocycle.
38. The compound according to any one of 1 to 2, 5 and 33, wherein t is 2 and two instances of R' are linked together to form a heterocycle.
39. The compound according to any one of the preceding items, wherein G¹ is selected from any one of the group consisting of: 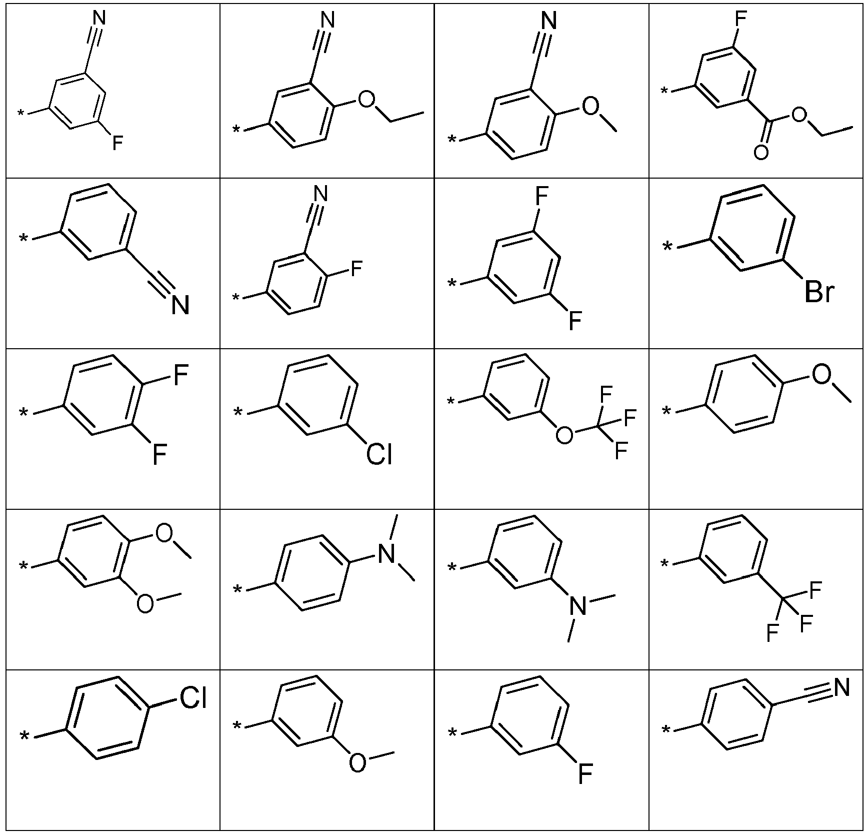 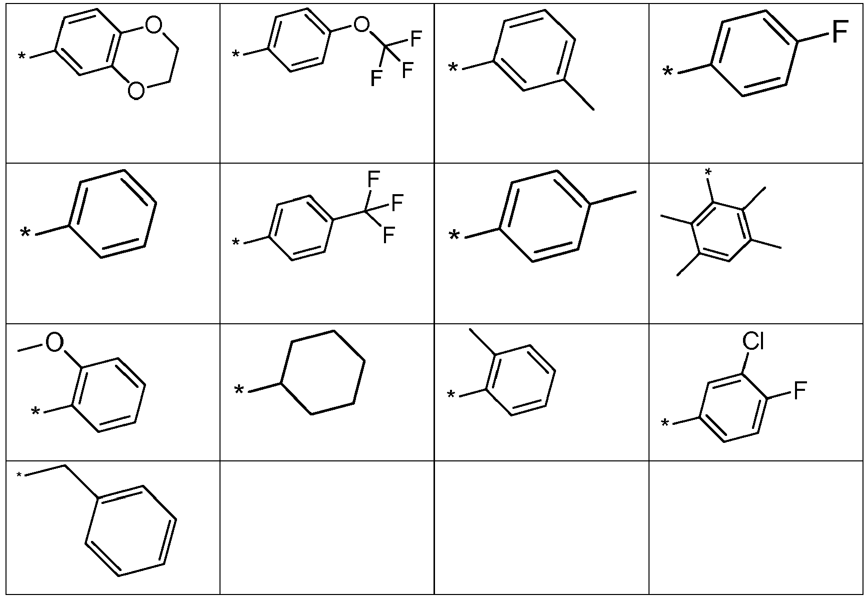
40. The compound according to any one of the preceding items, wherein G¹ is 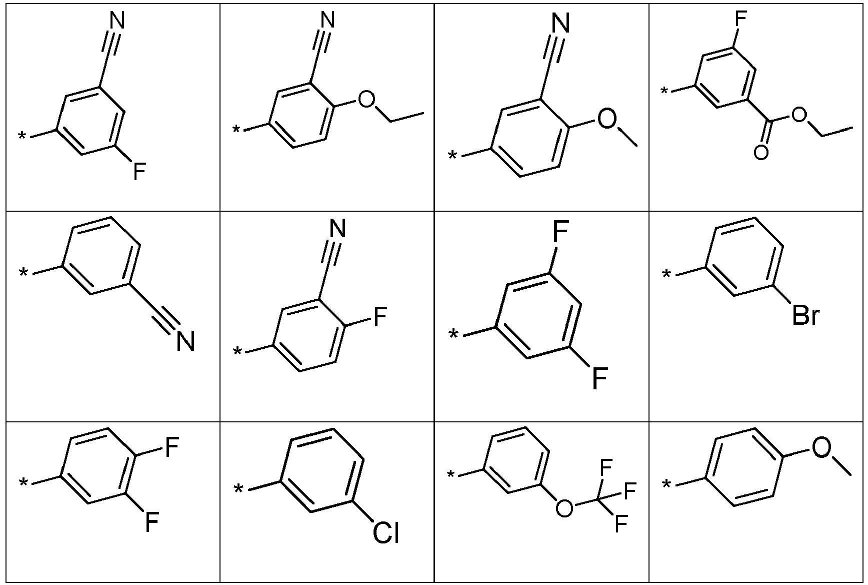 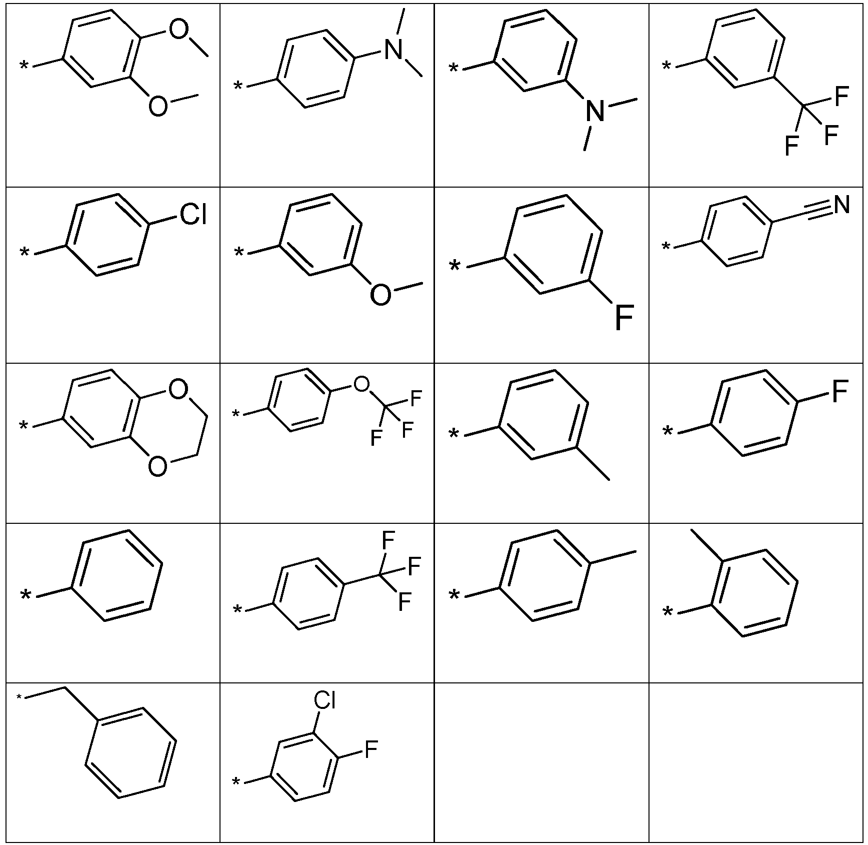
41. The compound according to any one of the preceding items, wherein G¹ is selected from the group consisting of: 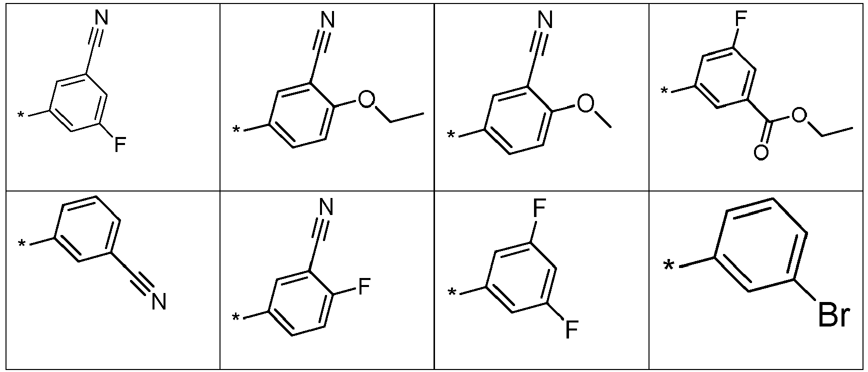 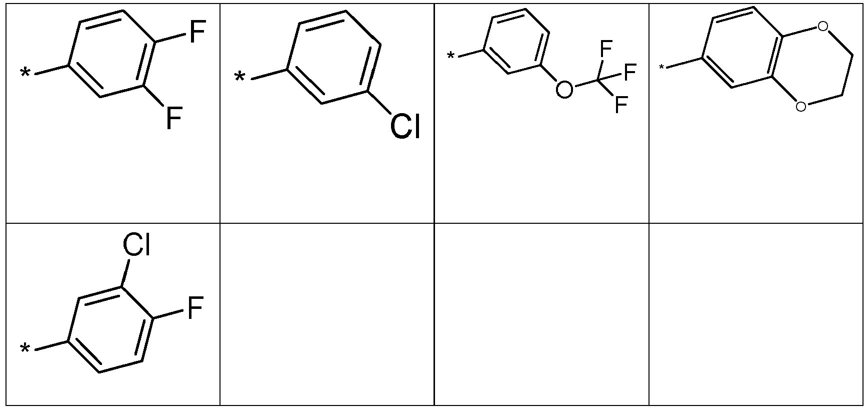
42. The compound according to any one of the preceding items, wherein one of X¹, X² or X³ is N.
43. The compound according to any one of the preceding items, G² is 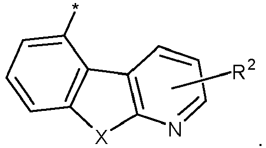
44. The compound according to any one of items 1 to 42, wherein X³ is N, X¹ is CH and X² is CH.
45. The compound according to any one of items 1 to 42, wherein G² is 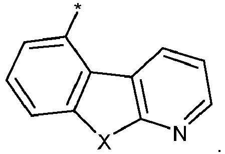
46. The compound according to any one of items 1 to 45, wherein X is O.
47. The compound according to any one of items 1 to 45, wherein X is S.
48. The compound according to any one of items 1 to 45, wherein X is 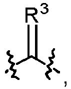 wherein R³ is (=O).
49. The compound according to any one of items 1 to 45, wherein X is 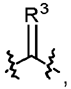 wherein R³ is or (=S).
50. The compound according to any one of items 1 to 45, wherein X is 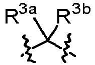 wherein R^{3a} is H, and R^{3b} is H, -OH, -O-(C₁-C₃ alkyl), -O-CF₃, halogen, -S-(C₁ alkyl), or -C₁-C₃ alkyl.
51. The compound according to according to any one of items 1 to 45, wherein X is 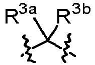 wherein R^{3a} is halogen, and R^{3b} is H, -OH, -O-(C₁-C₃ alkyl), -O-CF₃, halogen, -S-(C₁ alkyl), or -C₁-C₃ alkyl.
52. The compound according to according to any one of items 1 to 45, wherein X is 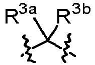 wherein R^{3a} is -O-(C₁ alkyl) or -S-(C₁ alkyl); and R^{3b} is H, -OH, -O-(C₁-C₃ alkyl), -O-CF₃, halogen, -S-(C₁ alkyl), or -C₁-C₃ alkyl.
53. The compound according to according to any one of items 1 to 45, wherein X is 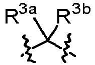 wherein are R^{3a} and R^{3b} are linked together to form a carbocycle or heterocycle.
54. The compound according to according to any one of items 1 to 45, wherein X is 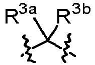 wherein are R^{3a} and R^{3b} are linked together to form a carbocycle.
55. The compound according to according to any one of items 1 to 45, wherein X is 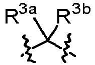 wherein are R^{3a} and R^{3b} are linked together to form a heterocycle.
56. The compound according to any one of items 1 to 45, wherein X is 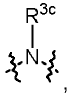 wherein R^{3c} is H, C₁-C₃ alkyl, or -CF₃.
57. The compound according to any one of items 1 to 45, wherein X is 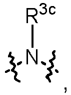 wherein R^{3c} C₁-C₃ alkyl, such as C₁ alkyl, C₂ alkyl, or C₃ alkyl.
58. The compound according to any one of items 1 to 45, wherein X is selected from any one of the group consisting of: 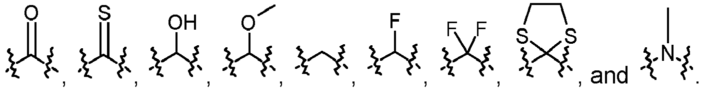
59. The compound according to any one of items 1 to 45, wherein X is 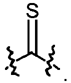
60. The compound according to any one of items 1 to 45, wherein X is 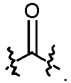
61. The compound according to any one of items 1 to 45, wherein X is 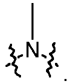
62. The compound according to any one of items 1 to 45, wherein X is 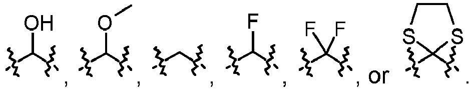
63. The compound according to any one of items 1 to 45, wherein X is 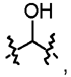 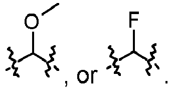
64. The compound according to any one of items 1 to 45, wherein X is 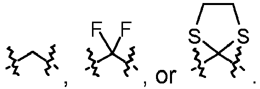
65. The compound according to any one of items 1 to 45, wherein X is 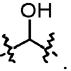
66. The compound according to any one of items 1 to 45, wherein X is 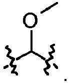
67. The compound according to any one of items 1 to 45, wherein X is 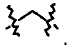
68. The compound according to any one of items 1 to 45, wherein X is 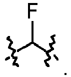
69. The compound according to any one of items 1 to 45, wherein X is 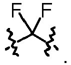
70. The compound according to any one of items 1 to 45, wherein X is 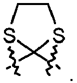
71. The compound according to any one of items 1 to 41, wherein G² is any one of: 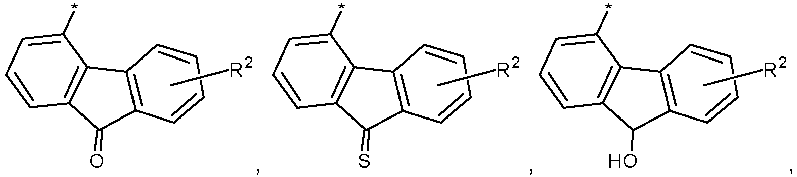 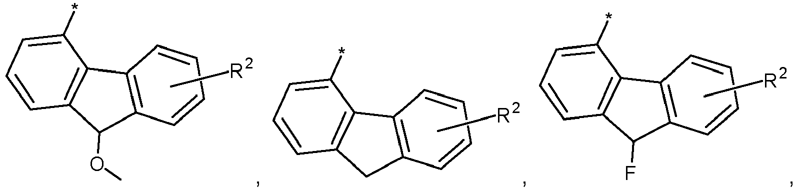 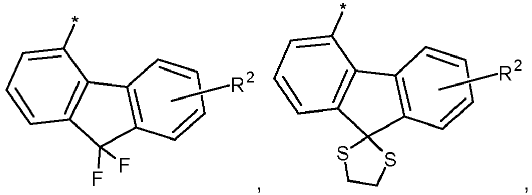 or 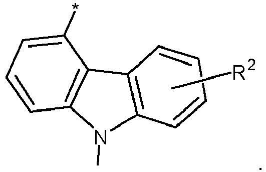
72. The compound according to any one of items 1 to 71, wherein R² is -F, -Cl, - CH₃, or -O-CH₃.
73. The compound according to any one of items 1 to 71, wherein R² is H.
74. The compound according to any one item 73, wherein G² is any one of the group consisting of: 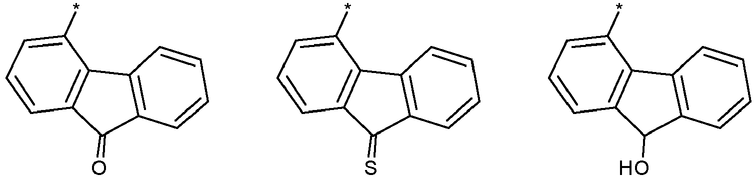 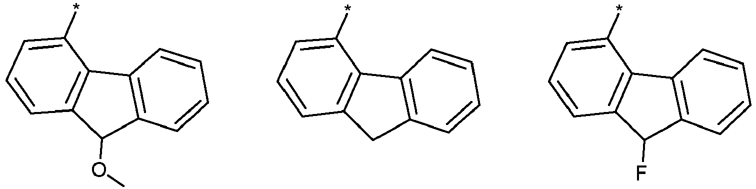 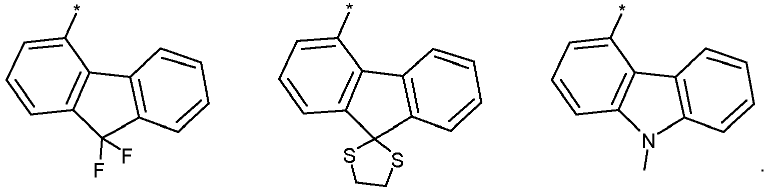
75. The compound according to any one of items 1 to 74, wherein R⁴ is H.
76. The compound according to any one of items 1 to 74, wherein R⁴ is C₁ alkyl.
77. The compound according to any one of items 1 to 74, wherein R⁴ is C₂ alkyl.
78. The compound according to any one of items 1 to 74, wherein R⁴ is C₃ alkyl.
79. The compound according to any one of items 1 to 76, wherein R⁵ is H.
80. The compound according to any one of items 1 to 76, wherein R⁵ is F.
81. The compound according to any one of items 1 to 80, wherein n is 0.
82. The compound according to any one of items 1 to 81, wherein m is 0.
83. The compound according to any one of items 1 to 82, wherein q is 0.
84. The compound according to any one of items 1 to 83, wherein p is 0.
85. The compound according to any one of items 1 to 80 and 82 to 84, wherein n is 1.
86. The compound according to any one of items 1 to 81 and 83 to 85, wherein m is 1.
87. The compound according to any one of items 1 to 82 and 84 to 86, wherein q is 1.
88. The compound according to any one of items 1 to 83 and 85 to 87, wherein p is 1.
89. The compound according to any one of items 1 to 80, wherein n, m, and q are 0 and p is 1.
90. The compound according to any one of items 1 to 80, wherein n is 1, m is 1; p is 1 and q is 0.
91. The compound according to any one of items 1 to 80, wherein n, m, p and q are 0.
92. The compound according to any one of items 1 to 80, wherein n, m, p and q are 1.
93. The compound according to any one of items 1 to 80, wherein n and p are 1; and m and q are 0.
94. The compound according to any one of items 1 to 80, wherein n and m are 0; and p and q are 1.
95. The compound according to any one of the preceding items, wherein R⁶ is -OH.
96. The compound according to any one of items 1 to 94, wherein R⁶ is -O-C₁-C₁₀ alkyl.
97. The compound according to any one items 1 to 94, wherein R⁶ is -O-C₃-C₁₀ cycloalkyl.
98. The compound according to any one of items 1 to 94, wherein R⁶ is 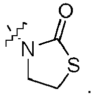
99. The compound according to any one of the preceding items, wherein the compound is any one of the compounds in **Table B** in the detailed description, or a pharmaceutically acceptable salt thereof.
100. The compound according to any one of the preceding items, wherein the wherein the compound is capable of binding to the Keap1 Kelch domain and inhibiting the Keap1-Nrf2 interaction.
101. The compound according to any one of the preceding items, wherein the compound has a Kᵢ of less than 500 µM, such as less than 100 µM, such as less than 50 µM, such as less than 25 µM, such as less than 10 µM, such as less than 5 µM, such as less than 1 µM, such as less than 0.5 µM, such as less than 0.1 µM, such as less than 0.01 µM, e.g as determined by a fluorescence polarization (FP) competition assay.
102. A composition comprising a compound according to any one of the preceding items, or a pharmaceutically acceptable salt thereof, and a pharmaceutically acceptable carrier or excipient.
103. A compound according to any one of items 1 to 101, or a composition according to item 102, according to any one of the preceding items, for use as a medicament.
104. A compound according to any one of items 1 to 101, or a composition according to item 102, for use in treating, ameliorating and/or preventing a disease where reactive oxygen species (ROS) and/or inflammation is involved.
105. The compound, or the composition for use according to item 104, wherein the disease is selected from the group consisting of: a Central Nervous System (CNS) disease; a lung disease; an autoimmune disorder; cancer; a metabolic and fibrotic kidney and liver condition; Alport syndrome; and a skin disease, in a subject.

### Examples

### Example 1: Preparation of compounds

Compound **K1-7, 9-31** and **K49** were designed and synthesized according to **Scheme 1.** The commercially available compound 2-phenylethan-1-amine underwent treatment with trifluoroacetic anhydride (TFAA) to get **K1a.** The introduction of an acetyl group via Friedel-Crafts reaction yielded intermediate **K1b,** which was subsequently cyclized with paraformaldehyde (PFA) under acidic catalysis, providing the 1,2,3,4-tetrahydroquinoline **K1c.** Riley oxidation of ketone **K1c** to **K1d,** followed by acid protection using ethanol, yielded intermediate **K1e** as an α-ketoester. Oxime formation and reduction resulted in **K1g,** which, upon amide coupling with 9-fluorenone-4-carboxylic acid, gave rise to **K1h.** Simultaneous base-induced deprotection of the amine and ethyl ester groups, and reprotection of the carboxylic acid led to intermediate **K1j.** Sulfonylation of **K1j** with various sulfonyl chlorides, followed by hydrolysis of the ester group, produced the final compounds. In some cases (**K3, K27, K29**), sulfonylation was performed with the acidic intermediate **K1i,** instead of **K1j,** to produce the final products directly. 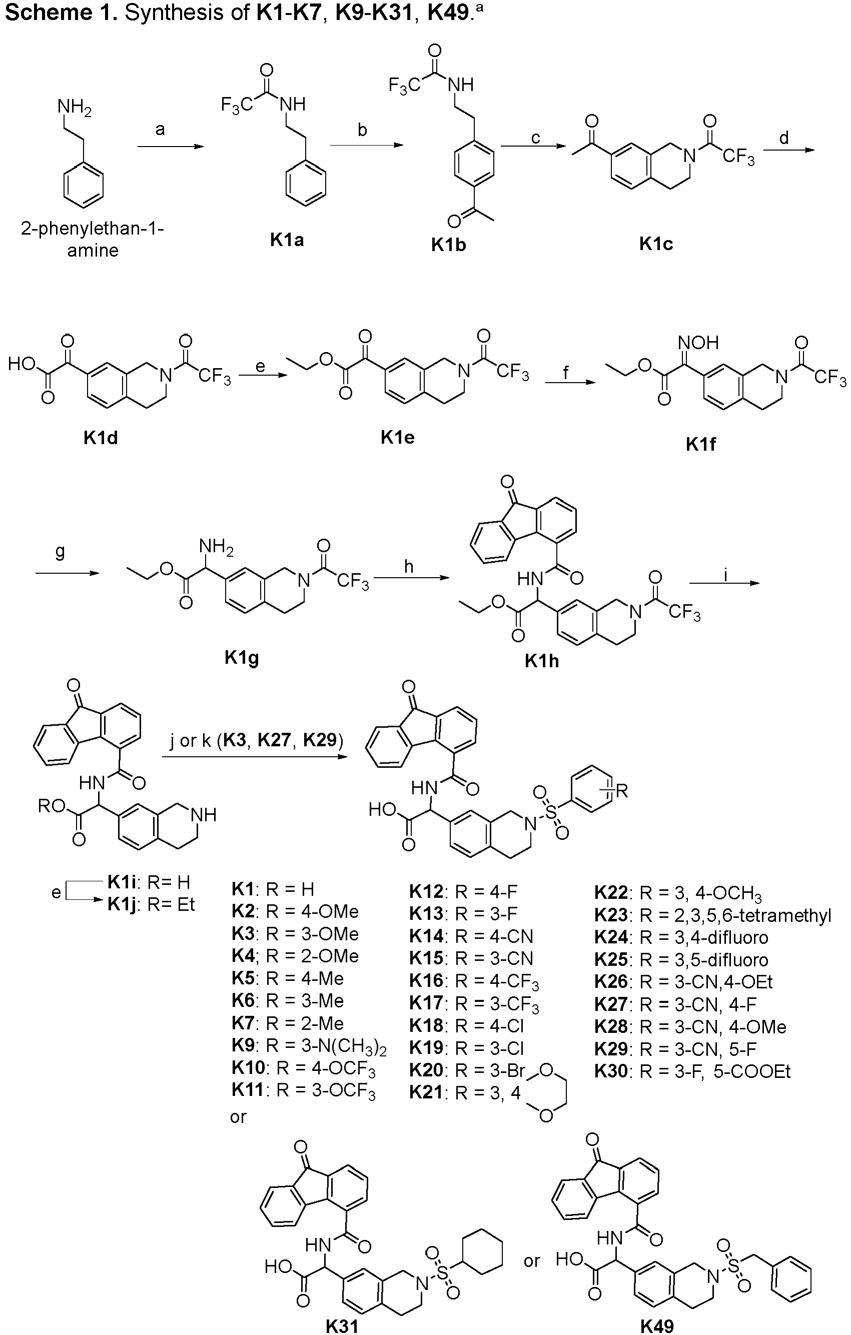 *^{a}*Conditions (Scheme 1) : (a) TFAA, TEA, DCM, RT, overnight, quantitative; (b) AlCl₃, CS₂, acetyl chloride, refluxed, 1 h, 97%; (c) PFA, AcOH, H₂SO₄, RT, 1 h, 15%; (d) SeO₂, Pyridine, 110 °C, 1 h; then 90 °C, 4 h, 18%; (e) TMS-CI, EtOH, RT, overnight, quantitative; (f) NH₂OH HCI, NaOAc, MeOH, 60 °C, 3 h, quantitative; (g) Zn, HCO₂H, MeOH, H₂O, 0 °C to RT, overnight, quantitative; (h) 9-oxo-9H-fluorene-4-carboxylic acid, EDC·HCI, HOBt, DMF, 0 °C, 30 min; then DIPEA, RT, 1-24 h, 68%; (i) aq. NaOH, EtOH, RT, 24 h, quantitative; (j) Sulfonyl chloride, Et₃N, DCM, 50 °C, 3 h, quantitative, then aq. NaOH, EtOH, RT, overnight, 4-82%; (k) Sulfonyl chloride, pyridine, RT, 10 min, 40%.

Compound **K8** was synthesized according to **Scheme 2**. Amine analogue **K8b** was obtained through the reduction of **K8a,** and subsequently, it was transformed into the dimethylamine analogue **K8** using iodomethane. 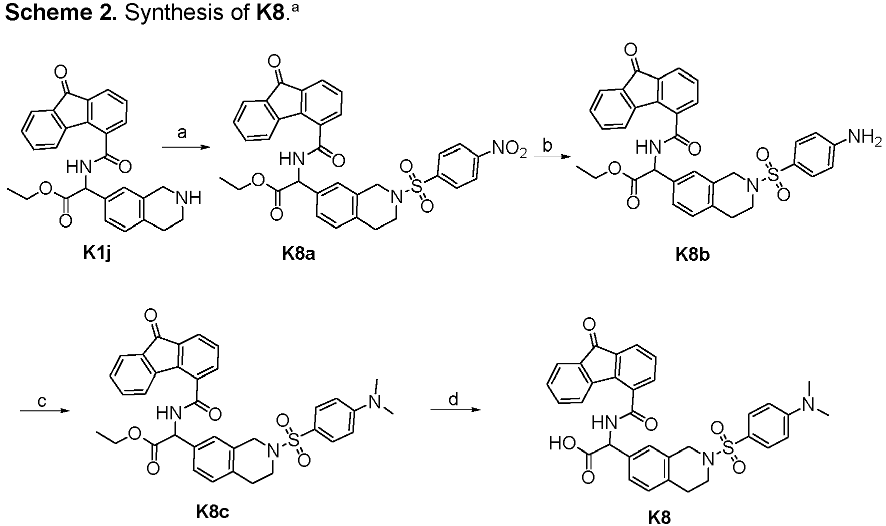 *^{a}*Conditions (Scheme 2): (a) 4-nitrobenzenesulfonyl chloride, Et₃N, DCM, 50 °C, 3 h, 98%; (b) SnCl₂, HCI, 0 °C to RT, 2.5 h, 13%; (c) Mel, Et₃N, DMF, 72 h, Yield: not determined (nd); (d) aq. NaOH, EtOH, RT, overnight, 50%, over two steps.

To explore the SAR of fluorenone moiety, compound **K32-K44** were designed and synthesized (**Scheme 3-12**). Compound **K32a** was obtained through the sulfonylation of **K1j** directly followed by a ketone reduction. Ester hydrolysis resulted in the formation of compound **K32,** where the fluorine is substituted with ethoxy via nucleophilic substitution, as illustrated in **Scheme 3.** 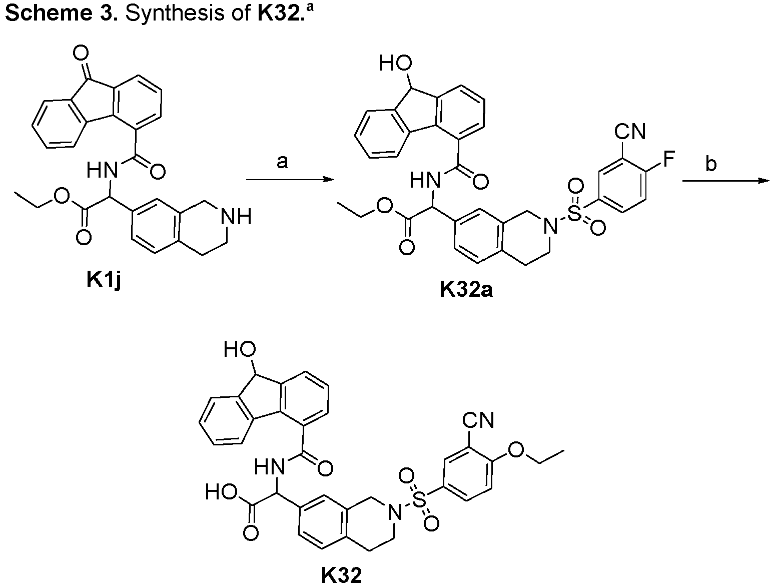 *^{a}*Conditions (Scheme 3): (a) 3-cyano-4-fluorobenzenesulfonyl chloride, Et₃N, DCM, 50 °C, 3 h; NaBH₄, MeOH, RT, 2 h, 84%); (b) aq. NaOH, EtOH, RT, overnight, 9% over two steps.

Compound **K33a** was obtained by reducing **K1j** using sodium borohydride (**Scheme 4**). The introduction of methoxy was facilitated by an acid catalyst, leading to the formation of **K33b.** Following this, sulfonylation and hydrolysis processes were employed, resulting in the production of compound **K33** (**Scheme 4**). 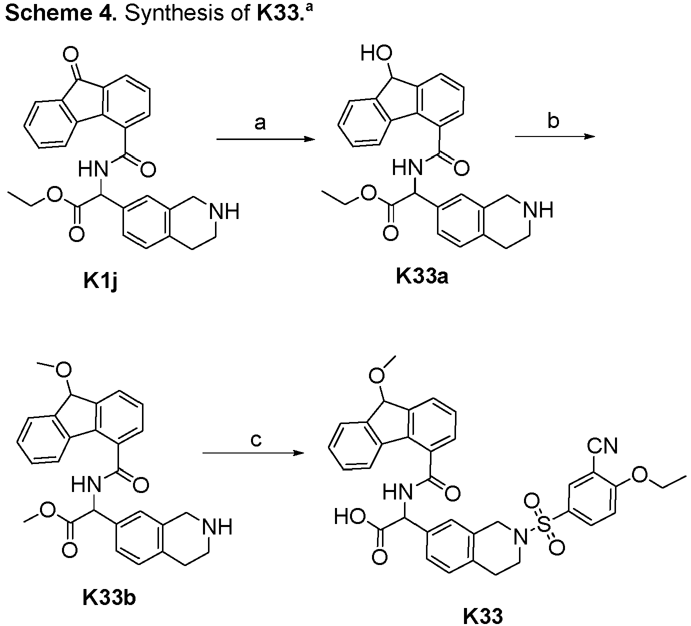 *^{a}*Conditions (Scheme 4): (a) NaBH₄, MeOH, RT, 2 h, 55%; (b) concentrated sulfuric acid, MeOH, RT, 48 h, Yield: nd; (c) 3-cyano-4-fluorobenzenesulfonyl chloride, Et₃N, DCM, 50 °C, 3 h; aq. NaOH, EtOH, RT, overnight, 5% over two steps.

Beginning with **K1j,** the addition of the nucleophilic 1,2-ethanedithiol resulted in the formation of the cyclic thioketal **K34a,** which was then eliminated using Nal and TMS-CI, leading to the generation of fluorene **K34b.** Sulfonylation followed by subsequent hydrolysis yielded compounds **K34** and **K35** (**Scheme 5**). 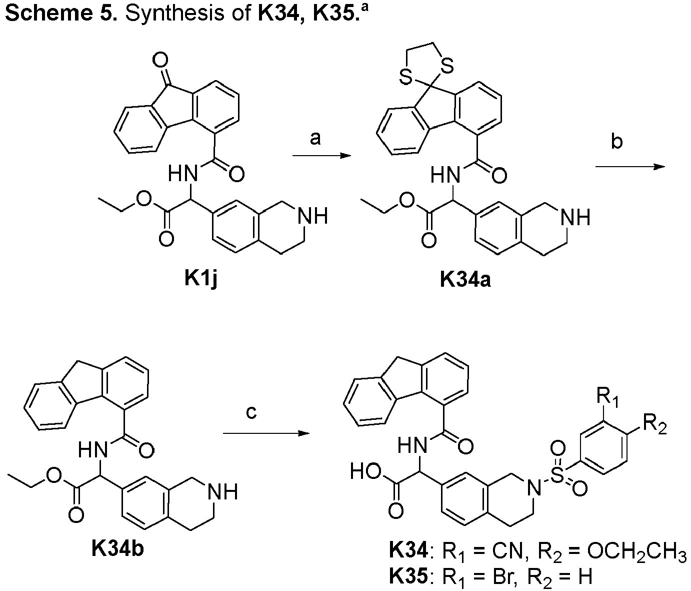 *^{a}*Conditions (Scheme 5): (a) 1,2-ethanedithiol, boron trifluoride etherate, DCM, RT, 2 h, 66%; (b) Nal, TMS-Cl, DCM, RT, 5h, 60%; (c) Sulfonyl chloride, Et₃N, DCM, 50 °C, 3 h; aq. NaOH, EtOH, RT, overnight, 16 or 49%.

The synthesis of **K36** and **K37** was initiated with the ethyl esterification of the commercially available compound 9-oxo-9H-fluorene-4-carboxylic acid, leading to the formation of **K36a.** Subsequent steps comprised a ketone reduction, nucleophilic fluorination of the hydroxyl group by using Deoxo-Fluor, and ester deprotection, resulting in the production of acid **K36d** as a key intermediate. Further synthetic steps involved amide coupling with **K1g,** base-induced deprotection of the trifluoroacetyl and ethyl ester protecting groups, esterification, sulfonylation, and hydrolysis, yielded compounds **K36** and **K37** (**Scheme 6**). 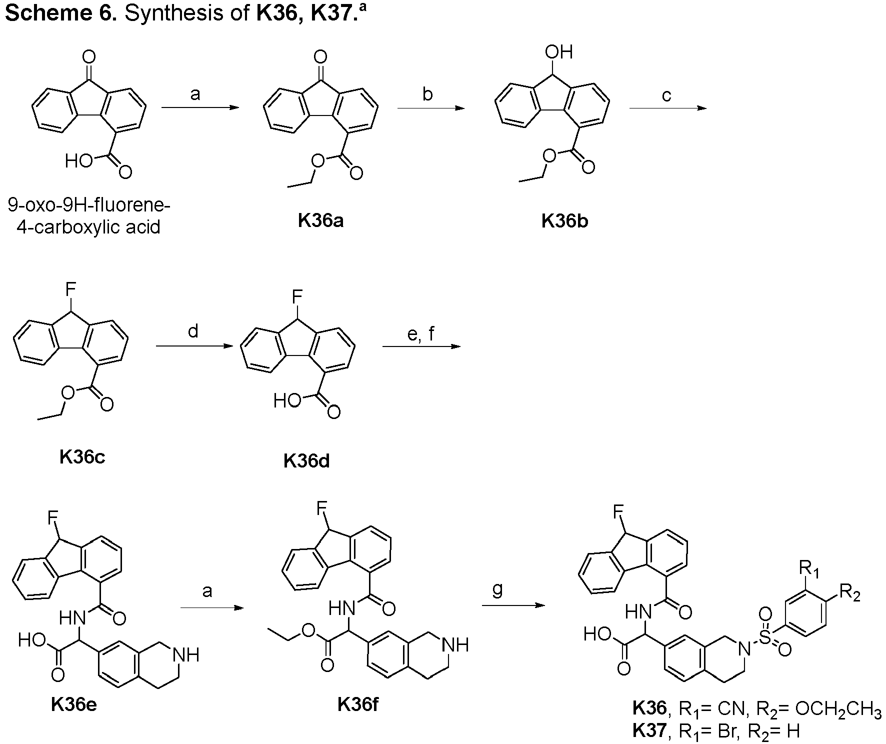 *^{a}*Conditions (Scheme 6): (a) TMS-Cl, EtOH, RT, overnight, quantitative; (b) NaBH₄, MeOH, RT, 2 h, 57%; (c) Deoxo-fluor, DCM, -20 °C; then RT, 30 min, 64%; (d) aq. NaOH, EtOH, RT, 16%. (e) **K1g,** EDC·HCl, HOBt, DMF, 0 °C, 30 min; then DIPEA, RT, overnight, Yield: nd; (f) aq. NaOH, EtOH, RT, 24 h, 68% over two steps; (g) Sulfonyl chloride, Et₃N, DCM, 50 °C, 3 h; aq. NaOH, EtOH, rt overnight, 11 or 16 %.

The thioketal fluorenone analogue **K38** was made from compound **K27** according to **Scheme 7.** The 1,3-dithiolane intermediate **K38a** was synthesized by esterification of **K27** and treatment with 1,2-ethanedithiol followed by base in ethanol for deprotection and nucleophilic substitution (**Scheme 7**). 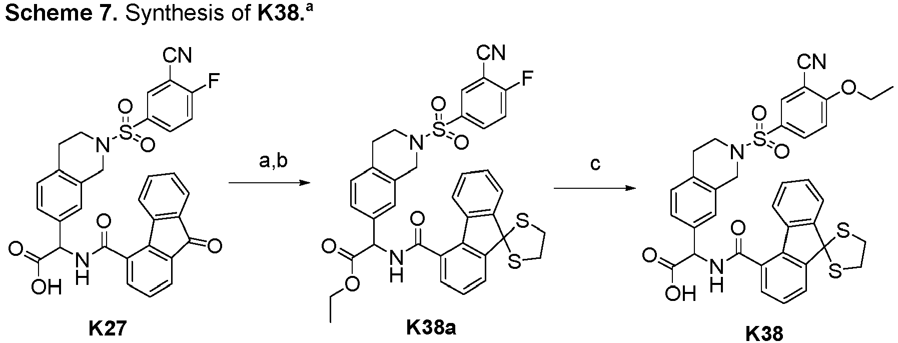 *^{a}*Conditions (Scheme 7): (a) TMS-Cl, EtOH, RT, overnight, quantitative; (b) 1,2-ethanedithiol, boron trifluoride etherate, DCM, RT, 2 h; (c) aq. NaOH, EtOH, RT, 24 h, 7% over two steps.

To make the difluoro-substituted fluorenone compound **K39,** 1,2-ethanedithiol was first applied to ketone **K36a,** resulting in the formation of the cyclic dithioketal **K39a** (**Scheme 8**). Fluorodesulfurization was performed by treating **K39a** with diethylaminosulfur trifluoride (DAST) and *N*-Bromosuccinimide (NBS), and the ethyl ester was converted to acid to reach **K39c.** The subsequent steps shown in **Scheme 8** towards **K39** followed the synthesis route for **K36** and **K37** (**Scheme 6**). 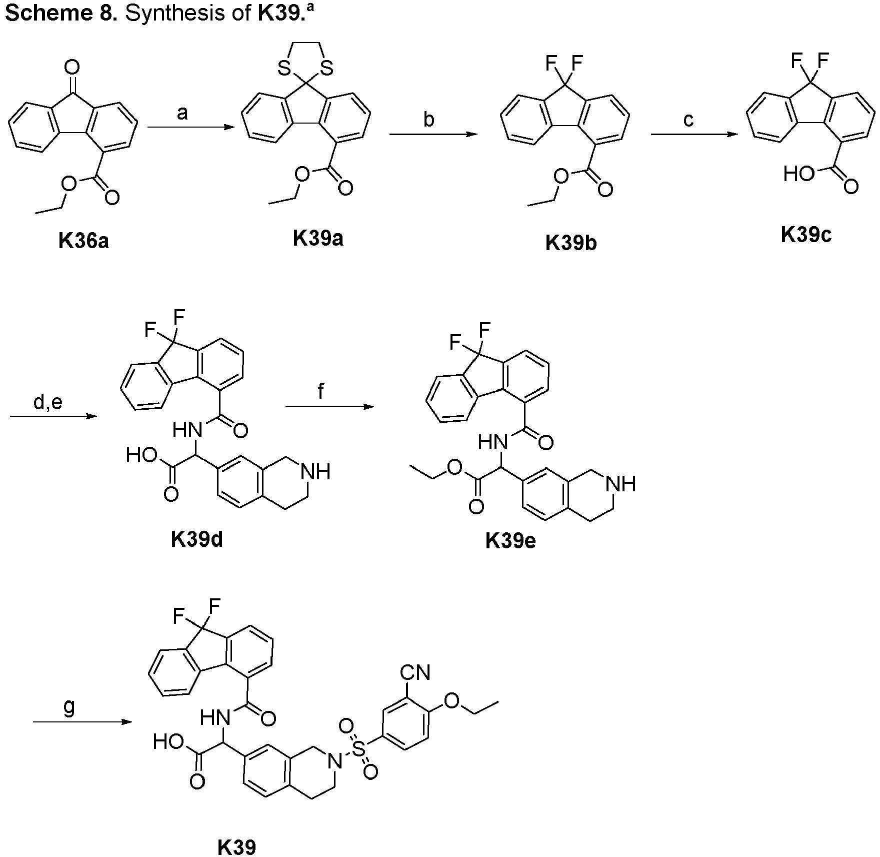 *^{a}*Conditions (Scheme 8): (a) 1,2-ethanedithiol, boron trifluoride etherate, DCM, RT, 2 h, 95%; (b) NBS, DAST, DCM, RT, overnight, quantitative. (c) aq. NaOH, EtOH, RT, 13%; (d) **K1g,** EDC·HCl, HOBt, DMF, 0 °C, 30 min; then DIPEA, RT, overnight, Yield: nd; (e) aq. NaOH, EtOH, RT, 24 h, 57% over two steps; (f) TMS-Cl, EtOH, RT, overnight, quantitative; (g) 3-cyano-4-fluorobenzenesulfonyl chloride, Et₃N, DCM, 50 °C, 3 h; aq. NaOH, EtOH, RT overnight, 13%.

Carbazole analogues **K40, K41, K42, K43,** and **K44** were synthesized following **Scheme 9.** The process was initiated with the aromatization of commercially available methyl 1H-indole-4-carboxylate utilizing 2,5-dimethoxytetrahydrofuran under acidic catalysis. Methylation and ester deprotection led to the formation of the key intermediate **K40c.** Amide coupling with **K1g,** followed by basic deprotection of the amine and acid, resulted in the formation of **K40d.** Direct sulfonylation of **K40d** with 3-cyano-4-fluorobenzenesulfonyl chloride under pyridine catalysis yielded compound **K40.** TMS-Cl-mediated esterification of **K40d** to **K40e,** followed by sulfonylation with respective sulfonyl chlorides under trimethylamine catalyst and subsequent hydrolysis, generated compounds **K41, K42, K43,** and **K44.** 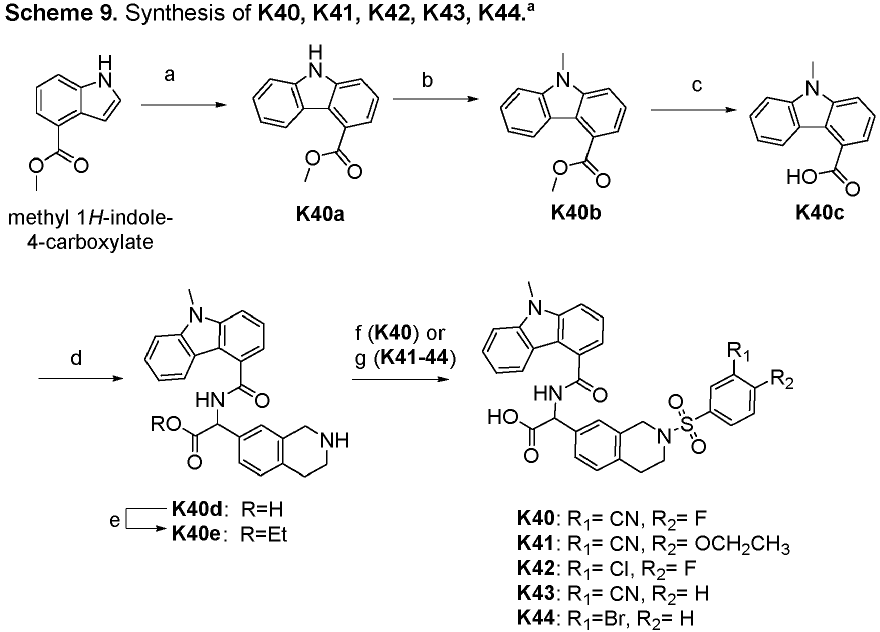 *^{a}*Conditions (Scheme 9): (a) 2,5-dimethoxytetrahydrofuran, TsOH·H₂O, MeOH, 65 °C, 16 h, 5-26%; (b) NaH, Mel, DMF, refluxed, 1 h, 60%; (c) aq. NaOH, EtOH, RT, 24 h, 58%; (d) **K1g,** EDC·HCl, HOBt, DMF, 0 °C, 30 min; then DIPEA, RT, overnight; aq. NaOH, EtOH, RT, 24 h, 45%; (e) TMS-Cl, EtOH, RT, overnight, quantitative; (f) Sulfonyl chloride, pyridine, RT, 10min, 36%; (g) 3-cyano-4-fluorobenzenesulfonyl chloride, Et₃N, DCM, 50 °C, 3 h, quantitative, then aq. NaOH, EtOH, RT, 6-24 h, 19-44%.

Compounds **K45, K46, K47,** and **K48** were synthesized following **Schemes 10-13.** Sulfonylation of commercially available 7-bromo-1,2,3,4-tetrahydroisoquinoline with benzenesulfonyl chloride or phenylmethanesulfonyl chloride yielded **K45a** or **K47a,** respectively (**Scheme 10** and **Scheme 12**). Subsequent Heck reaction resulted in the formation of the cinnamate esters **K45b** or **K47b** (**Scheme 10** and **Scheme 12**). Michael addition reactions of **K45b** using nitromethane or hydroxylamine and reduction with zinc resulted in **K45c** (**Scheme 10**) or **K46a** (**Scheme 11**), respectively. Likewise, Michael addition and reduction of **K47b** gave **K47c** (**Scheme 12**) and **K48a** (**Scheme 13**). The final acid products (**K45, K46, K47,** and **K48**) were obtained after amide coupling and subsequent hydrolysis under basic conditions (**Schemes 10-13**). 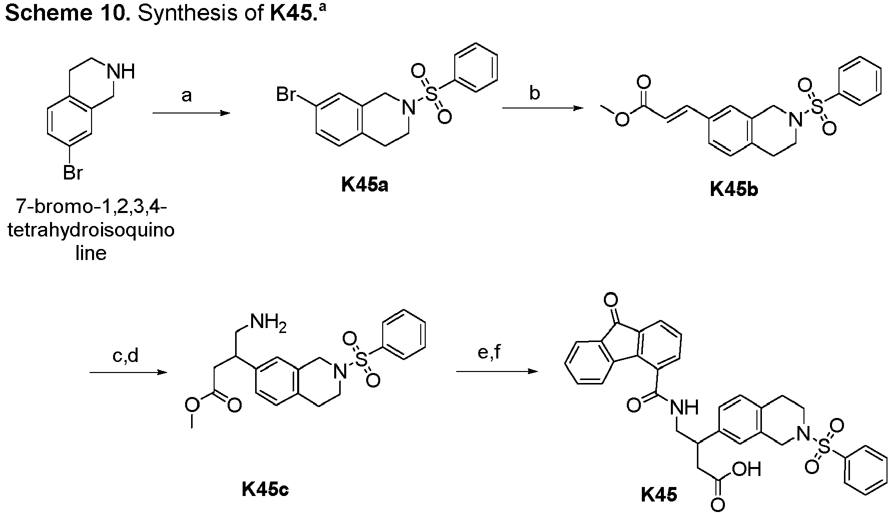 *^{a}*Conditions (Scheme 10): (a) Benzenesulfonyl chloride, Et₃N, DCM, 50 °C, 3 h, quantitative; (b) Methyl acrylate, Pd(OAc)₂, tri(o-tolyl)phosphine, DIPEA, DMF, 100°C, 3 h, 16%; (c) Nitromethane, tetrabutylammonium fluoride, toluene, 50°C, overnight, 86%; (d) Zn, 1 M HCl, MeOH, RT, overnight, 41%; (e) 9-oxo-9H-fluorene-4-carboxylic acid, EDC·HCl, HOBt, DMF, 0 °C, 30 min; then DIPEA, RT, overnight, Yield: nd; (f) aq. NaOH, EtOH, RT, overnight, 35 % over two steps. 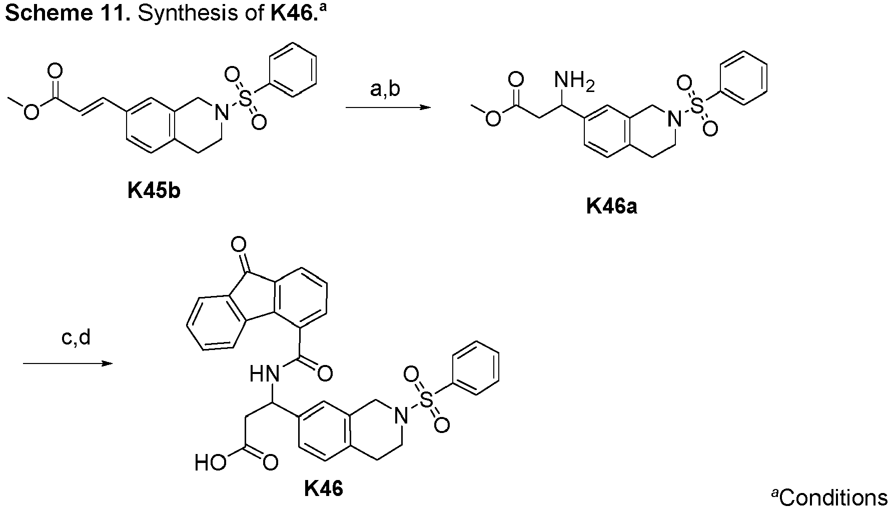 *^{a}*Conditions (Scheme 11): (a) NH₂OH·HCl, NaOAc, tetrabutylammonium hydrogen sulfate, dioxane, reflux, overnight, Yield: nd; (b) Zn, HCO₂H, MeOH, H₂O, 0 °C to RT, overnight, 29% over two steps; (c) 9-oxo-9H-fluorene-4-carboxylic acid, EDC·HCl, HOBt, DMF, 0 °C, 30 min; then DIPEA, RT, overnight, Yield: nd; (d) aq. NaOH, EtOH, RT, overnight, 3% over four steps. 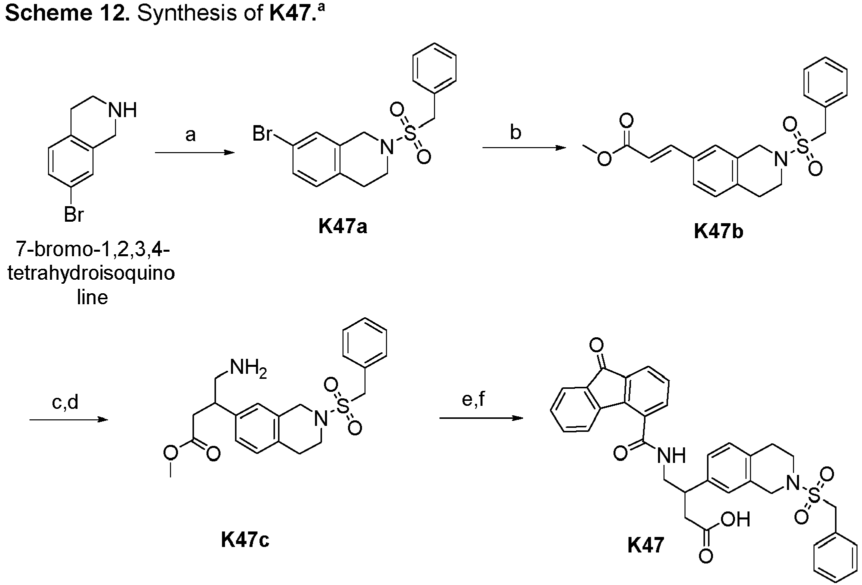 *^{a}*Conditions (Scheme 12): (a) Phenylmethanesulfonyl chloride, Et₃N, DCM, 50 °C, 3 h, 73%; (b) Methyl acrylate, Pd(OAc)₂, tri(o-tolyl)phosphine, DIPEA, DMF, 100°C, 3 h, 16%; (c) Nitromethane, tetrabutylammonium fluoride, toluene, 50°C, overnight, Yield: nd; (d) Zn, 1 M HCl, MeOH, RT, overnight, Yield: nd; (e) 9-oxo-9H-fluorene-4-carboxylic acid, EDC·HCl, HOBt, DMF, 0 °C, 30 min; then DIPEA, RT, overnight, Yield: nd; (f) aq. NaOH, EtOH, RT, overnight, 2 % over four steps. 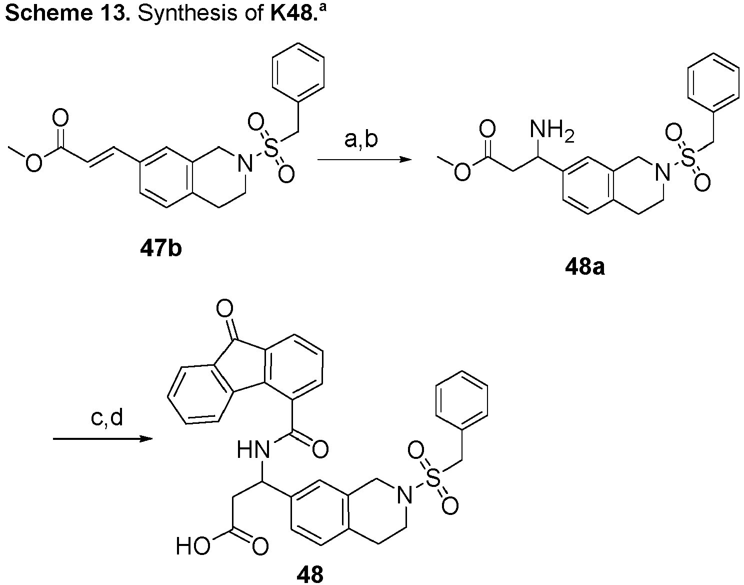 *^{a}*Conditions (Scheme 13): (a) NH₂OH·HCl, NaOAc, tetrabutylammonium hydrogen sulfate, dioxane, reflux, overnight, Yield: nd; (b) Zn, HCO₂H, MeOH, H₂O, 0 °C to RT, overnight, Yield: nd; (c) 9-oxo-9H-fluorene-4-carboxylic acid, EDC·HCl, HOBt, DMF, 0 °C, 30 min; then DIPEA, RT, overnight, Yield: nd; (d) aq. NaOH, EtOH, RT, overnight, 0.6% over four steps.

In order to investigate the impact of ring size, 5-membered-ring analogues **K50** and **K51** were synthesized according to **Scheme 14.** Beginning with the protection of isoindoline using TFAA, compound **K50a** next underwent acetylation through a Friedel-Crafts reaction to yield **K50b.** Subsequent oxidation of **K50b** with selenium dioxide produced the α-ketoacid **K50c.** Ester protection, oxime formation, reduction, amide coupling, and base-catalyzed deprotection were employed to generate compound **K50h.** Compound **K50** was obtained through sulfonylation of **K50h** with 3-cyano-4-fluorobenzenesulfonyl chloride followed by a nucleophilic substitution reaction with ethanol. Sulfonylation of **K50h** with pyridine and 4-methoxybenzenesulfonyl chloride resulted in the formation of compound **K51.** 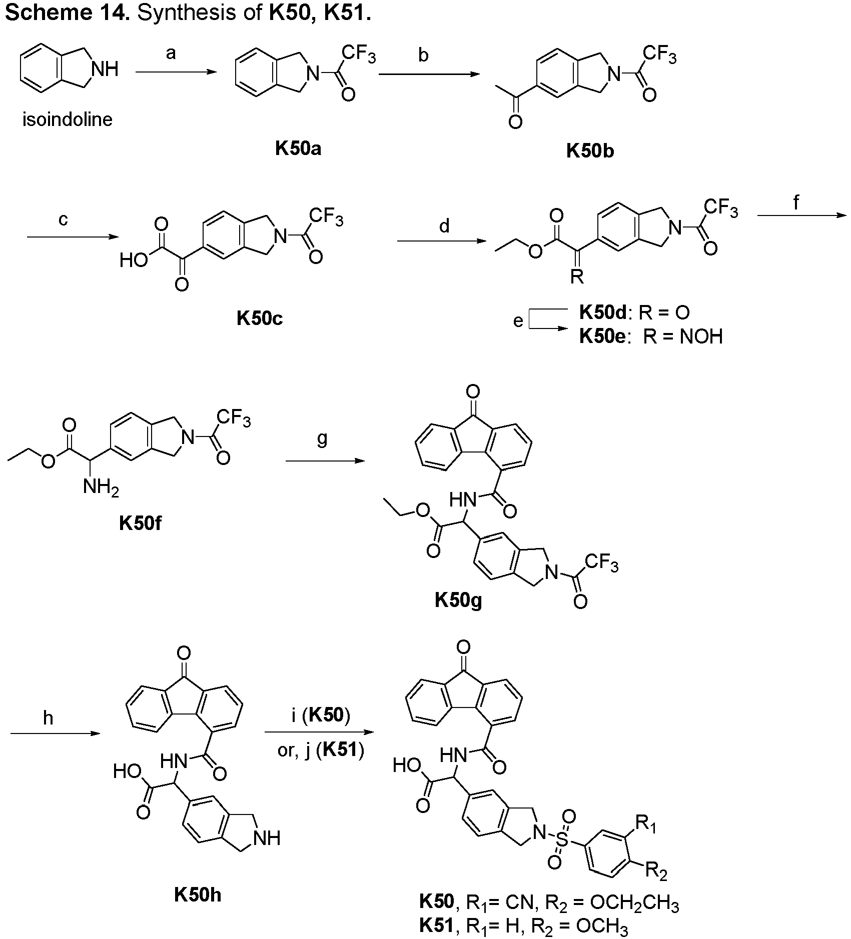 *^{a}*Conditions (Scheme 14): (a) TFAA, TEA, DCM, RT, overnight, quantitative; (b) AlCl₃, CS₂, acetyl chloride, refluxed, 1 h, 39%; (c) SeO₂, Pyridine, 110 °C, 1 h; then 90 °C 4 h, 85%; (d) TMS-Cl, EtOH, RT, overnight, quantitative; (e) NH₂OH·HCl, NaOAc, MeOH, 60 °C, 3 h, 53% over two steps; (f) Zn, HCO₂H, MeOH, H₂O, 0 °C to RT, overnight, quantitative; (g) **K1g,** EDC·HCl, HOBt, DMF, 0 °C, 30 min; then DIPEA, RT, overnight, Yield: nd; (h) aq. NaOH, EtOH, RT, 24 h, 37% over two steps; (i) 3-cyano-4-fluorobenzenesulfonyl chloride, pyridine, RT, 10 min; aq. NaOH, EtOH, RT, overnight, 3%; (j) 4-methoxybenzenesulfonyl chloride, pyridine, RT, 10 min, 15%.

The synthesis of compound **K52** commenced with commercially available methyl 3-bromopicolinate and involved a series of reactions. This included a Suzuki-Miyaura coupling, intramolecular Friedel-Crafts acylation, and oxidation of the methyl group in **K52b** to generate **K52c** as an essential acid building block. This acid was subsequently employed in an amide coupling with **K1g** and basic deprotection to get **K52d.** The subsequent steps leading to the final compound **K52** followed a similar route as that employed for the fluorenone scaffold analogues (**Scheme 15**). 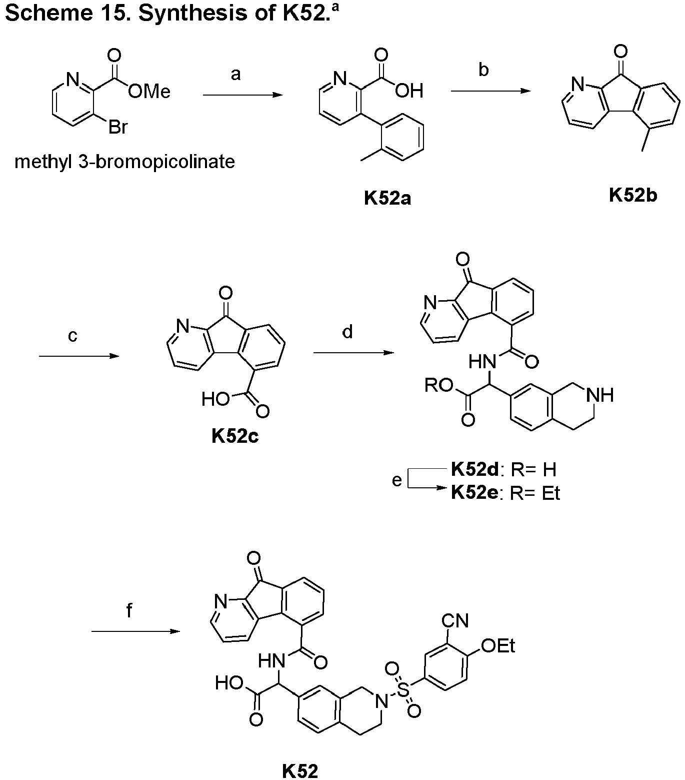 *^{a}*Conditions (Scheme 15): (a) o-tolylboronic acid, Pd(OAc)₂, dppf, K₃PO₄, DME, 80 °C, overnight, 63%; (b) SOCl₂, RT, 6 h; AlCl₃, DCM, RT, overnight, 67%; (c) KMnO₄, pyridine: H₂O (1:1), reflux, overnight, 4%; (d) **K1g,** EDC·HCl, HOBt, DMF, 0 °C, 30 min; then DIPEA, RT, 4 h; aq. NaOH, EtOH, RT, 24 h, quantitative; (e) TMS-Cl, EtOH, RT, overnight, quantitative; (f) 3-cyano-4-fluorobenzenesulfonyl chloride, Et₃N, DCM, 50 °C, 3 h, Yield: nd; then aq. NaOH, EtOH, RT, overnight, 14%.

The methyl ester prodrug **K53** was synthesized through TMSCI-mediated esterification of compound **K26** (**Scheme 16**). 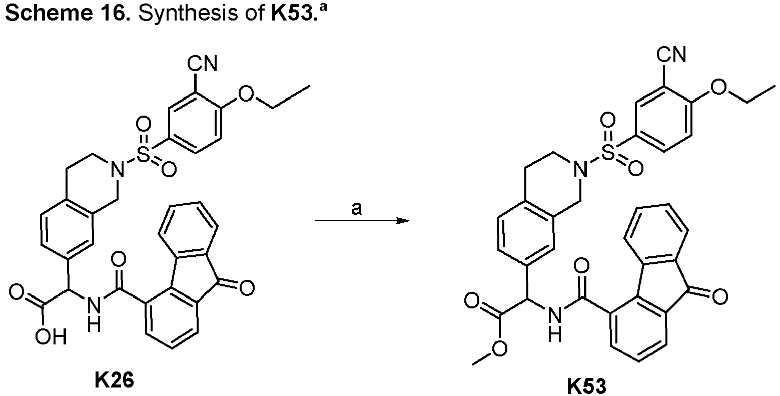 *^{a}*Conditions (Scheme 16): (a) TMS-Cl, MeOH, RT, overnight, 17%.

The synthesis of the methyl ester prodrug **K54** was performed by a methyl esterification of **K1i** followed by sulfonylation with 3-cyano-4-fluorobenzenesulfonyl chloride (**Scheme 17**). 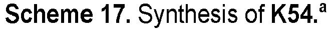 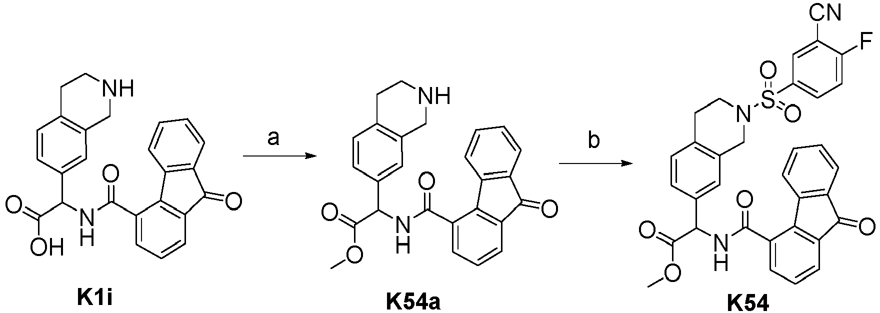 *^{a}*Conditions (Scheme 17): (a) TMS-Cl, MeOH, RT, overnight, quantitative; (b) 3-cyano-4-fluorobenzenesulfonyl chloride, Et₃N, DCM, 50 °C, 1 h, 62%.

**Detailed synthesis protocols for making the compounds are described:**

**Procedures for purification and characterization:** ¹H NMR, ¹³C NMR, and 2D NMR (COSY, HSQC, and HMBC) spectra were obtained using either a 600 MHz Bruker Avance III HD instrument equipped with a cryogenically cooled 5 mm dual probe or a 400 MHz Bruker Avance III instrument equipped with a 5 mm broad band probe. NMR samples were prepared in DMSO-*d*₆. Chemical shift (δ) was reported in parts per million (ppm), while coupling constant (*J*) was reported in Hz. LC-MS analysis was performed on an Agilent 6130 Mass Spectrometer instrument using electron spray ionization (ESI) coupled to an Agilent 1200 HPLC system (ESI-LCMS) with a C18 reverse-phase column (Zorbax Eclipse XBD-C18, 4.6 mm × 50 mm), autosampler and diode array detector, using a linear gradient of the binary solvent system of buffer A (Milli-Q H₂O:MeCN:formic acid, 95:5:0.1 v/v%) to buffer B (MeCN:formic acid, 100:0.1 v/v%) with a flow rate of 1 mL/min for the other compounds. All of the following LC-MS date were collected in positive mode (*mlz* as [M+1]⁺) or negative mode (*mlz* as [M-1]⁻). Normal phase flash chromatography was carried out using prepacked RediSep Rf silica flash cartridges on a CombiFlash^{®} Rf+ apparatus. Reverse-phase flash chromatography was carried out on the CombiFlash^{®} Rf+ apparatus using prepacked RediSep Rf Gold^{®} Reversed-phase C18 cartridge (50 g). Microwave reaction was performed on Biotage^{®} Initiator+ apparatus.

**General procedure A: Amine protection.** To a solution of the amine (1.0 equiv.) in dry DCM (1.5 mL/mmol) was added trifluoroacetic anhydride (1.2 equiv.) and triethylamine (2.0 equiv.) dropwise under the protection of argon. The reaction was stirred at RT for 16 h. Upon reaction completion, the mixture was diluted with DCM (5 mL/mmol), washed with sat. NaHCOs (6 mL/mmol), the organic layer was dried with MgSO₄ and concentrated to give a dryness *in vacuo.* The crude was then purified by normal phase column chromatography (Heptane:EtOAc).

**General procedure B: Friedel-Crafts acylation.** To a stirred slurry of phenethyl (1.0 equiv.) and AlC1₃ (6.0 equiv.) in CS₂ (1.5 mL/mmol) was added acetyl chloride (3.0 equiv.) dropwise. Addition was controlled so that gentle reflux was maintained. After the addition of the acetyl chloride, the reaction mixture was refluxed for 1 h. Excess CS₂ and acetyl chloride were removed *in vacuo,* and the residue was decomposed carefully with of ice-cold 3N HCl (5 mL/mmol) and extracted with DCM. The DCM extract was washed successively with H₂O, 5% NaHC0₃, and H₂O and then was dried MgSO₄ and concentrated. The crude was then purified by normal phase column chromatography (Heptane: EtOAc).

**General procedure C: 1,2,3,4-tetrahydroisoquinoline formation.** To a mixture of the amide (1.0 equiv.) and paraformaldehyde (1.5 equiv.) was added acetic acid (0.81 mL/mmol). The reaction mixture was stirred at room temperature for 5 min before concentrated sulfuric acid (0.81 mL/mmol). An exothermic reaction was observed. The color changed into black. After 30 min, TLC showed a complete conversion. The mixture was cooled to RT before poured onto ice water and extracted with EtOAc (3 × 100 mL). Organic layers were combined and washed with water, sat. NaHCOs and brine, dried over MgSO₄, filtered, evaporated and dried in vacuum. The crude was then purified by normal phase column chromatography (Heptane:EtOAc).

**General procedure D: Oxalate acid synthesis.** Methyl ketone (1.0 equiv.), SeO₂ (1.2 equiv.), and pyridine 4 mL/mmol were added to a round-bottom flask. The reaction mixture was stirred at 110 °C for 1 h in an oil bath, and then the temperature was reduced to 90 °C for 4 h. The products were purified by reverse phase flash chromatography on C18 column to give desired oxalate acid α-ketoacids.

**General procedure E: TMS-Cl-mediated esterification.** TMS-Cl (2.0 equiv.) was slowly added to the acid (1.0 equiv.) followed by MeOH or EtOH (1.0 - 5.0 mL/mmol). The mixture was stirred at RT for overnight. Upon reaction completion, the mixture was concentrated to dryness *in vacuo* and used in the next step without further purification.

**General procedure F: Oxime formation.** A mixture of the appropriate aryl glyoxylate (1.0 equiv.), anhydrous NaOAc (1.2 equiv.) and NH₂OH·HCl (1.6 equiv.) in MeOH (3.5 mL/mmol) was stirred at 60 °C for 3 h. Upon reaction completion, the mixture was concentrated *in vacuo.* The residue was redissolved in EtOAc and washed with water and sat. brine, dried over Na₂SO₄, filtered and concentrated to dryness *in vacuo.*

**General procedure G: Oxime reduction.** To a solution of oxime (1.0 equiv.) and 88% formic acid in methanol and water at 0 °C was added zinc dust (3.0 equiv.) portion-wise over 1 h. The suspension was stirred for 9 - 24 h at room temperature. Upon reaction completion, the mixture was filtered through celite and washed several times with EtOAc or methanol. The filtrate was concentrated. And the compound was used as starting material in next steps without further purification.

**General procedure H: EDC-based amide coupling.** A solution of EDC·HCl (1.5 equiv.) and HOBt (1.5 equiv.) in DMF (2.3 mL/mmol) was added dropwise to a stirred suspension of acid (1.0 equiv.) and amine (1.0 equiv.) in DMF (5.1 mL/mmol) at 0 °C under argon atmosphere for 30 min. DIPEA (2.2 equiv.) in DMF (3.5 mL/mmol) was added dropwise to the mixture, which was then stirred at 25 °C for 24 h. Water (3 mL/mmol) was added and the mixture extracted with EtOAc (3 × 3 mL/mmol). The combined organic layers were washed with 1 M HCl (3 mL/mmol), sat. Na₂CO₃ (3 mL/mmol) and sat. brine (3 mL/mmol), dried over Na₂SO₄, filtered and evaporated to dryness *in vacuo.* And the compound was used as starting material in next steps without further purification.

**General procedure I: Deprotection of amine.** To a solution of trifluoroacetamide (1.0 equiv.) in ethanol (3 mL/mmol) at ice bath was added 6 M NaOH (0.91 mL/mmol). Then the reaction was stirred at RT for 24 h. Upon reaction completion, the mixture was concentrated *in vacuo.* Water was added and the mixture extracted with EtOAc (3 × 3 mL/mmol). The combined organic layers were washed with sat. brine,dried over Na₂SO₄, filtered and evaporated to dryness *in vacuo.* The desired products were purified by reverse phase flash chromatography on C18 column.

**General procedure J: Sulfonamide coupling.** To a solution of the appropriate amine (1.0 equiv.) and Et₃N (3.0 equiv.) in dry DCM under N₂ was added the appropriate sulfonyl chloride (1.0 - 1.2 equiv.). The mixture was stirred at reflux for 3 h. Upon reaction completion, the solvent was evaporated *in vacuo.*

**General procedure K: Alternative sulfonamide coupling.** To a solution of the 1,2,3,4-tetrahydroisoquinolin (1.0 equiv.) in pyridine was added the appropriate benzenesulfonyl chloride (1.0 - 1.5 equiv.). The mixture was stirred at RT for 10 min. Upon reaction completion, the mixture was concentrated to a dryness *in vacuo.* The residue was then extracted with EtOAc. The desired products were purified by reverse phase flash chromatography on C18 column.

**General procedure L: Base-catalyzed ester hydrolysis.** A solution of ester (0.1 - 0.2 M, 1.00 equiv.) in either MeOH or EtOH: H₂O (1:1) was added aq. 1 M or 6 M NaOH (2.0-20.0 equiv.). The mixture was stirred at RT for 10 min to 72 h. mixture was concentrated to dryness *in vacuo.* The desired products were purified by reverse phase flash chromatography on C18 column.

**General procedure M: EDC-based amide coupling and ester deprotection.** A solution of EDC·HCl (1.5 equiv.) and HOBt (1.5 equiv.) in DMF was added dropwise to a stirred suspension of acid (1.0 equiv.) and amine (1.0 equiv.) in DMF at 0 °C under argon atmosphere for 30 min. DIPEA (2.2 equiv.) was added dropwise to the mixture, which was then stirred at 25 °C for another 2 h. Upon reaction completed, 6 M NaOH (2.0 equiv.) was added into the resulting mixture. Then the reaction was stirred at 25 °C for 20 min. Upon reaction completion, the mixture was concentrated *in vacuo.* The desired products were purified by reverse phase flash chromatography on C18 column.

**General procedure N: Ketone reduction.** An ice-bathed solution of ketone (1.0 equiv.) in MeOH (20 mL/mmol) was slowly treated with NaBH₄ (1.5 - 3.0 equiv.). The mixture was stirred at RT for 2 h. Upon reaction completion as detected by TLC or LCMS, the mixture was cooled to 0 °C and quenched with sat. aq. NH₄Cl (6 mL/mmol) and H₂O (6 mL/mmol). The mixture was then partitioned between EtOAc (20 mL/ mmol) and H₂O (20 mL/mmol), the aq. phase extracted with EtOAc (3 × 15 mL/mmol), and the combined organic phases washed with sat. brine (20 mL), dried over Na₂SO₄ and concentrated in vacuo. The crude was purified by normal-phase flash chromatography or reverse phase flash chromatography to afford the hydroxyl product.

**General procedure O: Formation of dithioketal.** To a solution of ketone (1.0 equiv.) in DCM (20 mL/mmol) was added the 1,2-ethanedithiol (5.0 equiv.), followed by boron trifluoride etherate (1 mL/mmol). The solution was stirred at RT for 3h. Then, to the solution was added H₂O (20 mL/mmol) and DCM (3 mL/mmol). The organic layer was separated, washed with H₂O (2 × 1 mL/mmoL) and sat. brine, dried over MgSO₄, and concentrated under reduced pressure to give a residue. The crude product was treated as starting materials in following steps without further purification.

**2,2,2-trifluoro-N-phenethylacetamide (K1a).** The reaction was conducted following the general procedure A, using phenethylamine (1 mL, 7.94 mmol), trifluoroacetic anhydride (1.32 mL, 9.53 mmol) and Et₃N (2.22 mL; 15.9 mmol). Off-white solid (1.69 g, 7.74 mmol, 100%). ¹H NMR (600 MHz, Chloroform-d) δ 7.27 (t, J = 7.5 Hz, 2H), 7.20 (d, J = 7.4 Hz, 1H), 7.14 - 7.10 (m, 2H), 3.55 (q, J = 6.7 Hz, 2H), 2.82 (t, J = 7.0 Hz, 2H). LC-MS (ESI): m/z = 218.1 [M+1]⁺, 435.2 [2M+1]⁺, t_{R} = 4.14 min.

**N-(4-acetylphenethyl)-2,2,2-trifluoroacetamide (K1b).** The reaction was conducted following the general procedure **B,** using **K1a** (8.62 g, 39.71 mmol), aluminum chloride (32 g, 238.2 mmol) and acetyl chloride (9.35 g, 119.1 mmol). White solid (10.735 g, 38 mmol, 97%). ¹H NMR (400 MHz, DMSO-*d*₆) δ 9.50 (t, *J =* 5.8 Hz, 1H), 7.90 (d, *J =* 8.2 Hz, 2H), 7.37 (d, *J* = 8.2 Hz, 2H), 3.46 (q, *J* = 6.8 Hz, 2H), 2.89 (t, *J* = 7.2 Hz, 2H), 2.56 (s, 3H). ¹H NMR (400 MHz, CDCl₃) δ 7.81 (t, 1H), 7.74 (d, 1H), 7.26 (dd, 1H), 4.82 (d, 2H), 3.88 (m, 2H), 3.02 (m, 2H), 2.59 (s, 3H). LC-MS (ESI): m/z = 260.1 [M+1]⁺, 519.2 [2M+1]⁺, t_{R} = 3.92 min.

**1-(7-acetyl-3,4-dihydroisoquinolin-2(1H)-yl)-2,2,2-trifluoroethan-1-one (K1c).** The reaction was conducted following the general procedure **C,** using **K1b** (10 g, 38 mmol), paraformaldehyde (1.7g, 57 mmol), acetic acid (31 mL), concentrated sulfuric acid (31 mL). White solid (1.5 g, 5.6 mmol, 15%). ¹H NMR (400 MHz, DMSO-d6) δ 7.92 (dd, J = 15.0, 1.8 Hz, 1H), 7.80 (td, J = 8.4, 1.9 Hz, 1H), 7.36 (dd, J = 8.0, 3.3 Hz, 1H), 4.85 (d, J = 13.5 Hz, 2H), 3.88 - 3.80 (m, 2H), 2.99 (dt, J = 9.5, 6.1 Hz, 2H), 2.57 (s, 3H). LC-MS (ESI): m/z = 272.0 [M+1]⁺, t_{R} = 4.09 min.

**2-oxo-2-(2-(2,2,2-trifluoroacetyl)-1,2,3,4-tetrahydroisoquinolin-7-yl)acetic acid (K1d).** The reaction was conducted following the general procedure **D,** using **K1c** (249 mg, 0.92 mmol), selenium dioxide (122 mg, 1.1 mmol), 4 mL of pyridine. White solid (50 mg, 0.16 mmol, 18%). ¹H NMR (400 MHz, DMSO-d6) δ 7.86 (d, J = 1.8 Hz, 1H), 7.82 - 7.76 (m, 1H), 7.46 (dd, J = 8.1, 1.7 Hz, 1H), 4.88 (d, J = 15.7 Hz, 2H), 3.85 (q, J = 5.9 Hz, 2H), 3.03 (dt, J = 10.2, 6.0 Hz, 2H). LC-MS (ESI): m/z = 302.1 [M+1]⁺, t_{R} = 3.04 min.

**Ethyl 2-oxo-2-(2-(2,2,2-trifluoroacetyl)-1,2,3,4-tetrahydroisoquinolin-7-yl)acetate (K1e).** The reaction was conducted following the general procedure **E,** using **K1d** (320 mg, 1.06 mmol), TMS-Cl (0.27 mL, 2.1 mmol), 1 mL of ethanol. Oil (280mg, 0.85 mmol, 85%). ¹H NMR (600 MHz, DMSO-d6) δ 7.96 - 7.88 (m, 1H), 7.81 (ddd, J = 11.7, 7.9, 1.9 Hz, 1H), 7.47 (dd, J = 8.0, 2.3 Hz, 1H), 4.87 (d, J = 22.6 Hz, 2H), 4.42 (qd, J = 7.1, 2.1 Hz, 2H), 3.84 (dt, J = 8.8, 5.9 Hz, 2H), 3.03 (dq, J = 14.3, 7.8, 6.9 Hz, 2H), 1.33 (td, J = 7.1, 2.0 Hz, 3H). LC-MS (ESI): m/z = 330.1 [M+1]⁺, t_{R} = 4.48 min.

**Ethyl 2-(hydroxyimino)-2-(2-(2,2,2-trifluoroacetyl)-1,2,3,4-tetrahydroisoquinolin-7-yl)acetate (K1f).** The reaction was conducted following the general procedure F, using **K1e** (349 mg, 1.06 mmol), hydroxylamine hydrochloride (117 mg, 1.70 mmol), anhydrous sodium acetate (101 mg, 1.27 mmol) and methanol (3.5 mL), the crude **K1f** was obtained as colorless oil (360 mg, 1.04 mmol, 98%) and used in next step without further purification. LC-MS (ESI): m/z = 345.1 [M+1]⁺, t_{R} = 3.97 and 4.17 min.

**Ethyl 2-amino-2-(2-(2,2,2-trifluoroacetyl)-1,2,3,4-tetrahydroisoquinolin-7-yl)acetate (K1g).** The reaction was conducted following the general procedure **G,** using crude **K1f** (76 mg, 0.22 mmol), 88% formic acid in methanol (0.418 mL formic acid, 0.22 mL water, 0.44 mL methanol) and zinc dust (218 mg, 0.66 mmol). Crude **K1g** was obtained and used in next step without further purification. LC-MS (ESI): m/z =331.2 [M+1]⁺, t_{R} = 2.88 min. NMR for the acid form was collected. ¹H NMR (600 MHz, DMSO-d6) δ 13.38 (s, 1H), 8.25 (d, J = 7.7 Hz, 1H), 7.77 (d, J = 7.6 Hz, 2H), 7.63 (d, J = 7.3 Hz, 1H), 7.43 (dt, J = 19.4, 7.9 Hz, 3H), 5.59 (s, 1H), 3.08 (s, 3H).

**Ethyl 2-(9-oxo-9H-fluorene-4-carboxamido)-2-(2-(2,2,2-trifluoroacetyl)-1,2,3,4-tetrahydroisoquinolin-7-yl)acetate (K1h)** The reaction was conducted following the general procedure **H,** using EDCI (848 mg, 4.42 mmol), HOBt (597 mg, 4.42 mmol), **K1g** (973 mg, 2.95 mmol), 9-oxo-9H-fluorene-4-carboxylic acid (661 mg, 2.95 mmol) and DIPEA (838.8 mg, 6.50 mmol). Colorless oil (1.07g, 1.99 mmol, 68%). ¹H NMR (400 MHz, DMSO-*d*₆) δ 9.57 (dd, *J* = 6.8, 3.0 Hz, 1H), 7.73 (ddt, *J* = 9.3, 7.3, 0.9 Hz, 2H), 7.69 - 7.62 (m, 1H), 7.58 (dd, *J* = 7.7, 1.2 Hz, 1H), 7.51 (m, 1H), 7.50 - 7.32 (m, 4H), 7.30 - 7.22 (m, 1H), 5.67 (dd, *J* = 6.9, 1.4 Hz, 1H), 4.77 (d, *J=* 8.2 Hz, 2H), 4.30 - 4.08 (m, 2H), 3.83 (dd, *J* = 9.8, 5.1 Hz, 2H), 2.94 (dt, *J* = 10.8, 6.0 Hz, 2H), 1.20 (td, *J* = 7.1, 1.8 Hz, 3H). LC-MS (ESI): m/z = 537.2 [M+1]⁺, t_{R} = 4.73 min.

**2-(9-oxo-9H-fluorene-4-carboxamido)-2-(1,2,3,4-tetrahydroisoquinolin-7-yl)acetic acid (K1i)** The reaction was conducted following the general procedure **I,** using **K1h** (1.3 g, 2.42 mmol), 7.26 mL of ethanol and 2.2 mL of 6 M NaOH. Light yellow solid (1 g, 2.42 mmol, 100%). ¹H NMR (600 MHz, DMSO-*d*₆) δ 13.12 (s, 1H), 9.52 (d, *J =* 7.4 Hz, 1H), 9.01 (br, 1H), 7.71 (dt, *J* = 7.3, 2.9 Hz, 2H), 7.66 (d, *J =* 7.3 Hz, 1H), 7.56 (dd, *J* = 7.7, 1.1 Hz, 1H), 7.53 - 7.42 (m, 2H), 7.44 - 7.38 (m, 2H), 7.37 (d, *J =* 1.8 Hz, 1H), 7.27 (d, *J =* 8.1 Hz, 1H), 5.64 (dd, *J* = 7.3, 2.9 Hz, 1H), 4.28 (dd, *J* = 27.0, 14.2 Hz, 2H), 3.41 (s, 2H), 3.01 (t, *J=* 6.4 Hz, 2H). LC-MS (ESI): m/z = 413.1 [M+1]⁺, t_{R} = 2.97 min.

**Ethyl 2-(9-oxo-9H-fluorene-4-carboxamido)-2-(1,2,3,4-tetrahydroisoquinolin-7-yl)acetate (K1j)** The reaction was conducted following the general procedure **E,** using **K1i** (1g, 2.4 mmol), TMS-Cl (0.53 g, 0.61 mL, 4.48 mmol) and 2.42 mL of ethanol. Yellow solid (1.1g, 2.4 mmol, 100%). 1H NMR (400 MHz, DMSO-d6) δ 9.81 (br s, 1H), 9.52 (d, J = 7.3 Hz, 1H), 7.76 - 7.68 (m, 2H), 7.65 (d, J = 7.2 Hz, 1H), 7.57 (dd, J = 7.7, 1.2 Hz, 1H), 7.51 (td, J = 7.6, 1.3 Hz, 1H), 7.47 - 7.35 (m, 4H), 7.25 (d, J = 7.9 Hz, 1H), 5.62 (d, J = 7.2 Hz, 1H), 4.32 - 4.15 (m, 2H), 3.45 (q, J = 7.0 Hz, 2H), 3.35 (t, J = 5.5 Hz, 2H), 3.03 (t, J = 6.2 Hz, 2H), 1.06 (t, J = 7.0 Hz, 3H). LC-MS (ESI): m/z = 441.2 [M+1]⁺, t_{R} = 3.35 min.

**2-(9-oxo-9H-fluorene-4-carboxamido)-2-(2-(phenylsulfonyl)-1,2,3,4-tetrahydroisoquinolin-7-yl)acetic acid (K1)** The reaction was conducted following the general procedure **J,** using **K1j** (38 mg, 0.086 mmol), Et₃N (26 mg, 35 µL, 0.29 mmol), benzenesulfonyl chloride (15 mg, 0.086 mmol) 1mL of dry DCM. The intermediate was obtained as solid. LC-MS (ESI): m/z = 598.2 [M+18]⁺, t_{R} = 4.70 min. The subsequent reaction was conducted in the initial flask according to general procedure L, using ethanol (0.1 mL), water (0.052 mL) and 6 M NaOH (29 µL, 0.172 mmol, 2.00 equiv.). Yellow solid (7mg, 0.013 mmol, 14%). ¹H NMR (600 MHz, DMSO-d6) δ 13.02 (s, 1H), 9.41 (d, J = 7.1 Hz, 1H), 7.86 - 7.81 (m, 2H), 7.74 - 7.68 (m, 3H), 7.68 - 7.60 (m, 3H), 7.56 (dd, J = 7.7, 1.2 Hz, 1H), 7.44 (t, J = 7.6 Hz, 2H), 7.38 (td, J = 7.4, 1.0 Hz, 1H), 7.32 - 7.28 (m, 2H), 7.15 (d, J = 8.5 Hz, 1H), 5.55 (d, J = 7.1 Hz, 1H), 4.20 (d, J = 15.5 Hz, 1H), 4.17 (d, J = 15.5 Hz, 1H), 3.35 - 3.26 (m, 2H), 2.87 (t, J = 6.0 Hz, 2H). ¹³C NMR (151 MHz, DMSO) δ 192.42, 171.58, 167.42, 142.65, 140.36, 135.73, 135.09, 134.18, 133.72, 133.32, 133.23, 133.08, 132.08, 131.70, 129.65, 129.41, 129.14, 128.89, 127.40, 126.57, 126.11, 124.72, 124.11, 123.78, 56.70, 47.30, 43.48, 27.79. LC-MS (ESI): m/z = 553.1 [M+1]⁺, t_{R} = 4.19 min. Purity > 95% (UV).

**2-(2-((4-methoxyphenyl)sulfonyl)-1,2,3,4-tetrahydroisoquinolin-7-yl)-2-(9-oxo-9H-fluorene-4-carboxamido)acetic acid (K2).** The reaction was conducted following the general procedure **J,** using **K1j** (12.5 mg, 0.027 mmol), Et₃N (8 mg, 11 µL, 0.08 mmol), 4-methoxybenzenesulfonyl chloride (6 mg, 0.027 mmol) and 1 mL of dry DCM. The subsequent reaction was conducted in the initial flask according to general procedure **L,** using crude intermediate (assumed as 0.027 mmol), ethanol (35 µL), water (18 µL), 6M NaOH (9 µL, 0.05 mmol, equiv.). Yellow solid (13 mg, 0.022 mmol, 82%). ¹H NMR (600 MHz, DMSO-d6) δ 13.03 (s, 1H), 9.41 (d, J = 7.1 Hz, 1H), 7.79 - 7.74 (m, 2H), 7.70 (t, J = 7.7 Hz, 2H), 7.64 (d, J = 7.2 Hz, 1H), 7.58 - 7.54 (m, 1H), 7.43 (td, J = 7.6, 2.1 Hz, 2H), 7.37 (t, J = 7.4 Hz, 1H), 7.29 (s, 2H), 7.18 - 7.11 (m, 3H), 5.55 (d, J = 7.1 Hz, 1H), 4.18 - 4.09 (m, 2H), 3.84 (s, 3H), 3.25 (tt, J = 9.1, 4.5 Hz, 2H), 2.87 (t, J = 6.1 Hz, 2H). ¹³C NMR (151 MHz, DMSO-d6) δ 192.42, 171.59, 167.43, 162.75, 142.65, 140.36, 135.09, 134.18, 133.72, 133.32, 133.10, 132.09, 131.78, 129.70, 129.64, 129.14, 128.87 (2C), 127.09, 126.53, 126.14, 124.72, 124.11 (2C), 123.77, 114.54 (2C), 56.71, 55.68, 47.36, 43.48, 27.82. LC-MS (ESI): m/z = 583.1 [M+1]⁺, t_{R} = 4.22 min. Purity > 95% (UV).

**2-(2-((3-methoxyphenyl)sulfonyl)-1,2,3,4-tetrahydroisoquinolin-7-yl)-2-(9-oxo-9H-fluorene-4-carboxamido)acetic acid (K3).** The reaction was conducted following the general procedure **K,** using **K1i** (8.6 mg, 0.02 mmol), 3-methoxybenzenesulfonyl chloride (4 mg, 0.02 mmol), pyridine (1 mL). Yellow solid (1.7 mg, 0.003 mmol, 15%). 1H NMR (600 MHz, DMSO-d6) δ 13.01 (s, 1H), 9.41 (d, J = 7.1 Hz, 1H), 7.70 (dd, J = 7.5, 4.7 Hz, 2H), 7.64 (d, J = 7.3 Hz, 1H), 7.55 (tt, J = 8.3, 5.0 Hz, 2H), 7.44 (t, J = 7.5 Hz, 2H), 7.41 - 7.36 (m, 2H), 7.33 - 7.24 (m, 4H), 7.15 (d, J = 7.8 Hz, 1H), 5.55 (d, J = 7.0 Hz, 1H), 4.24 - 4.17 (m, 2H), 3.81 (s, 3H), 3.33 (t, J = 5.9 Hz, 2H), 2.85 (t, J = 5.9 Hz, 2H). 13C NMR (151 MHz, DMSO-d6) δ 192.44, 171.59, 167.44, 159.55, 142.66, 140.38, 137.11, 135.11, 134.21, 134.19, 133.74, 133.33, 133.10, 132.10, 131.78, 130.65, 129.65, 129.15, 128.91, 126.56, 126.14, 124.74, 124.13, 123.80, 119.45, 119.19, 112.13, 56.73, 55.65, 47.33, 43.52, 27.77. LC-MS (ESI): m/z = 583.1 [M+1]⁺, tR = 4.34 min. Purity > 95% (UV).

**2-(2-((2-methoxyphenyl)sulfonyl)-1,2,3,4-tetrahydroisoquinolin-7-yl)-2-(9-oxo-9H-fluorene-4-carboxamido)acetic acid (K4).** The reaction was conducted following the general procedure **J,** using **K1j** (28 mg, 0.065 mmol), Et₃N (20 mg, 30 µL, 0.195 mmol), 2-methoxybenzenesulfonyl chloride (13 mg, 0.065 mmol). The crude intermediate was obtained. LC-MS (ESI): m/z = 628.2 [M+18], t_{R} = 4.61 min. The subsequent reaction was conducted in the initial flask according to general procedure L, using crude intermediate (assumed as 0.065 mmol), ethanol (77 µL) and water (38 µL), 6 M NaOH (21 µL, 0.130 mmol). Yellow solid (13 mg, 0.022 mmol, 82%). ¹H NMR (600 MHz, DMSO-d6) δ 13.07 (s, 1H), 9.41 (d, J = 7.0 Hz, 1H), 7.83 (d, J = 7.8 Hz, 1H), 7.71 (t, J = 6.3 Hz, 2H), 7.65 (d, J = 7.3 Hz, 1H), 7.61 (t, J = 7.9 Hz, 1H), 7.56 (d, J = 7.6 Hz, 1H), 7.45 (q, J = 8.1 Hz, 2H), 7.39 (t, J = 7.5 Hz, 1H), 7.30 (d, J = 11.1 Hz, 2H), 7.15 - 7.07 (m, 3H), 5.56 (d, J = 7.1 Hz, 1H), 4.43 (t, J = 11.5 Hz, 2H), 3.63 (s, 3H), 3.51 (d, J = 6.0 Hz, 2H), 2.66 (t, J = 6.1 Hz, 2H). ¹³C NMR (151 MHz, DMSO-d6) δ 192.45, 171.63, 167.41, 156.58, 142.67, 140.38, 135.13, 134.96, 134.19, 133.75, 132.84, 132.14, 130.68, 129.68, 129.18, 128.92, 126.47, 126.20, 125.75, 124.74, 124.13, 123.81, 120.18, 118.92, 117.71, 112.85, 56.80, 55.67, 46.85, 42.95, 27.51. LC-MS (ESI): m/z = 583.2 [M+1]⁺, t_{R} = 4.10 min. Purity > 95% (UV).

**2-(9-oxo-9H-fluorene-4-carboxamido)-2-(2-tosyl-1,2,3,4-tetrahydroisoquinolin-7-yl)acetic acid (K5).** The reaction was conducted following the general procedure **J,** using **K1j** (30 mg, 0.068 mmol), Et₃N (21 mg, 28 µL, 0.204 mmol), 4-methylbenzenesulfonyl chloride (12 mg, 0.068 mmol) in 2 mL of dry DCM. The crude intermediate was obtained as yellow solid. The subsequent reaction was conducted in the initial flask according to general procedure **L,** using intermediate (assumed as 0.068 mmol), methanol (80 µL), water (40 µL), 6 M NaOH (23 µL, 0.136 mmol). Yellow solid (1.9 mg, 0.003 mmol, 5 %). ¹H NMR (600 MHz, DMSO-d6) δ 13.04 (s, 1H), 9.41 (d, J = 7.1 Hz, 1H), 7.71 (td, J = 7.6, 1.5 Hz, 4H), 7.64 (dt, J = 7.2, 0.9 Hz, 1H), 7.56 (dd, J = 7.7, 1.2 Hz, 1H), 7.45 - 7.42 (m, 4H), 7.37 (td, J = 7.4, 1.0 Hz, 1H), 7.29 (s, 2H), 7.16 (d, J = 7.8 Hz, 1H), 5.55 (d, J = 7.1 Hz, 1H), 4.20 - 4.11 (m, 2H), 3.27 (td, J = 6.2, 3.1 Hz, 2H), 2.87 (t, J = 6.0 Hz, 2H), 2.39 (s,3H). ¹³C NMR (151 MHz, DMSO-d6) δ 192.44, 171.59, 167.43, 143.66, 142.66, 140.38, 135.10, 134.21, 134.19, 133.73, 133.33, 133.11, 132.76, 132.10, 131.75, 129.86 (2C), 129.65, 129.15, 128.89, 127.49 (2C), 126.55, 126.13, 124.73, 124.12, 123.79, 56.72, 47.31, 43.49, 27.86, 20.99. LC-MS (ESI): m/z = 567.2 [M+1]⁺, t_{R} = 4.25 min. Purity > 95% (UV).

**2-(9-oxo-9H-fluorene-4-carboxamido)-2-(2-(m-tolylsulfonyl)-1,2,3,4-tetrahydroisoquinolin-7-yl)acetic acid (K6).** The reaction was conducted following the general procedure **J,** using **K1j** (28 mg), Et₃N (19 mg, 26 µL, 0.191 mmol), 3-methylbenzenesulfonyl chloride (12 mg, 0.063 mmol), 2 mL of dry DCM. The crude product was obtained as yellow solid. LC-MS (ESI): m/z = 595.2 [M+1]⁺, t_{R} = 4.77 min. The subsequent reaction was conducted in the initial flask according to general procedure L using crude intermediate (assumed as 0.063 mmol), methanol (74 µL), water (37 µL), 6 M NaOH (21 µL, 0.126 mmol). Yellow solid (2.4 mg, 0.004 mmol, 7 %). ¹H NMR (600 MHz, DMSO-*d*₆) δ 13.07 (s, 1H), 9.38 (d, *J =* 7.1 Hz, 1H), 7.70 (t, *J* = 7.6 Hz, 2H), 7.63 (d, *J=* 9.2 Hz, 3H), 7.56 (d, *J=* 7.9 Hz, 1H), 7.51 (d, *J=* 4.6 Hz, 2H), 7.43 (d, *J =* 8.1 Hz, 2H), 7.37 (t, *J* = 7.4 Hz, 1H), 7.30 (s, 2H), 7.15 (d, *J =* 7.9 Hz, 1H), 5.54 (d, *J =* 7.1 Hz, 1H), 4.19 (t, *J =* 11.3 Hz, 2H), 3.33 - 3.27 (m, 2H), 2.86 (t, *J* = 6.1 Hz, 2H), 2.40 (d, *J=* 7.7 Hz, 3H). ¹³C NMR (151 MHz, DMSO-d6) δ 192.43, 171.59, 167.39, 142.65, 140.37, 139.26, 135.69, 135.09, 134.36, 134.16, 133.84, 133.73, 133.33, 133.03, 132.13, 131.72, 129.64, 129.22, 129.15, 128.86, 127.55, 126.55, 126.08, 124.72, 124.58, 124.12, 123.78, 56.79, 47.34, 43.51, 27.76, 20.78. LC-MS (ESI): m/z = 567.2 [M+1]⁺, t_{R} = 4.30 min. Purity > 95% (UV).

**2-(9-oxo-9H-fluorene-4-carboxamido)-2-(2-(o-tolylsulfonyl)-1,2,3,4-tetrahydroisoquinolin-7-yl)acetic acid (K7).** The reaction was conducted following the general procedure **J,** using **K1j** (28 mg, 0.063 mmol), Et₃N (19 mg, 26 µL, 0.191 mmol), 2-methylbenzenesulfonyl chloride (12 mg, 0.063 mmol), 2 mL of dry DCM. The intermediate was obtained as yellow solid. LC-MS (ESI): m/z = 595.2 [M+1]⁺, t_{R} = 4.77 min. The subsequent reaction was conducted in the initial flask according to general procedure **L,** using intermediate (assumed as 0.063 mmol), methanol (74 µL) and water (37 µL), 6M NaOH (21 µL, 0.126 mmol). Yellow solid (1.9 mg,0.003 mmol, 5 %). ¹H NMR (600 MHz, DMSO-d6) δ 13.04 (s, 1H), 9.37 (d, J = 8.2 Hz, 1H), 7.89 (dd, J = 7.9, 1.4 Hz, 1H), 7.70 (m, 2H), 7.66 (dd, J = 21.7, 7.4 Hz, 1H), 7.57 (m, 1H), 7.47 - 7.40 (m, 4H), 7.38 (td, J = 7.5, 1.0 Hz, 1H), 7.31 (m, 1H), 7.15 (dd, J = 8.2, 3.2 Hz, 1H), 5.52 (d, J = 7.1 Hz, 1H), 4.18 - 4.11 (m, 2H), 3.30 - 3.22 (m, 2H), 2.87 (d, J = 6.3 Hz, 2H), 2.39 (s, 3H). ¹H NMR (600 MHz, DMSO-*d*₆) δ 13.03 (s, 1H), 9.43 (d, *J=* 7.1 Hz, 1H), 7.89 (d, *J=* 7.9 Hz, 1H), 7.71 (t, *J* = 7.0 Hz, 2H), 7.64 (d, *J=* 7.3 Hz, 1H), 7.57 (d, *J* = 8.2 Hz, 2H), 7.46 - 7.41 (m, 4H), 7.39 (d, *J* = 7.2 Hz, 1H), 7.32 (d, *J* = 8.4 Hz, 2H), 7.19 (d, *J* = 7.8 Hz, 1H), 5.56 (d, *J* = 7.2 Hz, 1H), 4.33 (q, *J* = 15.8 Hz, 2H), 3.48 (s, 2H), 2.86 (s, 2H), 2.56 (s, 3H). ¹³C NMR (151 MHz, DMSO-*d*₆) δ 192.42, 171.59, 167.42, 142.65, 140.37, 137.19, 136.10, 135.09, 134.18, 134.13, 133.71, 133.32, 133.14, 132.90, 132.14, 132.08, 129.64, 129.40, 129.35, 129.13, 129.07, 126.65, 126.49, 126.03, 124.72, 124.12, 123.77, 56.71, 46.29, 42.54, 27.90, 20.00. LC-MS (ESI): m/z = 567.2 [M+1]⁺, t_{R} = 4.76 min. Purity > 95% (UV).

**Ethyl 2-(2-((4-nitrophenyl)sulfonyl)-1,2,3,4-tetrahydroisoquinolin-7-yl)-2-(9-oxo-9H-fluorene-4-carboxamido)acetate (K8a)** The reaction was conducted following the general procedure **J,** using **K1j** (40 mg, 0.091 mmol), Et₃N (28 mg, 40 µL, 0.272 mmol), 4-nitrobenzenesulfonyl chloride (20 mg, 0.091 mmol) and 1mL of dry DCM. Upon reaction completion, the mixture was extracted with EtOAc, washed with 1 M HCl (2 × 1 mL) and sat. brine and concentrated to dryness *in vacuo.* Yellow solid (56 mg, 0.089 mmol, 98%). ¹H NMR (600 MHz, Chloroform-d) δ 8.28 (t, J = 9.2 Hz, 3H), 8.10 (t, J = 7.9 Hz, 1H), 7.98 - 7.90 (m, 2H), 7.64 (d, J = 7.3 Hz, 1H), 7.60 - 7.53 (m, 1H), 7.48 - 7.43 (m, 2H), 7.25 (t, J = 7.5 Hz, 1H), 7.13 (s, 1H), 7.07 (d, J = 7.9 Hz, 1H), 7.03 (d, J = 7.0 Hz, 1H), 5.67 (d, J = 6.8 Hz, 1H), 4.31 - 4.25 (m, 2H), 4.26 - 4.06 (m, 2H), 3.39 (ddt, J = 46.5, 12.1, 6.0 Hz, 2H), 2.89 (q, J = 5.7 Hz, 2H), 1.19 (t, J = 7.0 Hz, 4H). LC-MS (ESI): m/z = 626.2 [M+1]⁺, 643.2 [M+18]⁺, t_{R} = 4.68 min.

**Ethyl 2-(2-((4-aminophenyl)sulfonyl)-1,2,3,4-tetrahydroisoquinolin-7-yl)-2-(9-oxo-9H-fluorene-4-carboxamido)acetate (K8b).** A solution of **K8a** (56 mg, 0.09 mmol, 1.00 equiv.) in EtOH (0.3 mL) was slowly added to a solution of SnCl₂ (59 mg, 0.31 mmol, 3.50 equiv.) in 13% aq. HCl (0.1 mL) under cooling with an ice bath. After 30 min, the reaction mixture was allowed to warm to RT and then stirred for an addition 2 h. Upon reaction completion, the solvent was evaporated under reduced pressure and the residue treated with 2 N KOH and EtOAc. The organic phase was dried and concentrated *in vacuo* to afford the title compound **K8b** (7 mg crude, 0.01 mmol, 13%). LC-MS (ESI): m/z = 596.2 [M+1]⁺, t_{R} = 4.34 min.

**Ethyl 2-(2-((4-(dimethylamino)phenyl)sulfonyl)-1,2,3,4-tetrahydroisoquinolin-7-yl)-2-(9-oxo-9H-fluorene-4-carboxamido)acetate (K8c).** To a solution of **K8b** (7 mg, 0.01 mmol, 1.0 equiv.) in DMF (0.3 mL) was added Et₃N (3.57 mg, 5 µL, 0.035 mmol, 3.00 equiv.) and then CH₃I (5 mg, 0.035 mmol, 3.0 equiv.). The reaction mixture was stirred at RT for 72 h. Upon reaction completion as detected by LCMS, the reaction mixture was quenched with water and extracted with DCM. The combined organic layers were washed with sat. brine, dried over Na₂SO₄, filtered, and concentrated *in vacuo.* The crude product **8c** was used as a starting material without further purification LC-MS (ESI): m/z = 624.2 [M+1]⁺, t_{R} = 4.72 min.

**2-(2-((4-(dimethylamino)phenyl)sulfonyl)-1,2,3,4-tetrahydroisoquinolin-7-yl)-2-(9-oxo-9H-fluorene-4-carboxamido)acetic acid (K8).** The reaction was conducted following the general procedure **L,** using **K8c** (assumed as 0.01 mmol), ethanol (50 µL), water (25 µL) and 6 M NaOH (4 µL, 0.02 mmol). Yellow solid (3 mg, 0.005 mmol, 50%, over two steps). ¹H NMR (600 MHz, DMSO-d6) δ 12.99 (s, 1H), 9.41 (d, J = 7.1 Hz, 1H), 7.69 (dd, J = 13.1, 7.4 Hz, 2H), 7.63 (d, J = 7.3 Hz, 1H), 7.57 (dd, J = 12.6, 8.1 Hz, 3H), 7.43 (q, J = 7.0 Hz, 2H), 7.37 (t, J = 7.4 Hz, 1H), 7.29 (d, J = 9.5 Hz, 2H), 7.15 (d, J = 7.8 Hz, 1H), 6.79 (d, J = 8.7 Hz, 2H), 5.55 (d, J = 7.1 Hz, 1H), 4.12 - 4.02 (m, 2H), 3.19 (t, J = 6.1 Hz, 2H),2.99 (s, 6H), 2.87 (t, J = 5.8 Hz, 2H). ¹³C NMR (151 MHz, DMSO-*d*₆) δ 192.45, 171.61, 167.45, 152.85, 142.67, 140.38, 135.10, 134.19, 134.17, 133.74, 133.34, 133.20, 132.11, 131.98, 129.66, 129.21, 129.16, 128.86, 126.50, 126.17, 124.74, 124.11, 123.79, 119.96, 110.96, 56.73, 47.47, 43.55, 39.58, 27.95. LC-MS (ESI): m/z = 596.2 [M+1]⁺, t_{R} = 4.24 min. Purity > 95% (UV).

**2-(2-((3-(dimethylamino)phenyl)sulfonyl)-1,2,3,4-tetrahydroisoquinolin-7-yl)-2-(9-oxo-9H-fluorene-4-carboxamido)acetic acid (K9).** The reaction was conducted following the general procedure **J,** using **K1j** (45 mg, 0.091 mmol), Et₃N (28 mg, 40 µL, 0.272 mmol), 3-(dimethylamino)benzenesulfonyl chloride (19 mg, 0.091 mmol) in 1 mL of dry DCM. The crude intermediate was obtained as solid. The subsequent reaction was conducted in the initial flask according to general procedure **L** using intermediate (assumed as 0.091 mmol), ethanol (106 µL), water (53 µL) and 6M NaOH (46 µL, 0.273 mmol). Yellow solid (3.2 mg, 0.005 mmol, 6%). ¹H NMR (600 MHz, DMSO-d6) δ 13.02 (s, 1H), 9.40 (d, J = 7.1 Hz, 1H), 7.72 - 7.66 (m, 2H), 7.64 (dd, J = 7.6, 1.2 Hz, 1H), 7.61 - 7.54 (m, 3H), 7.46 - 7.39 (m, 2H), 7.37 (td, J = 7.4, 1.0 Hz, 1H), 7.28 (s, 1H), 7.15 (d, J = 7.8 Hz, 1H), 6.82 - 6.76 (m, 2H), 5.55 (d, J = 7.1 Hz, 1H), 4.11 - 4.03 (m, 2H), 3.19 (t, J = 5.9 Hz, 2H), 3.00 (s, 6H), 2.87 (t, J = 6.1 Hz, 2H). ¹³C NMR (151 MHz, DMSO-d6) δ 192.43, 171.60, 167.42, 152.84, 142.65, 140.36, 135.08, 134.17, 134.14, 133.72, 133.32, 133.18, 132.10, 131.96, 129.64, 129.19, 129.14, 128.84, 126.48, 126.15, 124.72, 124.10, 123.77, 119.95, 110.95, 56.71, 47.45, 43.53, 40.06, 27.93. LC-MS (ESI): m/z = 596.2 [M+1]⁺, t_{R} = 4.27 min. Purity > 95% (UV).

**2-(9-oxo-9H-fluorene-4-carboxamido)-2-(2-((4-(trifluoromethoxy)phenyl)sulfonyl)-1,2,3,4-tetrahydroisoquinolin-7-yl)acetic acid (K10).** The reaction was conducted following the general procedure **J,** using **K1j** (28 mg, 0.063 mmol), Et₃N (19 mg, 26 µL, 0.19 mmol), 4-(trifluoromethoxy)benzenesulfonyl chloride (16 mg, 0.063 mmol), in 1mL of dry DCM. The crude intermediate was obtained as solid. LC-MS (ESI): t_{R} = 4.91 min. The subsequent reaction was conducted in the initial flask according to general procedure L, using intermediate (assumed as 0.063 mmol), ethanol (0.073 mL), water (0.036 mL), 6 M NaOH (21 µL, 0.126 mmol). Yellow solid (5 mg, 0.008 mmol, 12%). ¹H NMR (600 MHz, DMSO-d6) δ 13.04 (s, 1H), 9.41 (d, J = 7.1 Hz, 1H), 8.01 - 7.92 (m, 2H), 7.73 - 7.68 (m, 2H), 7.66 - 7.62 (m, 1H), 7.62 - 7.58 (m, 2H), 7.56 (dd, J = 7.7, 1.2 Hz, 1H), 7.47 - 7.40 (m, 2H), 7.38 (td, J = 7.4, 1.0 Hz, 1H), 7.30 (d, J = 6.8 Hz, 2H), 7.15 (d, J = 8.3 Hz, 1H), 5.55 (d, J = 7.1 Hz, 1H), 4.28 - 4.19 (m, 2H), 3.41 - 3.33 (m, 2H), 2.87 (t, J = 6.0 Hz, 2H). ¹³C NMR (151 MHz, DMSO-*d*₆) δ 192.42, 171.57, 167.42, 151.33, 142.65, 140.37, 135.10, 134.87, 134.28, 134.16, 133.73, 133.33, 132.99, 132.09, 131.60, 130.08, 129.63, 129.14, 128.89, 126.58, 126.12, 124.73, 124.12, 123.77, 121.52, 119.81 (d, *J* = 258.3 Hz), 56.72, 47.27, 43.43, 27.69. LC-MS (ESI): m/z = 637.1 [M+1]⁺, t_{R} = 4.47 min. Purity > 95% (UV).

**2-(9-oxo-9H-fluorene-4-carboxamido)-2-(2-((3-(trifluoromethoxy)phenyl)sulfonyl)-1,2,3,4-tetrahydroisoquinolin-7-yl)acetic acid (K11)** The reaction was conducted following the general procedure **J,** using **K1j** (28 mg, 0.063 mmol), Et₃N (19 mg, 26 µL, 0.19 mmol), 3-(trifluoromethoxy)benzenesulfonyl chloride (16 mg, 0.063 mmol), 1 mL of dry DCM. The crude intermediate was obtained as solid. LC-MS (ESI): m/z = 665.1 [M+1]⁺, t_{R} = 4.90 min. The subsequent reaction was conducted in the initial flask according to general procedure **L,** using the intermediate (assumed as 0.063 mmol, 1.00 equiv.), ethanol (0.073 mL), water (0.036 mL) and 6 M NaOH (21 µL, 0.126 mmol, 2.00 equiv.). Yellow solid (5 mg, 0.008 mmol, 12%). ¹H NMR (600 MHz, DMSO-d6) δ 13.03 (s, 1H), 9.42 (d, J = 7.1 Hz, 1H), 7.88 (dt, J = 7.5, 1.5 Hz, 1H), 7.80 - 7.75 (m, 2H), 7.74 (d, J = 8.6 Hz, 1H), 7.70 (d, J = 7.4 Hz, 2H), 7.64 (d, J = 7.3 Hz, 1H), 7.56 (d, J = 7.7 Hz, 1H), 7.47 - 7.41 (m, 2H), 7.38 (t, J = 7.4 Hz, 1H), 7.31 (d, J = 6.1 Hz, 2H), 7.16 (d, J = 8.4 Hz, 1H), 5.55 (d, J = 7.1 Hz, 1H), 4.30 - 4.21 (m, 2H), 3.35 (m, 2H), 2.87 (t, J = 6.0 Hz, 2H).¹³C NMR (151 MHz, DMSO-*d*₆) δ 192.44, 171.58, 167.44, 148.40, 142.66, 140.39, 138.13, 135.10, 134.28, 134.17, 133.74, 133.34, 132.97, 132.09, 131.92, 131.58, 129.64, 129.15, 128.92, 126.62, 126.57, 126.09, 125.88, 124.74, 124.13, 123.79, 119.94, 119.90 (d, *J* = 257.7 Hz), 56.72, 47.23, 43.46, 27.69. LC-MS (ESI): m/z = 637.1 [M+1]⁺, t_{R} = 4.90 min. Purity > 95% (UV).

**2-(2-((4-fluorophenyl)sulfonyl)-1,2,3,4-tetrahydroisoquinolin-7-yl)-2-(9-oxo-9H-fluorene-4-carboxamido)acetic acid (K12).** The reaction was conducted following the general procedure **J,** using **K1j** (28 mg, 0.063 mmol), Et₃N (19 mg, 26 µL, 0.19 mmol), 4-fluorobenzenesulfonyl chloride (12 mg, 0.063 mmol), 1mL of dry DCM. The crude intermediate was obtained as solid. LC-MS (ESI): t_{R} = 4.68 min. The subsequent reaction was conducted in the initial flask according to general procedure L, using intermediate (assumed as 0.063 mmol), ethanol (0.073 mL), water (0.036 mL), 6 M NaOH (21 µL, 0.126 mmol). Yellow solid (6 mg, 0.010 mmol, 17%). ¹H NMR (600 MHz, DMSO-d6) δ 13.04 (s, 1H), 9.42 (d, J = 7.1 Hz, 1H), 7.94 - 7.88 (m, 2H), 7.73 - 7.68 (m, 2H), 7.64 (dt, J = 7.2, 0.9 Hz, 1H), 7.56 (dd, J = 7.7, 1.2 Hz, 1H), 7.50 - 7.41 (m, 4H), 7.38 (td, J = 7.4, 1.0 Hz, 1H), 7.30 (s, 2H), 7.16 (d, J = 7.8 Hz, 1H), 5.55 (d, J = 7.1 Hz, 1H), 4.24 - 4.15 (m, 2H), 3.37 - 3.27 (m, 2H), 2.87 (t, J = 6.0 Hz, 2H). ¹³C NMR (151 MHz, DMSO-*d*₆) δ 192.42, 171.58, 167.43, 164.62 (d, *J=* 251.7 Hz), 142.65, 140.37, 135.10, 134.21 (d, *J =* 9.5 Hz), 133.73, 133.33, 133.04, 132.19 (d, *J =* 2.9 Hz), 132.09, 131.67, 130.53 (d, *J* = 9.5 Hz), 129.64, 129.14, 128.89, 126.58, 126.12, 124.73, 124.12, 123.78, 116.59 (d, *J* = 22.7 Hz), 56.72, 47.29, 43.45, 40.06, 39.92, 27.73.LC-MS (ESI): m/z = 571.1 [M+1]⁺, t_{R} = 4.19 min. Purity > 95% (UV).

**2-(2-((3-fluorophenyl)sulfonyl)-1,2,3,4-tetrahydroisoquinolin-7-yl)-2-(9-oxo-9H-fluorene-4-carboxamido)acetic acid (K13).** The reaction was conducted following the general procedure **J,** using **K1j** (28 mg, 0.063 mmol), Et₃N (19 mg, 26 µL, 0.19 mmol), 3-fluorobenzenesulfonyl chloride (12 mg, 0.063 mmol), 1mL of dry DCM. The crude intermediate was obtained as solid. LC-MS (ESI): m/z = 599.2 [M+1]⁺, t_{R} = 4.68 min. The subsequent reaction was conducted in the initial flask according to general procedure **L,** using the intermediate (assumed as 0.063 mmol), ethanol (0.073 mL), water (0.036 mL), 6 M NaOH (21 µL, 0.126 mmol). Yellow solid (5.2 mg, 0.009 mmol, 14%). ¹H NMR (600 MHz, DMSO-d6) δ 13.03 (s, 1H), 9.42 (d, J = 7.1 Hz, 1H), 7.69 (qd, J = 8.5, 7.9, 2.3 Hz, 5H), 7.64 (d, J = 7.3 Hz, 1H), 7.62 - 7.54 (m, 2H), 7.44 (td, J = 7.6, 3.3 Hz, 2H), 7.38 (t, J = 7.4 Hz, 1H), 7.31 (d, J = 6.9 Hz, 2H), 7.16 (d, J = 8.2 Hz, 1H), 5.56 (d, J = 7.1 Hz, 1H), 4.28 - 4.19 (m, 2H), 3.35 (d, J = 6.3 Hz, 3H), 2.88 (t, J = 6.0 Hz, 2H). ¹³C NMR (151 MHz, DMSO-d6) δ 192.43, 171.58, 167.44, 161.86 (d, J = 249.2 Hz), 142.66, 140.38, 137.93 (d, J = 6.7 Hz), 135.11, 134.21 (d, J = 7.4 Hz), 133.73, 133.33, 133.04, 132.09, 131.80 (d, J = 8.2 Hz), 131.66, 129.65, 129.15, 128.92, 126.62, 126.11, 124.73, 124.13, 123.79, 123.68 (d, J = 3.1 Hz), 120.42 (d, J = 21.0 Hz), 114.50 (d, J = 24.3 Hz), 56.72, 47.28, 43.49, 27.79. LC-MS (ESI): m/z = 571.1 [M+1]⁺, t_{R} = 4.21 min. Purity > 95% (UV).

**2-(2-((4-cyanophenyl)sulfonyl)-1,2,3,4-tetrahydroisoquinolin-7-yl)-2-(9-oxo-9H-fluorene-4-carboxamido)acetic acid (K14).** The reaction was conducted following the general procedure **J,** using **K1j** (28 mg, 0.063 mmol), Et₃N (19 mg, 26 µL, 0.19 mmol), 4-cyanobenzenesulfonyl chloride (13 mg, 0.063 mmol), 1mL of dry DCM. The crude intermediate was obtained as solid. LC-MS (ESI): t_{R} = 4.55 min. The subsequent reaction was conducted in the initial flask according to general procedure L, using intermediate (assumed as 0.063 mmol), ethanol (0.073 mL), water (0.036 mL), 6 M NaOH (21 µL, 0.126 mmol). Yellow solid (7.7 mg, 0.013 mmol, 21%). ¹H NMR (600 MHz, DMSO-d6) δ 13.03 (s, 1H), 9.40 (d, J = 7.2 Hz, 1H), 8.09 (d, J = 8.1 Hz, 2H), 8.01 (d, J = 8.0 Hz, 2H), 7.70 (t, J = 7.2 Hz, 2H), 7.64 (d, J = 7.3 Hz, 1H), 7.57 (d, J = 7.7 Hz, 1H), 7.44 (t, J = 7.5 Hz, 2H), 7.38 (t, J = 7.4 Hz, 1H), 7.31 (d, J = 9.3 Hz, 2H), 7.15 (d, J = 7.8 Hz, 1H), 5.55 (d, J = 7.1 Hz, 1H), 4.31 - 4.18 (m, 2H), 3.46 (s, 1H), 3.42 - 3.34 (m, 2H), 2.87 (d, J = 6.1 Hz, 2H). ¹³C NMR (151 MHz, DMSO-*d*₆) δ 192.44, 171.60, 167.42, 142.67, 140.39, 140.26, 135.12, 134.43, 134.17, 133.75 (2C), 133.53, 133.34, 132.96, 132.12, 131.55, 129.66, 129.16, 128.92, 128.13 (2C), 126.61, 126.09, 124.74, 124.13, 123.79, 117.61, 115.59, 56.77, 47.18, 43.42, 27.70. LC-MS (ESI): m/z = 578.1 [M+1]⁺, t_{R} = 4. 12 min. Purity > 95% (UV).

**2-(2-((3-cyanophenyl)sulfonyl)-1,2,3,4-tetrahydroisoquinolin-7-yl)-2-(9-oxo-9H-fluorene-4-carboxamido)acetic acid (K15).** The reaction was conducted following the general procedure **J,** using **K1j** (28 mg, 0.063 mmol), Et₃N (19 mg, 26 µL, 0.19 mmol), 3-cyanobenzenesulfonyl chloride (13 mg, 0.063 mmol), in 1mL of dry DCM. The crude intermediate was obtained as solid. The subsequent reaction was conducted in the initial flask according to general procedure **L,** using intermediate (assumed as 0.063 mmol), ethanol (0.073 mL), water (0.036 mL), 6 M NaOH (21 µL, 0.126 mmol). Yellow solid (5.4 mg product, 0.009 mmol, 15%). ¹H NMR (600 MHz, DMSO-d6) δ 13.04 (s, 1H), 9.42 (d, J = 7.1 Hz, 1H), 8.31 (s, 1H), 8.15 (dd, J = 17.5, 7.9 Hz, 2H), 7.83 (d, J = 8.2 Hz, 1H), 7.70 (d, J = 7.6 Hz, 2H), 7.64 (d, J = 7.3 Hz, 1H), 7.56 (d, J = 7.7 Hz, 1H), 7.44 (t, J = 7.1 Hz, 2H), 7.39 (d, J = 7.5 Hz, 1H), 7.31 (d, J = 9.1 Hz, 2H), 7.16 (d, J = 7.8 Hz, 1H), 5.55 (d, J = 7.0 Hz, 1H), 4.26 (s, 2H), 3.33 (s, 7H), 2.88 (d, J = 6.9 Hz, 2H).¹³C NMR (151 MHz, DMSO-d6) δ 192.46, 171.59, 167.47, 142.66, 140.39, 137.33, 136.78, 135.13, 134.24, 134.19, 133.74, 133.34, 133.03, 132.09, 131.82, 131.63, 131.03, 130.83, 129.68, 129.17, 128.94, 126.65, 126.10, 124.76, 124.13, 123.82, 117.49, 112.81, 56.76, 47.27, 43.46, 27.73. LC-MS (ESI): m/z = 578.1 [M+1]⁺, t_{R} = 4. 08 min. Purity > 95% (UV).

**2-(9-oxo-9H-fluorene-4-carboxamido)-2-(2-((4-(trifluoromethyl)phenyl)sulfonyl)-1,2,3,4-tetrahydroisoquinolin-7-yl)acetic acid (K16).** The reaction was conducted following the general procedure **J,** using **K1j** (28 mg, 0.063 mmol), Et₃N (19 mg, 26 µL, 0.19 mmol), 4-(trifluoromethyl)benzenesulfonyl chloride (15 mg, 0.063 mmol), 1mL of dry DCM. The crude intermediate was obtained as solid. LC-MS (ESI): t_{R} = 4. 84 min. The subsequent reaction was conducted in the initial flask according to general procedure **L,** using intermediate (assumed as 0.063 mmol), ethanol (0.073 mL) and water (0.036 mL), 6 M solution of NaOH (21 µL, 0.126 mmol).Yellow solid (7.2 mg, 0.011 mmol, 18%). ¹H NMR (600 MHz, DMSO-d6) δ 13.04 (s, 1H), 9.42 (d, J = 7.1 Hz, 1H), 8.08 - 7.97 (m, 5H), 7.73 - 7.68 (m, 2H), 7.64 (dt, J = 7.2, 0.9 Hz, 1H), 7.56 (dd, J = 7.7, 1.2 Hz, 1H), 7.47 - 7.41 (m, 2H), 7.38 (td, J = 7.4, 1.0 Hz, 1H), 7.31 (d, J = 1.9 Hz, 0H), 7.30 (s, 1H), 7.17 - 7.14 (m, 1H), 5.56 (d, J = 7.1 Hz, 1H), 4.28 (d, J = 15.5 Hz, 1H), 4.24 (d, J = 15.5 Hz, 1H), 3.39 (dp, J = 18.7, 6.0 Hz, 2H), 2.88 (t, J = 6.0 Hz, 2H).¹³C NMR (151 MHz, DMSO-d6) δ 192.42, 171.58, 167.43, 142.65, 140.38, 139.96, 135.10, 134.26, 134.17, 133.73, 133.33, 133.00, 132.77 (d, J = 32.1 Hz), 132.08, 131.56, 129.63, 129.14, 128.92, 128.40, 127.68, 126.59 (d, J = 4.0 Hz), 126.11, 124.74, 124.12, 123.77, 123.40 (d, J = 273.1 Hz), 56.70, 47.20, 43.41, 27.75. LC-MS (ESI): m/z = 621.1 [M+1]⁺, t_{R} = 4. 43 min. Purity > 95% (UV).

**2-(9-oxo-9H-fluorene-4-carboxamido)-2-(2-((3-(trifluoromethyl)phenyl)sulfonyl)-1,2,3,4-tetrahydroisoquinolin-7-yl)acetic acid (K17).** The reaction was conducted following the general procedure **J,** using **K1j** (28 mg, 0.063 mmol), Et₃N (19 mg, 26 µL, 0.19 mmol), 3-(trifluoromethyl)benzenesulfonyl chloride (15 mg, 0.063 mmol), 1 mL of dry DCM. The crude intermediate was obtained as solid. The subsequent reaction was conducted in the initial flask according to general procedure **L,** using intermediate (assumed as 0.063 mmol), ethanol (0.073 mL), water (0.036 mL), 6 M solution of NaOH (21 µL, 0.126 mmol). Yellow solid (4.9 mg, 0.008 mmol, 12%). ¹H NMR (600 MHz, DMSO-d6) δ 12.98 (s, 1H), 9.41 (d, J = 7.1 Hz, 1H), 8.16 (d, J = 7.9 Hz, 1H), 8.11 - 8.05 (m, 2H), 7.87 (t, J = 7.9 Hz, 1H), 7.70 (dd, J = 7.5, 4.3 Hz, 2H), 7.64 (d, J = 7.3 Hz, 1H), 7.56 (d, J = 7.7 Hz, 1H), 7.44 (td, J = 7.6, 2.7 Hz, 2H), 7.37 (t, J = 7.4 Hz, 1H), 7.30 (d, J = 9.5 Hz, 2H), 7.15 (d, J = 7.8 Hz, 1H), 5.55 (d, J = 7.1 Hz, 1H), 4.30 (d, J = 15.5 Hz, 1H), 4.26 (d, J = 15.6 Hz, 1H), 3.40 (d, J = 8.4 Hz, 1H), 2.87 (t, J = 6.1 Hz, 2H). ¹³C NMR (151 MHz, DMSO-d6) δ 192.92, 172.05, 167.91, 164.17, 143.14, 140.87, 137.94, 135.59, 134.78, 134.65, 134.22, 133.81, 133.44, 132.58, 132.07, 131.97, 131.59, 130.66, 130.46 (d, J = 4.1 Hz), 130.13, 129.63, 129.39, 127.09, 126.58, 125.23, 124.61, 124.28, 123.81 (d, J = 273.1 Hz), 57.21, 47.70, 43.91, 28.13. ¹³C NMR (151 MHz, DMSO-d6) δ 192.45, 171.58, 167.44, 142.66, 140.39, 137.47, 135.12, 134.30, 134.18, 133.75, 133.34, 132.97, 132.11, 131.59, 131.50, 131.11, 130.10 (d, J = 27.9 Hz), 129.97, 129.65, 129.16, 128.92, 126.62, 126.11, 124.75, 124.14, 123.80, 123.33 (d, J = 273.1 Hz), 56.74, 47.22, 43.43, 27.65. LC-MS (ESI): m/z = 621.1 [M+1]⁺, t_{R} = 4. 39 min. Purity > 95% (UV).

**2-(2-((4-chlorophenyl)sulfonyl)-1,2,3,4-tetrahydroisoquinolin-7-yl)-2-(9-oxo-9H-fluorene-4-carboxamido)acetic acid (K18)** The reaction was conducted following the general procedure **J,** using **K1j** (29 mg, 0.065 mmol), Et₃N (19 mg, 26 µL, 0.19 mmol), 4-(trifluoromethyl)benzenesulfonyl chloride (12 mg, 0.065 mmol), 1mL of dry DCM. The crude intermediate was obtained as solid. LC-MS (ESI): m/z = 615.1 [M+1]⁺, t_{R} = 4.86 min. The subsequent reaction was conducted in the initial flask according to general procedure **L,** using intermediate (assumed as 0.065 mmol), ethanol (0.077 mL), water (0.038 mL), 6M solution of NaOH (21 µL, 0.130 mmol). Yellow solid (4.8 mg, 0.008 mmol, 12%). ¹H NMR (600 MHz, DMSO-d6) δ 13.03 (s, 1H), 9.42 (d, J = 7.1 Hz, 1H), 7.88 - 7.82 (m, 2H), 7.70 (td, J = 6.7, 1.4 Hz, 4H), 7.67 - 7.62 (m, 1H), 7.56 (dd, J = 7.7, 1.2 Hz, 1H), 7.44 (td, J = 7.5, 1.2 Hz, 2H), 7.38 (td, J = 7.4, 1.0 Hz, 1H), 7.33 - 7.28 (m, 2H), 7.16 (d, J = 7.8 Hz, 1H), 5.56 (d, J = 7.1 Hz, 1H), 4.26 - 4.16 (m, 2H), 3.38 - 3.28 (m, 2H), 2.87 (t, J = 6.0 Hz, 2H), -3.84 (s, 1H). ¹³C NMR (151 MHz, DMSO-d6) δ 192.41, 171.57, 167.42, 142.65, 140.37, 138.18, 135.09, 134.73, 134.23, 134.17, 133.72, 133.32, 133.03, 132.08, 131.64, 129.64, 129.54, 129.34, 129.14, 128.90, 126.58, 126.11, 124.72, 124.11, 123.77, 56.69, 47.24, 43.42, 27.74. LC-MS (ESI): m/z = 587.1 [M+1]^{+,} t_{R} = 4.41 min. Purity > 95% (UV).

**2-(2-((3-chlorophenyl)sulfonyl)-1,2,3,4-tetrahydroisoquinolin-7-yl)-2-(9-oxo-9H-fluorene-4-carboxamido)acetic acid (K19).** The reaction was conducted following the general procedure **J,** using **K1j** (29 mg, 0.065 mmol), Et₃N (19 mg, 26 µL, 0.19 mmol), 3-(trifluoromethyl)benzenesulfonyl chloride (12 mg, 0.065 mmol), 1 mL of dry DCM. The crude intermediate was obtained as solid. LC-MS (ESI): m/z = 615.1 [M+1]⁺, t_{R} = 4.85 min. The subsequent reaction was conducted in the initial flask according to general procedure L, using intermediate (assumed as 0.065 mmol), ethanol (0.077 mL), water (0.038 mL), 6M solution of NaOH (21 µL, 0.130 mmol). Yellow solid (6.9 mg, 0.012 mmol). ¹H NMR (600 MHz, DMSO-d6) δ 13.02 (s, 1H), 9.42 (d, J = 7.1 Hz, 1H), 7.85 (t, J = 2.0 Hz, 1H), 7.83 - 7.76 (m, 2H), 7.70 (dt, J = 7.6, 1.6 Hz, 2H), 7.68 - 7.62 (m, 2H), 7.56 (dd, J = 7.7, 1.2 Hz, 1H), 7.47 - 7.41 (m, 2H), 7.38 (td, J = 7.4, 0.9 Hz, 1H), 7.31 (d, J = 6.9 Hz, 2H), 7.16 (d, J = 8.0 Hz, 1H), 5.55 (d, J = 7.1 Hz, 1H), 4.27 (d, J = 15.5 Hz, 1H), 4.22 (d, J = 15.6 Hz, 1H), 3.39 (d, J = 6.1 Hz, 0H), 3.31 (2H; partly hidden under water peak, cf. HSQC), 2.87 (t, J = 6.0 Hz, 2H). ¹³C NMR (151 MHz, DMSO-d6) δ 192.42, 171.57, 167.42, 142.65, 137.89, 135.10, 134.18, 134.14, 133.72, 133.22, 131.64, 131.44, 129.64, 129.13, 128.90, 126.87, 126.60, 126.12, 124.72, 124.12, 123.78, 56.70, 47.26, 43.46, 27.73. LC-MS (ESI): m/z = 587.1 [M+1]⁺, t_{R} = 4.41 min. Purity > 95% (UV).

**2-(2-((3-bromophenyl)sulfonyl)-1,2,3,4-tetrahydroisoquinolin-7-yl)-2-(9-oxo-9H-fluorene-4-carboxamido)acetic acid (K20).** The reaction was conducted following the general procedure **J,** using **K1j** (12.5 mg, 0.03 mmol), Et₃N (9.1 mg, 13 µL, 0.09 mmol), 4-(trifluoromethyl)benzenesulfonyl chloride (7.6 mg, 0.03 mmol), in 1mL of dry DCM. The crude intermediate was obtained as solid. The subsequent reaction was conducted in the initial flask according to general procedure **L,** using intermediate (assumed as 0.03 mmol), ethanol (35 µL), water (18 µL), 6M NaOH (10 µL, 0.06 mmol). Yellow solid (1.3 mg, 0.002 mmol, 6.8%). ¹H NMR (600 MHz, DMSO-d6) δ 13.02 (s, 1H), 9.42 (d, J = 7.1 Hz, 1H), 7.96 (t, J = 1.9 Hz, 1H), 7.92 (dd, J = 7.9, 2.0 Hz, 1H), 7.87 - 7.82 (m, 1H), 7.72 - 7.68 (m, 2H), 7.64 (d, J = 7.3 Hz, 1H), 7.61 - 7.54 (m, 2H), 7.44 (td, J = 7.5, 3.9 Hz, 2H), 7.38 (t, J = 7.4 Hz, 1H), 7.31 (d, J = 7.9 Hz, 2H), 7.16 (d, J = 7.8 Hz, 1H), 5.56 (d, J = 7.1 Hz, 1H), 4.30 - 4.20 (m, 2H), 3.36 (q, J = 5.5 Hz, 2H), 2.87 (t, J = 6.0 Hz, 2H). ¹³C NMR (151 MHz, DMSO-*d*₆) δ 192.43, 171.57, 167.44, 142.65, 140.38, 138.04, 136.11, 135.11, 134.21, 134.18, 133.73, 133.33, 133.02, 132.09, 131.65, 129.65, 129.59, 129.15, 128.91, 126.61, 126.47, 126.47, 126.12, 124.73, 124.12, 123.79, 122.43, 56.72, 47.26, 43.46, 27.73. LC-MS (ESI): m/z = 631.1 [M+1]⁺, t_{R} = 4.50 min. Purity > 95% (UV).

**2-(2-((2,3-dihydrobenzo[b][1,4]dioxin-6-yl)sulfonyl)-1,2,3,4-tetrahydroisoquinolin-7-yl)-2-(9-oxo-9H-fluorene-4-carboxamido)acetic acid (K21).** The reaction was conducted following the general procedure **J,** using **K1j** (34 mg, 0.078 mmol), Et₃N (20 mg, 30 µL, 0.195 mmol), 2,3-dihydrobenzo[b][1,4]dioxine-6-sulfonyl chloride (15 mg, 0.065 mmol), 2 mL of dry DCM. The crude intermediate was obtained as solid. LC-MS (ESI): m/z = 639.2 [M+1]⁺, t_{R} = 4.64 min. The subsequent reaction was conducted in the initial flask according to general procedure **L,** using the intermediate (assumed as 0.065 mmol), methanol (90 µL) and water (45 µL), 6M NaOH (26 µL, 0.156 mmol). Yellow solid (8.2 mg, 0.013 mmol, 21 %). ¹H NMR (600 MHz, DMSO-d6) δ 13.03 (s, 1H), 9.41 (d, J = 7.1 Hz, 1H), 7.70 (t, J = 6.4 Hz, 2H), 7.64 (d, J = 7.3 Hz, 1H), 7.56 (d, J = 7.6 Hz, 1H), 7.44 (t, J = 7.3 Hz, 2H), 7.38 (t, J = 7.5 Hz, 1H), 7.33 - 7.26 (m, 4H), 7.17 (d, J = 7.9 Hz, 1H), 7.08 (d, J = 8.4 Hz, 1H), 5.55 (d, J = 7.1 Hz, 1H), 4.31 (dd, J = 15.4, 5.0 Hz, 4H), 4.19 - 4.09 (m, 2H), 3.25 (d, J = 6.1 Hz, 2H), 2.88 (d, J = 6.1 Hz, 2H). ¹³C NMR (151 MHz, DMSO-d6) δ 192.43, 171.59, 167.43, 147.52, 143.44, 142.66, 140.37, 135.10, 134.18, 133.73, 133.33, 133.09, 132.09, 131.78, 129.64, 129.14, 128.88, 127.69, 126.52, 126.16, 124.72, 124.12, 123.78, 121.07, 117.75, 116.50, 64.40, 64.03, 56.71, 47.36, 43.54, 27.91. LC-MS (ESI): m/z = 611.1 [M+1]⁺, t_{R} = 4.17 min. Purity > 95% (UV).

**2-(2-((3,4-dimethoxyphenyl)sulfonyl)-1,2,3,4-tetrahydroisoquinolin-7-yl)-2-(9-oxo-9H-fluorene-4-carboxamido)acetic acid (K22).** The reaction was conducted following the general procedure **J,** using **K1j** (34 mg, 0.078 mmol), Et₃N (20 mg, 30 µL, 0.195 mmol), 3,4-dimethoxybenzenesulfonyl chloride (14 mg, 0.065 mmol), 2 mL of dry DCM. The crude intermediate was obtained as solid. LC-MS (ESI): m/z = 658.2 [M+1]⁺, t_{R} = 4.58 min. The subsequent reaction was conducted in the initial flask according to general procedure **L,** using the intermediate (assumed as 0.065 mmol), ethanol (90 µL) and water (45 µL), 6M NaOH (26 µL, 0.156 mmol). Yellow solid (3.1 mg, 0.005 mmol, 8 %). ¹H NMR (600 MHz, DMSO-*d*₆) δ 13.02 (s, 1H), 9.39 (d, *J=* 6.9 Hz, 1H), 7.72 - 7.66 (m, 2H), 7.64 (d, *J* = 7.2 Hz, 1H), 7.56 (dd, *J* = 7.7, 1.2 Hz, 1H), 7.47 - 7.39 (m, 3H), 7.37 (t, *J =* 7.4 Hz, 1H), 7.30 (d, *J=* 7.8 Hz, 2H), 7.21 (d, *J* = 2.2 Hz, 1H), 7.15 (dd, *J* = 8.2, 5.9 Hz, 2H), 5.54 (d, *J =* 7.1 Hz, 1H), 4.26 - 4.14 (m, 2H), 3.83 (s, 3H), 3.78 (s, 3H), 3.30 (d, *J* = 5.9 Hz, 2H, partly hidden under water peak, cf. HSQC), 2.83 (t, *J* = 6.0 Hz, 2H). ¹³C NMR (151 MHz, DMSO-d6) δ 192.42, 171.55, 167.38, 152.49, 148.73, 142.65, 140.36, 135.09, 134.42, 134.15, 133.72, 133.32, 133.07, 132.12, 131.89, 129.62, 129.14, 128.84, 127.33, 126.47, 126.10, 124.71, 124.11, 123.77, 121.30, 111.34, 109.88, 56.78, 55.82, 47.39, 43.48, 27.67. LC-MS (ESI): m/z = 613.2 [M+1]⁺, t_{R} = 4.11 min. Purity > 95% (UV).

**2-(9-oxo-9H-fluorene-4-carboxamido)-2-(2-((2,3,5,6-tetramethylphenyl)sulfonyl)-1,2,3,4-tetrahydroisoquinolin-7-yl)acetic acid (K23).** The reaction was conducted following the general procedure **J,** using **K1j** (29 mg, 0.065 mmol), Et₃N (20 mg, 30 µL, 0.195 mmol), 2,3,5,6-tetramethylbenzenesulfonyl chloride (15 mg, 0.065 mmol), 2 mL of dry DCM. The crude intermediate was obtained as solid. The subsequent reaction was conducted in the initial flask according to general procedure **L,** using intermediate (assumed as 0.065 mmol), methanol (90 µL), water (45 µL), 6 M solution of NaOH (22 µL, 0.13 mmol). Yellow solid (6.9 mg, 0.011 mmol, 18 %). ¹H NMR (600 MHz, DMSO-d6) δ 13.01 (s, 1H), 9.42 (d, J = 7.1 Hz, 1H), 7.73 - 7.68 (m, 2H), 7.64 (dt, J = 7.2, 0.9 Hz, 1H), 7.56 (dd, J = 7.7, 1.2 Hz, 1H), 7.44 (dt, J = 9.4, 7.6 Hz, 2H), 7.38 (td, J = 7.4, 1.0 Hz, 1H), 7.35 - 7.30 (m, 3H), 7.19 (d, J = 7.8 Hz, 1H), 5.55 (d, J = 7.1 Hz, 1H), 4.31 - 4.23 (m, 2H), 3.44 (p, J = 6.4 Hz, 2H), 2.85 (t, J = 6.0 Hz, 2H), 2.46 (s,6H), 2.24 (s, 6H). ¹³C NMR (151 MHz, DMSO-*d*₆) δ 192.42, 171.62, 167.43, 142.67, 140.38, 135.91, 135.83, 135.44, 135.08, 134.92, 134.20, 134.12, 133.72, 133.46, 133.33, 132.14, 132.08, 129.64, 129.13, 129.07, 126.50, 126.19, 124.73, 124.14, 123.78, 56.74, 45.36, 41.45, 27.68, 20.31, 17.35. LC-MS (ESI): m/z = 609.2 [M+1]⁺, t_{R} = 4.67 min. Purity > 95% (UV).

**2-(2-((3,4-difluorophenyl)sulfonyl)-1,2,3,4-tetrahydroisoquinolin-7-yl)-2-(9-oxo-9H-fluorene-4-carboxamido)acetic acid (K24).** The reaction was conducted following the general procedure **J,** using **K1j** (28 mg, 0.063 mmol), Et₃N (19 mg, 26 µL, 0.191 mmol), 3,4-difluorobenzenesulfonyl chloride (13 mg, 0.063 mmol), in 1mL of dry DCM. The crude intermediate was obtained as solid. LC-MS (ESI): t_{R} = 4.73 min. The subsequent reaction was conducted in the initial flask according to general procedure L, using the intermediate (assumed as 0.063 mmol), ethanol (73 µL), water (37 µL then), 6 M NaOH (21 µL, 0.126 mmol) Yellow solid (3.2 mg, 0.005 mmol, 6%). ¹H NMR (600 MHz, DMSO-d6) δ 13.06 (s, 1H), 9.43 (d, J = 7.1 Hz, 1H), 7.96 (t, J = 8.5 Hz, 1H), 7.73 - 7.68 (m, 4H), 7.64 (d, J = 7.2 Hz, 1H), 7.56 (d, J = 7.7 Hz, 1H), 7.46 - 7.41 (m, 2H), 7.38 (t, J = 7.5 Hz, 1H), 7.31 (d, J = 10.2 Hz, 2H), 7.17 (d, J = 7.9 Hz, 1H), 5.56 (d, J = 7.0 Hz, 1H), 4.25 (t, J = 10.3 Hz, 2H), 3.35 (dt, J = 15.0, 6.9 Hz, 2H), 2.89 (d, J = 6.3 Hz, 2H). ¹³C NMR (151 MHz, DMSO-d6) δ 192.46, 171.61, 167.47, 152.45 (dd, J = 254.0, 12.3 Hz), 149.43 (dd, J = 251.7, 13.2 Hz), 142.68, 140.41, 135.13, 134.30, 134.19, 133.76, 133.35, 133.03, 132.11, 131.67, 129.66, 129.17, 128.94, 126.63, 126.12, 125.44 (t, J = 5.6 Hz), 124.76, 124.15, 123.81, 118.81 (d, J = 18.2 Hz), 117.46 (d, J = 19.6 Hz), 56.75, 47.30, 43.49, 27.76. LC-MS (ESI): m/z = 589.10 [M+1]⁺, t_{R} = 4.275 min. Purity > 95% (UV).

**2-(2-((3,5-difluorophenyl)sulfonyl)-1,2,3,4-tetrahydroisoquinolin-7-yl)-2-(9-oxo-9H-fluorene-4-carboxamido)acetic acid (K25).** The reaction was conducted following the general procedure **J,** using **K1j** (29 mg, 0.065 mmol), Et₃N (20 mg, 27 µL, 0.195 mmol), 3,5-difluorobenzenesulfonyl chloride (13 mg, 0.065 mmol), 1mL of dry DCM. The crude intermediate was obtained as solid. LC-MS (ESI): m/z = 617.1 [M+1]⁺, t_{R} = 4.81 min. The subsequent reaction was conducted in the initial flask according to general procedure **L,** using the crude intermediate (assumed as 0.065 mmol), ethanol (73 µL), water (37 µL then), 6M NaOH (21 µL, 0.126 mmol). Yellow solid (6.2 mg, 0.011 mmol, 16%). ¹H NMR (600 MHz, DMSO-d6) δ 13.03 (s, 1H), 9.43 (d, J = 7.1 Hz, 1H), 7.71 (d, J = 1.1 Hz, 1H), 7.71 - 7.65 (m, 2H), 7.64 (dt, J = 7.3, 0.9 Hz, 1H), 7.63 - 7.57 (m, 2H), 7.56 (dd, J = 7.7, 1.2 Hz, 1H), 7.48 - 7.41 (m, 2H), 7.38 (td, J = 7.4, 1.0 Hz, 1H), 7.34 - 7.29 (m, 2H), 7.18 (d, J = 7.8 Hz, 1H), 5.56 (d, J = 7.1 Hz, 1H), 4.31 - 4.23 (m, 2H), 3.42 - 3.34 (2H; partly hidden underwater peak, cf. HSQC), 2.89 (t, J = 6.0 Hz, 2H). ¹³C NMR (151 MHz, DMSO-d6) δ 192.43, 171.58, 167.44, 162.35 (dd, J = 251.9, 12.4 Hz), 142.66, 140.39, 139.24 (t, J = 8.1 Hz), 135.11, 134.25, 134.17, 133.73, 133.33, 132.99, 132.09, 131.63, 129.64, 129.14, 128.93, 126.64, 126.07, 124.73, 124.13, 123.78, 111.27 (d, J = 28.3 Hz), 111.27 (d, J = 21.74 Hz), 109.04 (t, J = 25.8 Hz), 56.71, 47.26, 43.50, 27.79. LC-MS (ESI): m/z = 589.1 [M+1]⁺, t_{R} = 4.33 min. Purity > 95% (UV).

**2-(2-((3-cyano-4-ethoxyphenyl)sulfonyl)-1,2,3,4-tetrahydroisoquinolin-7-yl)-2-(9-oxo-9H-fluorene-4-carboxamido)acetic acid (K26).** The reaction was conducted following the general procedure **J,** using **K1j** (28 mg, 0.063 mmol), Et₃N (19 mg, 26 µL, 0.191 mmol), 3-cyano-4-fluorobenzenesulfonyl chloride (14 mg, 0.063 mmol), 1 mL of dry DCM. The crude intermediate was obtained as solid. LC-MS (ESI): m/z = 624.1 [M+1]⁺, t_{R} = 4.61 min. The subsequent reaction was conducted in the initial flask according to general procedure **L,** using crude intermediate (assumed as 0.063 mmol), ethanol (73 µL), water (37 µL), 6 M NaOH (21 µL, 0.126 mmol ). Yellow solid (8 mg product, 0.013 mmol, 20%). ¹H NMR (600 MHz, DMSO-d6) δ 8.20 (d, J = 2.4 Hz, 1H), 8.05 (dd, J = 9.0, 2.4 Hz, 1H), 7.70 (dd, J = 7.4, 1.1 Hz, 1H), 7.67 (d, J = 7.6 Hz, 1H), 7.64 (d, J = 7.2 Hz, 1H), 7.57 (dd, J = 7.7, 1.2 Hz, 1H), 7.46 - 7.41 (m, 3H), 7.37 (t, J = 7.4 Hz, 1H), 7.30 (d, J = 8.1 Hz, 2H), 7.15 (d, J = 7.8 Hz, 1H), 5.49 (s, 1H), 4.29 (q, J = 7.0 Hz, 2H), 4.24 - 4.16 (m, 2H), 3.38 - 3.34 (2H; partly hidden under water peak, cf. HSQC), 2.87 (t, J = 6.0 Hz, 2H), 1.38 (t, J = 7.0 Hz, 3H). ¹³C NMR (151 MHz, DMSO-d6) δ 192.44, 171.44, 167.24, 163.15, 142.66, 140.36, 135.11, 134.38, 134.10, 133.75, 133.40, 133.33, 132.78, 132.22, 131.59, 129.63, 129.18 (2C), 128.78, 128.00, 126.47, 126.00, 124.71, 124.12, 123.77, 115.02, 113.70, 101.50, 65.62, 56.96, 47.36, 43.48, 27.74, 14.15.LC-MS (ESI): m/z = 622.1 [M+1]⁺, t_{R} = 4.248 min. Purity > 95% (UV).

**2-(2-((3-cyano-4-fluorophenyl)sulfonyl)-1,2,3,4-tetrahydroisoquinolin-7-yl)-2-(9-oxo-9H-fluorene-4-carboxamido)acetic acid (K27).** The reaction was conducted following the general procedure **K,** using **K1i** (16 mg, 0.038 mmol), 3-cyano-4-fluorobenzenesulfonyl chloride (12 mg, 0.057 mmol) in 1mL of pyridine. Yellow solid (1.6 mg, 0.003 mmol, 7%). ¹H NMR (600 MHz, DMSO-d6) δ 13.04 (s, 1H), 9.43 (d, J = 7.1 Hz, 1H), 8.49 - 8.43 (m, 1H), 8.22 (ddd, J = 8.9, 4.9, 2.4 Hz, 1H), 7.75 (t, J = 8.9 Hz, 1H), 7.70 (d, J = 7.4 Hz, 2H), 7.64 (d, J = 7.3 Hz, 1H), 7.56 (d, J = 7.6 Hz, 1H), 7.45 (q, J = 7.6 Hz, 2H), 7.39 (t, J = 7.4 Hz, 1H), 7.31 (dd, J = 8.0, 1.8 Hz, 1H), 7.29 (s, 1H), 7.17 (d, J = 7.9 Hz, 1H), 5.56 (d, J = 7.1 Hz, 1H), 4.26 (s, 2H), 3.42 (tq, J = 16.7, 10.8, 8.4 Hz, 2H), 2.88 (t, J = 6.0 Hz, 2H). ¹³C NMR (151 MHz, DMSO-*d*₆) δ 192.44, 171.59, 167.46, 164.78 (d, *J* = 262.8 Hz), 142.66, 140.39, 135.27 (d, *J* = 10.1 Hz), 135.12, 134.22 (d, *J* = 10.2 Hz), 134.19, 133.78, 133.74, 133.46 (d, *J =* 3.4 Hz), 133.33, 133.00, 132.09, 131.61, 129.66, 129.15, 128.93, 126.64, 126.10, 124.75, 124.14, 123.80, 117.98 (d, *J* = 20.8 Hz), 112.80, 101.94 (d, *J* = 16.6 Hz), 56.74, 47.26, 43.42, 27.69. LC-MS (ESI): m/z = 596.2 [M+1]⁺, t_{R} = 4.30 min. Purity > 95% (UV).

**2-(2-((3-cyano-4-methoxyphenyl)sulfonyl)-1,2,3,4-tetrahydroisoquinolin-7-yl)-2-(9-oxo-9H-fluorene-4-carboxamido)acetic acid (K28).** The reaction was conducted following the general procedure **J,** using **K1j** (8 mg, 0.02 mmol), Et₃N (7 mg, 10 µL, 0.06 mmol), 3-cyano-4-fluorobenzenesulfonyl chloride (4 mg, 0.02 mmol), 1mL of dry DCM. The crude intermediate was obtained as solid. The subsequent reaction was conducted in the initial flask according to general procedure **L,** using the intermediate, methanol (90 µL), water (45 µL), 6 M NaOH (6 µL, 0.04 mmol). Yellow solid (1 mg, 0.01 mmol, 50%). ¹H NMR (600 MHz, DMSO-d6) δ 12.96 (s, 1H), 9.35 - 9.31 (m, 1H), 8.21 (d, J = 2.3 Hz, 1H), 8.09 (dd, J = 9.0, 2.4 Hz, 1H), 7.70 (d, J = 7.3 Hz, 1H), 7.67 (d, J = 7.6 Hz, 1H), 7.64 (d, J = 7.2 Hz, 1H), 7.57 (d, J = 7.6 Hz, 1H), 7.44 (dd, J = 8.3, 4.4 Hz, 3H), 7.37 (t, J = 7.4 Hz, 1H), 7.30 (d, J = 10.0 Hz, 2H), 7.15 (d, J = 7.8 Hz, 1H), 5.52 (d, J = 7.0 Hz, 1H), 4.25 - 4.16 (m, 2H), 4.00 (s, 3H), 3.33 - 3.25 (2H; partly hidden under water peak, cf. HSQC), 2.88 (t, J = 6.0 Hz, 2H). ¹³C NMR (151 MHz, DMSO-*d*₆) δ 192.45, 171.62, 167.33, 163.89, 142.67, 140.38, 135.13, 134.46, 134.14, 133.76, 133.34 (2C), 132.85, 132.20, 131.62, 129.65, 129.18 (2C), 128.83, 128.21, 126.53, 126.05, 124.73, 124.14, 123.79, 115.01, 113.18, 101.41, 57.20, 56.96, 47.36, 43.48, 27.76. LC-MS (ESI): m/z = 608.1 [M+1]⁺, t_{R} = 4.181 min. Purity > 95% (UV).

**2-(2-((3-cyano-5-fluorophenyl)sulfonyl)-1,2,3,4-tetrahydroisoquinolin-7-yl)-2-(9-oxo-9H-fluorene-4-carboxamido)acetic acid (K29).** The reaction was conducted following the general procedure **K,** using K**1i** (14 mg, 0.048 mmol), 3-cyano-5-fluorobenzenesulfonyl chloride (15 mg, 0.072 mmol), 1 mL of pyridine. Yellow solid (1.2 mg, 0.002 mmol, 4.2%). ¹H NMR (600 MHz, DMSO-d6) δ 12.89 (s, 1H), 9.45 (dd, J = 15.1, 7.1 Hz, 1H), 8.24 (d, J = 8.8 Hz, 1H), 8.20 (s, 1H), 8.07 (d, J = 8.1 Hz, 1H), 7.71 (d, J = 7.4 Hz, 2H), 7.64 (d, J = 7.3 Hz, 1H), 7.56 (d, J = 7.6 Hz, 1H), 7.45 (q, J = 8.1 Hz, 2H), 7.38 (t, J = 7.4 Hz, 1H), 7.32 (d, J = 8.0 Hz, 1H), 7.30 (s, 1H), 7.18 (d, J = 8.2 Hz, 1H), 5.56 (d, J = 6.8 Hz, 1H), 4.32 (d, J = 24.0 Hz, 2H), 3.44 (q, J = 6.1 Hz, 1H), 2.88 (q, J = 9.9, 8.0 Hz, 2H). ¹³C NMR (151 MHz, DMSO-*d*₆) δ 192.47, 171.72, 167.22, 161.63 (d, *J* = 252.4 Hz), 142.68, 140.39, 139.57 (d, *J* = 7.2 Hz), 135.15, 134.10, 133.78, 133.35, 132.29, 131.44, 129.66, 129.21, 128.82 (2C), 127.60 (d, *J =* 3.8 Hz), 126.57, 125.91, 124.73, 124.31 (d, *J* = 25.7 Hz), 124.16, 123.81, 119.75 (d, *J* = 24.2 Hz), 118.30, 116.48, 114.49 (d, *J=* 9.8 Hz), 57.18, 47.28, 43.52, 27.79. LC-MS (ESI): m/z = 596.2 [M+1]⁺, t_{R} = 4.32 min. Purity > 95% (UV).

**2-(2-((3-(ethoxycarbonyl)-5-fluorophenyl)sulfonyl)-1,2,3,4-tetrahydroisoquinolin-7-yl)-2-(9-oxo-9H-fluorene-4-carboxamido)acetic acid (K30).** The reaction was conducted following the general procedure **J,** using **K1j** (28 mg, 0.063 mmol), Et₃N (19 mg, 26 µL, 0.191 mmol), 3-cyano-5-fluorobenzenesulfonyl chloride (14 mg, 0.063 mmol), 1mL of dry DCM. The crude intermediate was obtained as solid. LC-MS (ESI): t_{R} = 4.76 min. The subsequent reaction was conducted in the initial flask according to general procedure L, using crude intermediate (assumed as 0.063 mmol), ethanol (73 µL), water (37 µL), 6M NaOH (21 µL, 0.126 mmol). Yellow solid. (1.2 mg, 0.002 mmol, 30%). ¹H NMR (600 MHz, DMSO-d₆) δ 13.03 (s, 1H), 9.41 (d, J = 7.2 Hz, 1H), 8.11 (d, J = 1.6 Hz, 1H), 8.04 - 7.96 (m, 2H), 7.70 (t, J = 7.8 Hz, 2H), 7.64 (d, J = 7.2 Hz, 1H), 7.56 (dd, J = 7.7, 1.1 Hz, 1H), 7.44 (t, J = 7.5 Hz, 2H), 7.37 (t, J = 7.4 Hz, 1H), 7.31 (d, J = 7.9 Hz, 2H), 7.17 (d, J = 7.8 Hz, 1H), 5.55 (d, J = 7.1 Hz, 1H), 4.37 (q, J = 7.1 Hz, 2H), 4.33 - 4.23 (m, 2H), 3.41 (ddd, J = 14.5, 10.2, 6.1 Hz, 2H), 2.89 (t, J = 6.0 Hz, 2H), 1.35 (t, J = 7.1 Hz, 3H). ¹³C NMR (151 MHz, DMSO-d6) δ 192.43, 171.57, 167.44, 163.49, 161.97 (d, J = 251.4 Hz), 142.65, 140.39, 138.80 (d, J = 6.4 Hz), 135.11, 134.27, 134.18, 133.73, 133.41 (d, J = 7.3 Hz), 133.33, 132.97, 132.09, 131.56, 129.63, 129.15, 128.92, 126.61, 126.12, 124.74, 124.13, 123.78, 123.63 (d, J = 2.5 Hz), 120.61 (d, J = 23.5 Hz), 119.06 (d, J = 24.5 Hz), 61.90, 56.70, 47.18, 43.46, 27.76, 14.00. LC-MS (ESI): m/z = 643.3 [M+1]⁺, t_{R} = 4.58 min. Purity > 95% (UV).

**2-(2-(cyclohexylsulfonyl)-1,2,3,4-tetrahydroisoquinolin-7-yl)-2-(9-oxo-9H-fluorene-4-carboxamido)acetic acid (K31).** The reaction was conducted following the general procedure **J,** using **1j** (29 mg, 0.065 mmol), Et₃N (20 mg, 30 µL, 0.195 mmol), cyclohexanesulfonyl chloride (12 mg, 0.065 mmol), 2 mL of dry DCM. The crude intermediate was obtained as solid. The subsequent reaction was conducted in the initial flask according to general procedure **L,** using crude intermediate (assumed as 0.065 mmol), ethanol (74 µL) and water (37 µL), 6 M NaOH (21 µL, 0.130 mmol). White solid (2 mg, 0.003 mmol, 5 %). ¹H NMR (600 MHz, DMSO-d6) δ 13.05 (s, 1H), 9.46 (d, J = 7.2 Hz, 1H), 7.72 (dd, J = 14.7, 7.5 Hz, 2H), 7.65 (d, J = 7.3 Hz, 1H), 7.57 (d, J = 7.6 Hz, 1H), 7.46 (dt, J = 21.7, 7.6 Hz, 2H), 7.39 (t, J = 7.5 Hz, 1H), 7.33 (d, J = 8.0 Hz, 1H), 7.29 (s, 1H), 7.21 (d, J = 8.1 Hz, 1H), 5.58 (d, J = 7.1 Hz, 1H), 4.45 (d, J = 9.2 Hz, 2H), 3.56 (d, J = 6.3 Hz, 2H), 3.21 (t, J = 11.9 Hz, 1H), 2.86 (t, J = 5.8 Hz, 2H), 2.00 (d, J = 12.3 Hz, 2H), 1.76 (d, J = 12.8 Hz, 2H), 1.61 (d, J = 13.0 Hz, 1H), 1.39 (q, J = 11.7, 11.2 Hz, 2H), 1.29 (d, J = 13.0 Hz, 1H), 1.25 (d, J = 7.8 Hz, 2H). ¹³C NMR (151 MHz, DMSO-d6) δ 192.43, 171.64, 167.44, 142.68, 140.39, 135.13, 134.21, 134.14, 133.72, 133.65, 133.32, 132.95, 132.10, 129.65, 129.19, 129.13, 126.34, 125.81, 124.72, 124.14, 123.78, 59.62, 56.73, 46.75, 43.15, 28.57, 26.12, 26.09, 24.77, 24.45. LC-MS (ESI): m/z = 559.2 [M+1]⁺, t_{R} = 4.31 min. Purity > 95% (UV).

**Ethyl 2-(2-((3-cyano-4-fluorophenyl)sulfonyl)-1,2,3,4-tetrahydroisoquinolin-7-yl)-2-(9-hydroxy-9H-fluorene-4-carboxamido)acetate (K32a).** The reaction was conducted following the general procedure **J,** using **K1j** (28 mg, 0.063 mmol), Et₃N (19 mg, 26 µL, 0.191 mmol), 3-cyano-4-fluorobenzenesulfonyl chloride (14 mg, 0.063 mmol), 1 mL of dry DCM. Yellow solid. LC-MS (ESI): m/z = 624.1 [M+1]⁺, t_{R} = 4.61 min. The subsequent reaction was conducted following the general procedure **N,** using intermediate (19 mg, 0.03 mmol) in MeOH and NaBH₄ (3.45 mg, 0.09 mmol). White solid (0.076 g, 0.34 mmol, 84%). LC-MS (ESI): m/z = 626.1 [M+1]⁺, t_{R} = 4.35 min.

**2-(2-((3-cyano-4-ethoxyphenyl)sulfonyl)-1,2,3,4-tetrahydroisoquinolin-7-yl)-2-(9-hydroxy-9H-fluorene-4-carboxamido)acetic acid (K32).** The reaction was conducted following the general procedure **L,** using **K32a** (0.076 g, 0.34 mmol), ethanol (1 mL), water (0.5 mL), 6 M NaOH (10 µL, 0.06 mmol). Yellow solid (1.61 mg, 0.002 mmol, 8.6%, over two steps; mix of four stereoisomers). ¹H NMR (600 MHz, DMSO-d6) δ 13.11 (s, 1H), 9.37 (t, J = 7.3 Hz, 1H), 8.21 (s, 1H), 8.09 (d, J = 9.0 Hz, 1H), 7.71 (d, J = 7.6 Hz, 1H), 7.66 (s, 1H), 7.59 (d, J = 7.4 Hz, 1H), 7.44 (d, J = 9.0 Hz, 1H), 7.35 (s, 2H), 7.30 (d, J = 7.6 Hz, 3H), 7.22 (dt, J = 15.1, 7.7 Hz, 1H), 7.16 (d, J = 7.8 Hz, 1H), 5.68 (d, J = 7.0 Hz, 1H), 5.46 (s, 1H), 4.25 - 4.16 (m, 2H), 4.00 (s, 3H), 3.35 - 3.30 (m, 2H), 2.88 (d, J = 7.5 Hz, 2H), 1.24 (s, 3H). ¹³C NMR (151 MHz, DMSO-*d*₆) δ 170.86, 168.73, 168.71, 163.88, 147.97, 147.85, 147.32, 147.28, 137.95, 135.99, 134.45, 133.83, 133.76, 133.33, 133.16, 131.74, 131.03, 131.00, 129.63, 128.94, 128.20, 128.01, 127.97, 127.60, 127.55, 126.81, 126.71, 126.64, 126.21, 126.16, 126.08, 126.05, 124.73, 123.09, 123.06, 114.98, 101.42, 73.00, 57.19, 56.55, 52.34, 47.29, 43.43, 27.76. LC-MS (ESI): m/z = 624.2 [M+1]⁺, t_{R} = 4.14 min. Purity > 95% (UV).

**Ethyl 2-(9-hydroxy-9H-fluorene-4-carboxamido)-2-(1,2,3,4-tetrahydroisoquinolin-7-yl)acetate (K33a).** The reaction was conducted following the general procedure **N,** using **K1j** (50 mg, 0.11 mmol), NaBH₄ (20 mg, 0.17 mmol), MeOH. White solid (26.6 mg, 0.06 mmol, 55%).¹H NMR (600 MHz, DMSO-d6) δ 9.44 (t, J = 6.5 Hz, 1H), 9.15 (s, 2H), 7.72 (dd, J = 25.4, 7.7 Hz, 1H), 7.67 (d, J = 7.0 Hz, 1H), 7.60 (d, J = 7.4 Hz, 1H), 7.41 (d, J = 8.0 Hz, 1H), 7.37 (d, J = 2.8 Hz, 1H), 7.38 - 7.34 (m, 1H), 7.36 - 7.29 (m, 2H), 7.29 - 7.22 (m, 2H), 5.93 (s, 1H), 5.72 (d, J = 7.2 Hz, 1H), 5.47 (s, 1H), 4.31 (d, J = 16.2 Hz, 1H), 4.26 (s, 1H), 4.25 - 4.18 (m, 1H), 4.18 - 4.10 (m, 1H), 3.01 (t, J = 6.2 Hz, 2H), 2.09 (s, 2H), 1.20 (t, J = 7.1 Hz, 3H). LC-MS (ESI): m/z = 441.2 [M+1]⁺, t_{R} = 3.21 min. Purity > 95% (UV).

**Methyl 2-(9-methoxy-9H-fluorene-4-carboxamido)-2-(1,2,3,4-tetrahydroisoquinolin-7-yl)acetate (K33b). K33a** (25 mg, 0.056 mmol, 1.00 equiv.) was added into to 1 mL methanol following with concentrated sulfuric acid 0.5 mL. The resulting reaction mixture was stirred at room temperature for 48 hours. Upon the reaction completed, the resulting reaction mixture was added into 10 mL water and extracted with DCM. The combined organic layers was dried over magnesium sulfate, filtered and concentrated to dryness *in vacuo.* The title compound **K33b** was obtained and used as a starting material in the next step without further purification. LC-MS (ESI): m/z = 443.1 [M+1]⁺, t_{R} = 3.19 min.

**2-(2-((3-cyano-4-ethoxyphenyl)sulfonyl)-1,2,3,4-tetrahydroisoquinolin-7-yl)-2-(9-methoxy-9H-fluorene-4-carboxamido)acetic acid (K33).** The reaction was conducted following the general procedure **J,** using **K33b** (19 mg, 0.043 mmol), Et₃N (13 mg, 18 µL, 0.129 mmol), 3-cyano-4-fluorobenzenesulfonyl chloride (9.44 mg, 0.043mmol), 2 mL of dry DCM. The crude product was obtained as solid. LC-MS (ESI): m/z = 626.2 [M+1]⁺, t_{R} = 4.54 min. The subsequent reaction was conducted in the initial flask according to general procedure **L,** using the intermediate, ethanol (1 mL) and water (0.5 mL), 6M NaOH (14 µL, 0.086 mmol). Yellow solid (3 mg, 0.005 mmol, 5 %, over two steps%; mix of four stereoisomers). ¹H NMR (600 MHz, DMSO-*d*₆) δ 9.14 (s, 1H), 8.19 (s, 1H), 8.05 (d, *J =* 8.9 Hz, 1H), 7.68 (dd, *J* = 17.1, 6.9 Hz, 2H), 7.58 (d, *J* = 7.6 Hz, 1H), 7.40 (h, *J* = 9.0, 8.0 Hz, 4H), 7.31 (d, *J* = 9.4 Hz, 3H), 7.22 (t, *J* = 7.6 Hz, 1H), 7.14 (d, *J* = 7.8 Hz, 1H), 5.55 (t, *J=* 9.3 Hz, 2H), 4.28 (q, *J=* 7.0 Hz, 2H), 4.19 (d, *J=* 5.8 Hz, 2H), 3.09 (d, *J* = 11.2 Hz, 3H), 2.88 (d, *J* = 6.2 Hz, 2H), 1.38 (t, *J =* 7.0 Hz, 3H.) ¹³C NMR (151 MHz, DMSO-*d*₆) δ 168.36, 163.21, 157.99, 157.79, 143.67, 143.62, 143.07, 143.04, 138.96, 138.92, 136.87, 136.81, 134.45, 133.44, 132.73, 131.73, 131.55, 128.80, 128.71, 128.68, 128.22, 128.19, 128.02, 128.01, 127.80, 127.11, 126.57, 126.31, 126.08, 125.09, 125.05, 123.50, 123.44, 115.08, 113.76, 101.55, 80.36, 65.69, 53.15, 52.94, 47.41, 43.55, 27.79, 14.20. LC-MS (ESI): m/z = 638.2 [M+1]⁺, t_{R} = 4.38 min. Purity > 95% (UV).

**Ethyl 2-(spiro[fluorene-9,2'-[1,3]dithiolane]-4-carboxamido)-2-(1,2,3,4-tetrahydroisoquinolin-7-yl)acetate (K34a).** The reaction was conducted following the general procedure **O,** using **K1j** (50 mg, 0.11 mmol), 2.2 mL of DCM, 1,2-ethanedithiol (51.8 mg, 46 µL, 0.55 mmol), and 0.11 mL of boron trifluoride etherate. White solid (37.4 mg, 0.07 mmol, 66%) and treated as starting materials without further purification. LC-MS (ESI): m/z = 517.2 [M+1]⁺, t_{R} = 3.67 min.

**Ethyl 2-(9H-fluorene-4-carboxamido)-2-(1,2,3,4-tetrahydroisoquinolin-7-yl)acetate (K34b).** A mixture of thioketal **K34a** (37mg, 0.07 mmol, 1.0 equiv.) and NaI (210 mg, 1.4 mmol, 20.0 equiv.) was stirred in DCM for 5 min. TMS-Cl (152 mg, 178 µL, 20.0 equiv.) were added to the solution which was stirred at RT for 5h. The reaction was hydrolysed for 5 min with H₂O (1 mL) and the brown mixture was washed with of a saturated Na₂S₂O₃ solution (2 mL) and extracted with DCM. Organic layers were dried over MgSO₄ and concentrated under reduced pressure to give a residue which was purified by on C18 column. White solid (17 mg, 0.040 mmol, 60%). ¹H NMR (600 MHz, DMSO-d6) δ 9.44 (d, J = 7.4 Hz, 1H), 8.95 (br s, 1H), 7.83 (d, J = 7.7 Hz, 1H), 7.71 - 7.66 (m, 1H), 7.60 (d, J = 7.5 Hz, 1H), 7.42 (dd, J = 8.0, 1.9 Hz, 1H), 7.41 - 7.30 (m, 4H), 7.29 - 7.23 (m, 2H), 5.75 (d, J = 7.3 Hz, 1H), 4.34 - 4.24 (m, 2H), 4.24 - 4.11 (m, 2H), 3.96 (s, 2H), 3.41 (d, J = 6.6 Hz, 2H), 3.01 (t, J = 6.3 Hz, 2H), 2.65 (s, 1H), 2.59 (s, 0H), 1.20 (t, J = 7.1 Hz, 3H). LC-MS (ESI): m/z = 427.2 [M+1]⁺, t_{R} = 3.48 min.

**2-(2-((3-cyano-4-ethoxyphenyl)sulfonyl)-1,2,3,4-tetrahydroisoquinolin-7-yl)-2-(9H-fluorene-4-carboxamido)acetic acid (K34).** The reaction was conducted following the general procedure **J,** using **K34b** (12 mg, 0.028 mmol), Et₃N (8.54 mg, 12 µL, 0.084 mmol), 3-cyano-4-fluorobenzenesulfonyl chloride (6.18 mg, 0.028 mmol), 2 mL of dry DCM. The crude intermediate was obtained as solid. The subsequent reaction was conducted in the initial flask according to general procedure **L,** using the intermediate (assumed as 0.028 mmol), ethanol (1 mL), water (0.5 mL), 6 M NaOH (9 µL, 0.056 mmol). White solid (2.8 mg, 0.005 mmol, 16 %) ¹H NMR (600 MHz, DMSO-d6) δ 12.97 (s, 1H), 9.21 (d, J = 7.4 Hz, 1H), 8.23 - 8.17 (m, 1H), 8.09 - 8.01 (m, 1H), 7.80 (d, J = 7.8 Hz, 1H), 7.67 (t, J = 4.5 Hz, 1H), 7.58 (d, J = 7.5 Hz, 1H), 7.41 (d, J = 9.0 Hz, 1H), 7.37 - 7.30 (m, 4H), 7.28 (t, J = 7.5 Hz, 1H), 7.19 - 7.10 (m, 2H), 5.61 (d, J = 7.3 Hz, 1H), 4.29 (q, J = 7.0 Hz, 2H), 4.24 - 4.15 (m, 2H), 3.95 (s, 2H), 3.32 (m, 2H), 2.88 (t, J = 6.0 Hz, 2H), 1.38 (t, J = 7.0 Hz, 3H). ¹³C NMR (151 MHz, DMSO-d6) δ 171.76, 168.83, 163.15, 143.83, 143.38, 139.63, 137.51, 134.64, 134.38, 133.39, 132.82, 131.58, 131.42, 129.63, 128.79, 127.97, 126.88, 126.59, 126.28, 126.13, 126.08, 125.98, 124.75, 123.28, 115.01, 113.70, 101.51, 65.62, 56.56, 47.33, 43.47, 36.30, 26.54, 14.15. LC-MS (ESI): m/z = 608.1 [M+1]⁺, t_{R} = 4.38 min. Purity > 95% (UV).

**2-(2-((3-bromophenyl)sulfonyl)-1,2,3,4-tetrahydroisoquinolin-7-yl)-2-(9H-fluorene-4-carboxamido)acetic acid (K35)** . The reaction was conducted following the general procedure **J,** using **K34b** (13mg, 0.03 mmol), Et₃N (7 mg, 10 µL, 0.06 mmol), 3-bromobenzenesulfonyl chloride (8 mg, 0.03 mmol), 2 mL of dry DCM. The crude intermediate was obtained as white solid (31 mg). LC-MS (ESI): m/z = 626.1 [M+1]⁺, t_{R} = 5.16 min. The subsequent reaction was conducted in the initial flask according to general procedure **L,** using the intermediate (assumed as 0.03 mmol), ethanol (1 mL) and water (0.5mL), 6 M NaOH (10 µL, 0.06 mmol). White solid (9 mg, 0.014 mmol, 49 %). ¹H NMR (600 MHz, DMSO-d6) δ 12.82 (s, 1H), 9.20 (d, J = 7.4 Hz, 1H), 7.96 (d, J = 2.1 Hz, 1H), 7.91 (dd, J = 7.9, 2.0 Hz, 1H), 7.82 (dd, J = 19.1, 7.8 Hz, 2H), 7.67 (q, J = 4.2 Hz, 1H), 7.58 (dq, J = 8.0, 4.6, 4.1 Hz, 2H), 7.40 - 7.31 (m, 4H), 7.28 (t, J = 7.4 Hz, 1H), 7.24 - 7.12 (m, 2H), 5.62 (d, J = 7.4 Hz, 1H), 4.28 - 4.19 (m, 2H), 3.94 (s, 2H), 3.36 (m, 2H), 2.87 (t, J = 6.0 Hz, 2H).¹³C NMR (151 MHz, DMSO-*d*₆) δ 171.76, 168.84, 143.84, 143.39, 139.64, 138.04, 137.52, 136.12, 134.65, 132.81, 131.66, 131.52, 131.43, 129.60, 128.83, 126.91, 126.65, 126.48 (2C), 126.29, 126.12, 126.09, 126.00, 124.77, 123.30, 122.44, 56.56, 47.28, 43.50, 36.31, 27.74.· LC-MS (ESI): m/z = 618.2 [M+1]⁺, t_{R} = 4.70 min. Purity > 95% (UV).

**Ethyl 9-oxo-9H-fluorene-4-carboxylate (K36a).** The reaction was conducted following the general procedure **E,** using 9-oxo-9H-fluorene-4-carboxylic acid (127.91 mg, 1.00 mmol), TMS-Cl (250 µL, 2.00 mmol), ethanol (1 mL). Yellow oil product. ¹H NMR (600 MHz, DMSO-d6) δ 8.12 (dt, J = 7.7, 0.9 Hz, 1H), 7.96 (dd, J = 7.8, 1.2 Hz, 1H), 7.84 (dd, J = 7.3, 1.2 Hz, 1H), 7.70 (dt, J = 7.3, 1.0 Hz, 1H), 7.66 (td, J = 7.6, 1.3 Hz, 1H), 7.52 (t, J = 7.6 Hz, 1H), 7.47 (td, J = 7.4, 0.9 Hz, 1H), 4.45 (q, J = 7.1 Hz, 2H), 1.39 (t, J = 7.1 Hz, 3H). LC-MS (ESI): m/z = 253.1 [M+1]⁺, t_{R} = 4.72 min.

**Ethyl 9-hydroxy-9H-fluorene-4-carboxylate (K36b).** The reaction was conducted following the general procedure **N,** using **K36a** (17 mg, 0.07 mmol) in MeOH, NaBH₄ (8 mg, 0.21 mmol). White solid (10 mg, 0.04 mmol, 57%).¹H NMR (600 MHz, Chloroform-d) δ 8.19 (d, J = 7.5 Hz, 1H), 7.74 (dd, J = 12.4, 7.4 Hz, 2H), 7.60 (d, J = 7.1 Hz, 1H), 7.31 (dq, J = 14.0, 7.0 Hz, 3H), 5.48 (s, 1H), 4.42 (q, J = 7.2 Hz, 2H), 1.38 (t, J = 7.1 Hz, 3H). LC-MS (ESI): m/z = 255.1 [M+1]⁺, t_{R} = 4.11 min.

**Ethyl 9-fluoro-9H-fluorene-4-carboxylate (K36c).** A solution of K**36b** (220 mg, 0.87 mmol, 1.00 equiv.) in 2 mL DCM was added Deoxo-fluor (50% in toluene, 2.7 M), 708 µL (1.91 mmol, 2.20 equiv.) dropwise at -20 °C. Then the mixture was moved to room temperature and stirred for 30 min. After that, the reaction mixture was poured into cooled water, neutralized with aq. NaHCO₃, and extracted with DCM. The combined organic phase was dried over MgSO₄, filtered and evaporated under reduced pressure. The crude was purified by column chromatography on silica gel (heptane: ethyl acetate). **K36c** was obtained as white solid (143 mg, 0.56 mmol, 64%). ¹H NMR (400 MHz, DMSO-*d*₆) δ 8.11 (dt, *J* = 7.8, 0.9 Hz, 1H), 7.92 - 7.80 (m, 2H), 7.71 (dddt, *J* = 7.2, 2.2, 1.5, 0.8 Hz, 1H), 7.55 - 7.34 (m, 3H), 6.53 (d, *J* = 53.4 Hz, 1H), 4.45 (q, *J* = 7.1 Hz, 2H), 1.38 (t, *J* = 7.1 Hz, 3H). LC-MS (ESI): m/z = 257.1 [M+1]⁺. t_{R} = 4.83 min.

**9-fluoro-9H-fluorene-4-carboxylic acid (K36d).** The reaction was conducted following the general procedure **L,** using **K36c** (143 mg, 0.56 mmol), ethanol (650 µL), and water (325 µL), 1 M solution of NaOH (1.12 mL 1.12 mmol). White solid (21 mg, 0.092 mmol, 16%). ¹H NMR (600 MHz, DMSO-*d*₆) δ 13.44 (s, 1H), 8.26 (d, *J* = 7.8 Hz, 1H), 7.87 - 7.79 (m, 2H), 7.70 (dt, *J* = 7.5, 1.5 Hz, 1H), 7.50 (tt, *J* = 7.6, 1.4 Hz, 1H), 7.49 - 7.46 (m, 1H), 7.43 (td, *J* = 7.4, 1.2 Hz, 1H), 6.51 (d, *J=* 53.7 Hz, 1H). ¹⁹F NMR (376 MHz, DMSO-*d*₆) δ -183.82 (d, *J* = 53.6 Hz). LC-MS (ESI) LC-MS (ESI): m/z = 227.1 [M-1]⁻, t_{R} = 3.95 min.

**2-(9-fluoro-9H-fluorene-4-carboxamido)-2-(1,2,3,4-tetrahydroisoquinolin-7-yl)acetic acid (K36e).** The reaction was conducted following the general procedure **M,** using EDCI (26 mg, 0.140 mmol) and HOBt (18 mg, 0.140 mmol), **K1g** (30 mg, 0.093 mmol), **K36d** (21 mg, 0.093 mmol), DIPEA (35 µL, 26 mg, 0.204 mmol). Intermediate was obtained as detected by LCMS: m/z = 541.2 [M+1]⁺, t_{R} = 4.76 min. Additional 6 M NaOH (31µL, 2.00 equiv.) was added.White solid (27 mg, 0.064 mmol, 68%).¹H NMR (600 MHz, DMSO-d6) δ 13.08 (s, 1H), 9.41 (d, J = 7.5 Hz, 1H), 9.04 (br s, 1H), 7.78 - 7.68 (m, 2H), 7.68 - 7.65 (m, 1H), 7.48 - 7.27 (m, 6H), 7.26 (d, J = 8.0 Hz, 1H), 6.51 (dd, J = 53.6, 2.2 Hz, 1H), 5.65 (dd, J = 11.0, 7.5 Hz, 1H), 4.35 - 4.23 (m, 2H), 3.41 (t, J = 6.4 Hz, 2H), 3.01 (t, J = 6.3 Hz, 2H). LC-MS (ESI): m/z = 417.2 [M+1]⁺, t_{R} = 2.93 min.

**Ethyl 2-(9-fluoro-9H-fluorene-4-carboxamido)-2-(1,2,3,4-tetrahydroisoquinolin-7-yl)acetate (K36f).** The reaction was conducted following the general procedure **E,** using **K36e** (27 mg, 0.062 mmol), TMS-Cl (16 µL, 0.125 mmol), ethanol (100 µL). White solid (19 mg, 0.04 mmol, 69%). LCMS (ESI): m/z = 445.3 [M+1]⁺, t_{R} = 3.17 min.

**2-(2-((3-cyano-4-ethoxyphenyl)sulfonyl)-1,2,3,4-tetrahydroisoquinolin-7-yl)-2-(9-fluoro-9H-fluorene-4-carboxamido)acetic acid (K36).** The reaction was conducted following the general procedure **J,** using **K36f** (19 mg, 0.042 mmol), Et₃N (11 mg, 16 µL, 0.114 mmol), 3-cyano-4-fluorobenzenesulfonyl chloride (9 mg, 0.042 mmol), 2 mL of DCM. The crude intermediate was obtained. LC-MS (ESI): m/z = 628.2 [M+1]⁺, t_{R} = 4.80 min. The subsequent reaction was conducted in the initial flask according to general procedure **L,** using the intermediate (assumed as 0.042 mmol), ethanol (0.5 mL) and water (0.25 mL), 6 M NaOH (14 µL, 0.08 mmol). White solid (3 mg, 0.005 mmol, 11%; mix of four stereoisomers). ¹H NMR (600 MHz, DMSO-d6) δ 13.52 (s, 1H), 9.32 (d, J = 7.3 Hz, 1H), 8.21 (d, J = 2.3 Hz, 1H), 8.06 (dt, J = 9.0, 2.3 Hz, 1H), 7.76 - 7.63 (m, 3H), 7.48 - 7.25 (m, 7H), 7.15 (d, J = 7.9 Hz, 1H), 6.49 (d, J = 53.7 Hz, 1H), 5.58 (dd, J = 11.4, 7.2 Hz, 1H), 4.29 (qd, J = 7.0, 3.5 Hz, 2H), 4.20 (d, J = 5.4 Hz, 2H), 3.33 (ddq, J = 28.4, 12.1, 6.0 Hz, 2H), 2.87 (t, J = 6.1 Hz, 2H), 1.38 (t, J = 6.9 Hz, 3H). ¹³C NMR (151 MHz, DMSO-*d*₆) δ 171.71, 171.66, 168.04, 168.00, 163.16, 141.54 (d, *J =* 15.59 Hz), 141.43 (d, *J* = 15.62 Hz), 140.99 (d, *J* = 15.3 Hz), 140.97 (d, *J* = 15.4 Hz),138.89 (m),136.93 (d, *J* = 2.3 Hz), 136.88 (d, *J* = 2.4 Hz), 134.48, 134.39, 133.41, 132.95, 132.92, 131.76, 131.66, 131.64, 130.04, 129.53, 129.49, 128.85, 128.33, 128.01, 127.98, 127.68, 126.93, 126.58, 126.16, 126.13, 125.73, 125.69, 123.70, 123.58, 115.02, 113.69, 101.53, 91.61 (d, *J =* 175.1 Hz), 91.56 (d, *J =* 175.4 Hz), 65.63, 56.69, 56.61, 47.34, 43.47, 27.75, 14.15. LC-MS (ESI): m/z = 626.2 [M+1]⁺, t_{R} = 4.42 min. Purity > 95% (UV).

**2-(2-((3-bromophenyl)sulfonyl)-1,2,3,4-tetrahydroisoquinolin-7-yl)-2-(9-fluoro-9H-fluorene-4-carboxamido)acetic acid (K37).** The reaction was conducted following the general procedure **J,** using **K36f** (17 mg, 0.038 mmol), Et₃N (11 mg, 16 µL, 0.114 mmol), 3-bromo-benzenesulfonyl chloride (10 mg, 0.038 mmol), 2 mL of DCM. The crude intermediate was obtained. LC-MS: t_{R} = 5.038 min. The subsequent reaction was conducted in the initial flask according to general procedure **L,** using the crude intermediate, ethanol (0.5 mL) and water (0.25 mL), 6M NaOH (13 µL, 0.08 mmol). White solid (4 mg product, 0.006 mmol, 16%%; mix of four stereoisomers) ¹H NMR (600 MHz, DMSO-d6) δ 12.97 (s, 1H), 9.31 (d, J = 7.2 Hz, 1H), 7.96 (s, 1H), 7.91 (d, J = 8.0 Hz, 1H), 7.84 (d, J = 7.8 Hz, 1H), 7.74 (t, J = 8.4 Hz, 1H), 7.66 (d, J = 7.3 Hz, 1H), 7.58 (t, J = 8.0 Hz, 1H), 7.43 (dq, J = 14.7, 7.7 Hz, 2H), 7.35 (d, J = 6.6 Hz, 1H), 7.33 - 7.25 (m, 4H), 7.15 (d, J = 7.8 Hz, 1H), 6.49 (d, J = 53.6 Hz, 1H), 5.61 - 5.55 (m, 1H), 4.25 (t, J = 11.8 Hz, 2H), 3.40 - 3.23 (m, 2H), 2.86 (d, J = 6.4 Hz, 2H). ¹³C NMR (151 MHz, DMSO) δ 171.70, 171.64, 168.04, 167.99, 141.54 (d, *J=* 16.5 Hz),141.43 (d, *J=* 16.2 Hz),140.99 (d, *J=* 15.1 Hz),140.96 (d, *J =* 15.4 Hz), 138.89, 138.04, 136.93 (d, *J=* 2.3 Hz), 136.88 (d, *J* = 2.4 Hz),136.11, 134.48, 134.38, 132.93, 132.90, 131.76, 131.65, 131.59, 131.57, 130.03, 129.60, 129.54, 129.49, 128.87, 128.34, 127.68, 127.55, 126.93, 126.63, 126.48, 126.14, 126.10, 125.74, 125.70, 123.70, 123.58, 122.44, 91.61 (d, *J* = 175.6 Hz),91.56 (d, *J* = 175.6 Hz), 56.68, 56.60, 47.27, 43.48, 27.73. LC-MS (ESI): m/z = 635.1 [M+1]⁺, t_{R} = 4.58 min. Purity > 95% (UV).

**Ethyl 2-(2-((3-cyano-4-fluorophenyl)sulfonyl)-1,2,3,4-tetrahydroisoquinolin-7-yl)-2-(spiro[fluorene-9,2'-[1,3]dithiolane]-4-carboxamido)acetate (K38a).** The reaction was conducted following the general procedure **E,** using compound **K27** (40 mg, 0.07 mmol), ethanol (85 µL), TMS-Cl (23 µL, 0.14 mmol). Afforded residue (treated as 0.07 mmol, 1.0 equvi.) was redissolved in 1.4 mL DCM. To such a solution was added the 1,2-ethanedithiol (29ul ,0.34 mmol, 5.0 equvi.) followed by boron trifluoride etherate (70 µL, 0.080 mmol, 1.15 equvi.). The solution was stirred at room temperature overnight. Then, H₂O (2 mL) and DCM (3 mL) were added to the solution. The organic layer was separated, washed with H₂O (2 × 5 mL) and saturated NaCl solution (2 × 5 mL), dried over MgSO₄, and concentrated under reduced pressure to give an orange crude residue 34 mg as starting material in next step without further purification. LC-MS (ESI): m/z = 700.1 [M + H]⁺, t_{R} = 4.85 min.

**2-(2-((3-cyano-4-ethoxyphenyl)sulfonyl)-1,2,3,4-tetrahydroisoquinolin-7-yl)-2-(spiro[fluorene-9,2'-[1,3]dithiolane]-4-carboxamido)acetic acid (K38).** The reaction was conducted following the general procedure **J,** using crude **K38a** (34 mg), ethanol (100 µL), and water (50 µL), 1M solution of NaOH (100 µL, 0.10 mmol). Yellow solid (3.2 mg, 0.004 mmol, 6.5% over two steps). LC-MS (ESI): m/z = 698.1 [M + H]⁺, t_{R} = 4.506 min. ¹H NMR (600 MHz, DMSO-*d*₆) δ 12.98 (s, 1H), 9.31 (d, *J* = 7.3 Hz, 1H), 8.20 (d, *J* = 2.4 Hz, 1H), 8.05 (dd, *J* = 9.0, 2.4 Hz, 1H), 7.81 (d, *J* = 7.6 Hz, 1H), 7.71 (d, *J=* 7.7 Hz, 1H), 7.64 (d, *J* = 7.8 Hz, 1H), 7.42 - 7.37 (m, 2H), 7.35 - 7.26 (m, 4H), 7.20 - 7.13 (m, 2H), 5.57 (d, *J* = 7.3 Hz, 1H), 4.28 (q, *J* = 7.0 Hz, 2H), 4.20 (d, *J* = 3.3 Hz, 2H), 3.85 (s, 4H), 3.32 - 3.25 (m, 2H), 2.86 (t, *J* = 6.0 Hz, 2H), 1.37 (t, *J =* 7.0 Hz, 3H). ¹³C NMR (151 MHz, DMSO-*d*₆) δ 171.66, 168.25, 163.13, 151.14, 150.39, 136.24, 134.45, 134.37, 134.16, 133.39, 132.88, 131.61, 131.31, 128.81, 128.53, 128.19, 127.98, 127.80, 127.70, 126.54, 126.41, 126.12, 125.13, 123.03, 115.00, 113.67, 101.50, 67.68, 65.61, 56.58, 47.32, 43.44, 42.14, 42.10, 27.73, 14.14. Purity> 95% (UV).

**Ethyl spiro[fluorene-9,2'-[1,3]dithiolane]-4-carboxylate (K39a).** The reaction was conducted following the general procedure **O,** using **K38a** (504 mg, 2 mmol) in 40 mL of DCM, 1,2-ethanedithiol (841 µL, 10 mmol), boron trifluoride etherate (2 mL). Yellow oil (695 mg, 1.9 mmol, 95%). ¹H NMR (400 MHz, DMSO-*d*₆) δ 8.04 - 7.94 (m, 2H), 7.82 - 7.74 (m, 1H), 7.71 (dd, *J* = 7.7, 1.2 Hz, 1H), 7.51 - 7.35 (m, 3H), 4.44 (q, *J* = 7.1 Hz, 2H), 3.88 (s, 4H), 1.37 (t, *J* = 7.1 Hz, 3H). LC-MS (ESI): m/z = 329.1 [M+1]⁺, t_{R} = 5.06 min.

**Ethyl 9,9-difluoro-9H-fluorene-4-carboxylate (K39b).** To a solution of **K39a** (100 mg, 0.30 mmol, 1.0 equiv.) in DCM 630 µL was added N-bromosuccinimide (14 mg, 0.08mmol, 0.26 equiv.). After 5 minutes DAST (66 µL, 0.9 mmol, 3.0 equiv.) was added and the mixture stirred for 16 h. DCM was added and the combined organics washed with sat. NaHCOs, dried MgSO₄ and the solvent was removed *in vacuo.* The residue was purified by normal phase chromatography (heptane: ethyl acetate) to give, the title compound **K39b** as white solid (122 mg, quantitative). ¹H NMR (400 MHz, DMSO-*d*₆) δ 8.14 (dt, *J* = 7.8, 0.9 Hz, 1H), 7.95 (ddd, *J* = 7.7, 4.5, 1.2 Hz, 2H), 7.78 (ddd, *J* = 7.4, 2.3, 1.2 Hz, 1H), 7.67 - 7.48 (m, 3H), 4.45 (q, *J* = 7.1 Hz, 2H), 1.38 (t, *J =* 7.1 Hz, 3H). ¹⁹F NMR (376 MHz, DMSO-*d*₆) δ -109.80. LC-MS (ESI): m/z = 297.2 [M+23]⁺, t_{R} = 4.57 min.

**9,9-difluoro-9H-fluorene-4-carboxylic acid (K39c).** The reaction was conducted following the general procedure L, using **K39b** (122 mg, 0.44 mmol), ethanol (510 µL), and water (255 µL). 6 M NaOH (146 µL, 0.88 mmol). White solid (14 mg, 0.057 mmol, 13%). ¹H NMR (600 MHz, DMSO-*d*₆) δ 13.68 (s, 1H), 8.28 (d, *J* = 7.9 Hz, 1H), 7.97 - 7.90 (m, 2H), 7.77 (dd, *J* = 7.5, 1.6 Hz, 1H), 7.62 (td, *J* = 7.7, 1.2 Hz, 1H), 7.55 (t, *J=* 7.6 Hz, 1H), 7.51 (td, *J* = 7.5, 1.0 Hz, 1H). LC-MS (ESI): m/z = 245.0 [*M*-1]⁻, t_{R} = 4.16 min.

**2-(9,9-difluoro-9H-fluorene-4-carboxamido)-2-(1,2,3,4-tetrahydroisoquinolin-7-yl)acetic acid (K39d).** The reaction was conducted following the general procedure **M,** using EDCI (6.9 mg, 0.036 mmol) and HOBt (4.9 mg, 0.036 mmol), **K1g** (8 mg, 0.024 mmol), **K39c** (6.9 mg, 0.029 mmol) and DIPEA (10 µL, 6.8 mg, 0.053 mmol). The intermediate was obtained as detected by LC-MS (ESI): m/z =576.2 [M+18]⁺, t_{R} = 4.89 min. Following by the addition of ethanol (150 µL), 6 M NaOH (27 µL). White solid (6 mg, 0.014 mmol, 57%). ¹H NMR (600 MHz, DMSO-d6) δ 13.12 (s, 1H), 9.51 (d, J = 7.4 Hz, 1H), 9.05 (br, 1H), 7.81 (d, J = 7.3 Hz, 1H), 7.73 (dd, J = 5.5, 3.3 Hz, 2H), 7.54 (d, J = 7.6 Hz, 1H), 7.50 (t, J = 7.5 Hz, 1H), 7.45 (dd, J = 5.6, 3.1 Hz, 2H), 7.41 (dd, J = 7.9, 1.9 Hz, 1H), 7.37 (d, J = 1.8 Hz, 1H), 7.27 (d, J = 8.0 Hz, 1H), 5.63 (d, J = 7.3 Hz, 1H), 4.33 (d, J = 15.8 Hz, 1H), 4.26 (d, J = 15.8 Hz, 1H), 3.40 (d, J = 6.4 Hz, 3H), 3.01 (t, J = 6.4 Hz, 2H). ¹³C NMR (151 MHz, DMSO-d6) δ 171.99, 167.88, 135.51, 133.01, 132.66, 132.30, 132.24, 130.01, 129.51, 129.45, 127.61, 126.94, 125.14, 124.54, 124.02, 57.04, 44.38, 41.25, 24.94. LC-MS (ESI): m/z = 435.2 [M+1]⁺, t_{R} =3.15 min.

**Ethyl 2-(9,9-difluoro-9H-fluorene-4-carboxamido)-2-(1,2,3,4-tetrahydroisoquinolin-7-yl)acetate (K39e).** The reaction was conducted following the general procedure **E,** using **K39d** (15 mg, 0.03 mmol), TMS-Cl (8 µL, 0.06 mmol), 200 µL of ethanol. White solid. LC-MS (ESI): m/z = 463.3 [M+1] ⁺, t_{R} = 3.39 min.

**2-(2-((3-cyano-4-ethoxyphenyl)sulfonyl)-1,2,3,4-tetrahydroisoquinolin-7-yl)-2-(9,9-difluoro-9H-fluorene-4-carboxamido)acetic acid (K39).** The reaction was conducted following the general procedure J, using **K39e** (0.03 mmol), Et₃N (11 mg, 16 µL, 0.114 mmol), 3-cyano-4-fluorobenzenesulfonyl chloride (7 mg, 0.03 mmol), 2 mL of DCM, the intermediate was obtained. LC-MS (ESI): m/z = 646.2 [M+1]⁺, t_{R} = 4.91 min. The subsequent reaction was conducted according to general procedure **L,** using the intermediate (assumed as 0.03 mmol), ethanol (0.5 mL) and water (0.25 mL), 6 M NaOH (10 µL, 0.06 mmol). White solid (2.6 mg product, 0.004 mmol, 13%). ¹H NMR (600 MHz, DMSO-d6) δ 12.98 (s, 1H), 9.42 (d, J = 7.1 Hz, 1H), 8.20 (d, J = 2.3 Hz, 1H), 8.06 (dd, J = 9.0, 2.4 Hz, 1H), 7.80 (d, J = 7.4 Hz, 1H), 7.72 (d, J = 7.6 Hz, 2H), 7.54 (d, J = 7.6 Hz, 1H), 7.49 (t, J = 7.6 Hz, 1H), 7.42 (dd, J = 8.6, 6.6 Hz, 3H), 7.29 (s, 1H), 7.16 (d, J = 7.9 Hz, 1H), 5.55 (d, J = 7.1 Hz, 1H), 4.29 (q, J = 6.9 Hz, 2H), 4.25 - 4.16 (m, 2H), 3.00 (s, 3H), 2.87 (t, J = 6.0 Hz, 2H), 1.38 (t, J = 6.9 Hz, 3H). ¹³C NMR (151 MHz, DMSO-d6) δ 171.59, 167.42, 163.15, 137.42 (d, J = 22.4 Hz), 136.80, 135.50, 135.48, 134.38, 134.23, 133.41, 133.03, 132.46, 132.21, 131.77, 131.72, 129.47, 128.93, 128.89, 128.25 (d, J = 21.6 Hz), 128.00, 126.56, 126.15, 124.58, 124.06, 123.48, 115.01, 113.69, 101.52, 65.62, 56.72, 47.33, 43.45, , 40.54, 27.74, 14.14. LC-MS (ESI): m/z = 644.2 [M+1]⁺, t_{R} = 4.53 min. Purity > 95% (UV).

**Methyl 9H-carbazole-4-carboxylate (K40a)** A mixture of methyl 1H-indole-4-carboxylate (1.0 g, 5.7 mmol, 1.0 equiv.) and 2,5-dimethoxytetrahydrofuran (0.98 g, 7.45 mmol, 1.3 equiv.) in MeOH (25 mL) was added TsOH·H₂O (0.5g, 2.85 mmol, 0.5 equiv.). The reaction mixture was stirred at 65°C for 16 h. The reaction mixtures were concentrated. The crude product was purified by silica gel column chromatography (heptane: ethyl acetate) to give the titled compound 70 mg (0.3 mmol, 5%) as brown solid. ¹H NMR (400 MHz, DMSO-*d*₆) δ 11.67 (s, 1H), 8.69 (d, *J* = 8.2 Hz, 1H), 7.77 (dd, *J=* 11.7, 7.8 Hz, 2H), 7.50 (ddd, *J* = 23.6, 15.6, 7.9 Hz, 3H), 7.18 (t, *J=* 7.6 Hz, 1H), 4.00 (s, 3H). LC-MS (ESI): m/z = 226.1 [M+1]⁺, t_{R} = 4.24 min.

**Methyl 9-methyl-9H-carbazole-4-carboxylate (K40b)** A solution of compound (45 mg, 0.20 mmol, 1.0 equiv.) in DMF (0.45 mL) was added NaH (12 mg, 0.3 mmol, 1.50 equiv.) at 0 °C. The reaction mixture was stirred at 0 °C for 0.5 h. Then Mel (56 mg, 25 µL, 2.0 equiv.) was added to the reaction mixture. The reaction mixture was allowed to warm to 15 °C with stirring for 16h. Saturated NH₄Cl (2 mL) was added to the reaction mixture. The product was extracted with EtOAc (3 mL × 2) and washed by water (2 mL × 2). The combined organic layer was concentrated to give compound 28 mg (0.12 mmol, 60%) as brown oil. The crude product was treated as starting material in next step without further purification. LC-MS (ESI): m/z = 240.1 [M+1]⁺, t_{R} = 4.72 min.

**Methyl 9-methyl-9H-carbazole4-carboxylic acid (K40c)** A round bottom flask was charged with above mentioned product (28 mg, 0.12 mmol), methanol (1 mL), and water (0.2 mL). 6 M solution of NaOH (40 µL, 1.6 mmol) was added in one portion and the reaction mixture was stirred for overnight at 50 °C. The reaction was concentrated in *vacuo.* The crude product is purified by revered phase on C18 column with standard solution A (95% water, 5% ACN and 0.1% TFA) and solution B (95% ACN, 5% water and 0.1% TFA). 17 mg (0.07 mmol, 58%) brown solid was obtained. ¹H NMR (400 MHz, DMSO-d6) δ 13.08 (s, 1H), 8.84 (d, J = 8.1 Hz, 1H), 7.89 (d, J = 8.1 Hz, 1H), 7.79 (d, J = 7.4 Hz, 1H), 7.65 (d, J = 8.2 Hz, 1H), 7.55 (dt, J = 11.4, 7.8 Hz, 2H), 7.22 (t, J = 7.6 Hz, 1H), 3.94 (s, 3H). LC-MS (ESI): m/z = 226.1 [M+1]⁺, t_{R} = 4.02 min.

**2-(9-methyl-9H-carbazole-4-carboxamido)-2-(1,2,3,4-tetrahydroisoquinolin-7-yl)acetic acid (K40d).** The reaction was conducted following the general procedure **H,** using EDCI (37.4 mg, 0.195 mmol), HOBt (26.34 mg, 0.263 mmol), **K1g** (44.5 mg, 0.13 mmol), **K40c** (30.3 mg, 0.13 mmol), DIPEA (52 µL, 38 mg, 0.3 mmol). The intermediate was obtained as brown oil (assumed as 0.013 mmol). ¹H NMR (400 MHz, DMSO-*d*₆) δ 9.57 (dd, *J* = 6.8, 3.0 Hz, 1H), 7.73 (ddt, *J* = 9.3, 7.3, 0.9 Hz, 2H), 7.69 - 7.62 (m, 1H), 7.58 (dd, *J* = 7.7, 1.2 Hz, 1H), 7.51 (tdd, *J* = 7.6, 5.0, 1.3 Hz, 1H), 7.45 (t, *J =* 7.5 Hz, 1H), 7.40 (ddd, *J* = 7.3, 5.4, 1.7 Hz, 2H), 7.39 - 7.30 (m, 1H), 7.26 (dd, *J* = 8.0, 4.8 Hz, 1H), 5.67 (dd, *J* = 6.9, 1.4 Hz, 1H), 4.77 (d, *J* = 8.2 Hz, 2H), 4.29 - 4.11 (m, 2H), 3.83 (dd, *J* = 9.8, 5.1 Hz, 2H), 2.94 (dt, *J* = 10.8, 6.0 Hz, 2H), 1.20 (td, *J* = 7.1, 1.8 Hz, 3H). LC-MS (ESI): m/z = 538.2 [M+1]⁺, t_{R} = 4.71 min. The reaction was conducted in the initial flask following the general procedure **I,** using intermediate (around 0.013 mmol), ethanol (500 µL), 6 M NaOH (120 µL). Brown solid (24 mg, 0.058 mmol, 44.6% over two steps). ¹H NMR (600 MHz, DMSO-d6) δ 13.02 (s, 1H), 9.19 (d, J = 7.6 Hz, 1H), 8.95 (br s, 1H), 8.24 - 8.21 (m, 1H), 7.74 (dd, J = 8.3, 0.9 Hz, 1H), 7.61 (d, J = 8.2 Hz, 1H), 7.51 (dd, J = 8.2, 7.3 Hz, 1H), 7.46 (ddd, J = 8.3, 4.6, 2.7 Hz, 2H), 7.42 - 7.39 (m, 1H), 7.32 (dd, J = 7.3, 0.9 Hz, 1H), 7.26 (d, J = 8.0 Hz, 1H), 7.11 - 7.08 (m, 1H), 5.72 (d, J = 7.6 Hz, 1H), 4.29 (q, J = 15.9 Hz, 2H), 3.91 (s, 3H), 3.51 - 3.37 (m, 2H), 3.00 (t, J = 6.3 Hz, 2H). ¹³C NMR (151 MHz, DMSO-d6) δ 171.78, 168.71, 140.91, 140.87, 135.64, 131.54, 130.34, 128.91, 128.81, 127.20, 126.45, 126.01, 124.83, 123.55, 120.67, 118.80, 118.52, 118.46, 110.98, 108.95, 56.43, 43.98, 40.81, 29.05, 24.44. LC-MS (ESI): m/z = 414.2 [M+1]⁺, t_{R} = 3.052 min.

**2-(2-((3-cyano-4-fluorophenyl)sulfonyl)-1,2,3,4-tetrahydroisoquinolin-7-yl)-2-(9-methyl-9H-carbazole-4-carboxamido)acetic acid (K40).** The reaction was conducted following the general procedure **K,** using **K40d** (10 mg, 0.02 mmol), 3-cyano-4-fluorobenzenesulfonyl chloride (6 mg, 0.02 mmol), 2 mL of pyridine. White (4.3 mg, 0.007 mmol, 36%). ¹H NMR (400 MHz, DMSO-d6) δ 12.95 (s, 1H), 9.12 (d, J = 7.3 Hz, 1H), 8.50 - 8.43 (m, 1H), 8.20 (d, J = 7.8 Hz, 2H), 7.74 (dd, J = 11.4, 8.3 Hz, 2H), 7.60 (d, J = 8.2 Hz, 1H), 7.48 (dt, J = 20.1, 7.8 Hz, 2H), 7.39 - 7.29 (m, 3H), 7.16 (d, J = 7.8 Hz, 1H), 7.06 (t, J = 7.6 Hz, 1H), 5.65 (d, J = 7.3 Hz, 1H), 4.26 (s, 2H), 3.91 (s, 3H), 3.38 (dtd, J = 18.2, 11.9, 5.8 Hz, 2H), 2.89 (d, J = 6.0 Hz, 2H).¹³C NMR (151 MHz, DMSO-d6) δ 171.88, 168.83, 164.79 (d, J = 262.7 Hz), 140.92, 140.87, 135.29 (d, J = 10.3 Hz), 134.86, 133.77, 133.46 (d, J = 3.3 Hz), 132.76, 131.47, 130.42, 128.84, 126.68, 126.12, 125.99, 124.86, 123.56, 120.71, 118.81, 118.50, 118.47, 117.99 (d, J = 21.0 Hz), 112.81, 110.91, 108.92, 101.96 (d, J = 16.5 Hz), 56.65, 47.27, 43.46, 29.06, 27.72. LC-MS (ESI): m/z = 597.2 [M+1]⁺, t_{R} = 4.34 min.

**Ethyl 2-(9-methyl-9H-carbazole-4-carboxamido)-2-(1,2,3,4-tetrahydroisoquinolin-7-yl)acetate (K40e).** The reaction was conducted following the general procedure **E,** using **K40d** (7 mg, 0.062 mmol), TMS-Cl (136 µL, 1.25 mmol), ethanol (1 mL). Brown oil (26 mg, 0.58 mmol, 95%). ¹H NMR (600 MHz, DMSO-d6) δ 9.70 (br s, 1H), 9.36 (d, J = 7.2 Hz, 1H), 8.23 (d, J = 7.9 Hz, 1H), 7.75 - 7.71 (m, 1H), 7.60 (d, J = 8.2 Hz, 1H), 7.53 - 7.44 (m, 2H), 7.44 - 7.38 (m, 2H), 7.35 - 7.29 (m, 1H), 7.25 (d, J = 8.0 Hz, 1H), 7.14 - 7.10 (m, 1H), 5.75 (d, J = 7.1 Hz, 1H), 4.27 - 4.11 (m, 4H), 3.90 (s, 3H), 3.34 (d, J = 5.6 Hz, 2H), 3.02 (t, J = 6.2 Hz, 2H), 1.20 (t, J = 7.1 Hz, 3H). ¹³C NMR (151 MHz, DMSO-d6) δ 170.47, 169.02, 140.95, 140.88, 134.57, 132.12, 130.16, 129.08, 129.02, 127.23, 126.57, 126.08, 124.88, 123.50, 120.69, 118.82, 118.60 (2C), 111.06, 109.00, 61.15, 56.70, 43.43, 40.45, 29.09, 24.45, 14.04. LC-MS (ESI): m/z = 442.3 [M+1]⁺, t_{R} = 4.31 min.

**2-(2-((3-cyano-4-ethoxyphenyl)sulfonyl)-1,2,3,4-tetrahydroisoquinolin-7-yl)-2-(9-methyl-9H-carbazole-4-carboxamido)acetic acid (K41).** The reaction was conducted following the general procedure **J,** using **K40e** (8 mg, 0.02 mmol), Et₃N (7 mg, 10 µL, 0.06 mmol), 3-cyano-4-fluorobenzenesulfonyl chloride (4 mg, 0.02 mmol), 2 mL of DCM. The intermediate was obtained. LC-MS (ESI): m/z = 625.2 [M+1]⁺, t_{R} = 4.85 min. The subsequent reaction was conducted in the initial flask according to general procedure **L,** using intermediate (assumed as 0.02 mmol), ethanol (0.5 mL) and water (0.25 mL), 6M NaOH (7 µL, 0.04 mmol). White solid (3 mg, 0.005 mmol, 23%). ¹H NMR (600 MHz, DMSO-d6) δ 12.96 (s, 1H), 9.12 (d, J = 7.3 Hz, 1H), 8.22 - 8.17 (m, 2H), 8.05 (dd, J = 8.9, 2.4 Hz, 1H), 7.73 (d, J = 8.2 Hz, 1H), 7.60 (d, J = 8.2 Hz, 1H), 7.51 (t, J = 7.7 Hz, 1H), 7.45 (t, J = 7.6 Hz, 1H), 7.40 (d, J = 9.0 Hz, 1H), 7.34 (q, J = 7.0 Hz, 3H), 7.15 (d, J = 8.0 Hz, 1H), 7.06 (t, J = 7.5 Hz, 1H), 5.65 (d, J = 7.3 Hz, 1H), 4.28 (q, J = 7.0 Hz, 2H), 4.21 (s, 2H), 3.91 (s, 3H), 3.32 (dp, J = 23.9, 6.0 Hz, 2H), 2.87 (t, J = 6.0 Hz, 2H), 1.37 (t, J = 7.0 Hz, 3H). ¹³C NMR (151 MHz, DMSO-*d*₆) δ 171.86, 168.78, 163.14, 140.89, 140.84, 134.80, 134.37, 133.38, 132.77, 131.57, 130.41, 128.78, 128.00, 126.60, 126.15, 125.95, 124.83, 123.54, 120.69, 118.79, 118.47, 118.45, 115.02, 113.69, 110.88, 108.89, 101.50, 65.60, 56.63, 47.34, 43.47, 29.04, 27.74, 14.14. LC-MS (ESI): m/z = 623.2 [M+1]⁺, t_{R} = 4.43 min.

**2-(2-((3-chloro-4-fluorophenyl)sulfonyl)-1,2,3,4-tetrahydroisoquinolin-7-yl)-2-(9-methyl-9H-carbazole-4-carboxamido)acetic acid (K42).** The reaction was conducted following the general procedure **J,** using **K40e** (10 mg, 0.02 mmol), Et₃N (11 mg, 16 µL, 0.06 mmol), 3-chloro-4-fluorobenzenesulfonyl chloride (4 mg, 2.8 µL, 0.02 mmol) in 2 mL of DCM, the intermediate was obtained. LC-MS (ESI): m/z = 634.1 [M+1]⁺, t_{R} = 5.11 min. The subsequent reaction was conducted in the initial flask according to general procedure **L,** using crude intermediate (assumed as 0.02 mmol), ethanol (0.5 mL) and water (0.25 mL), 6M NaOH (7 µL, 0.04 mmol). White solid (3 mg, 0.005 mmol, 23%). ¹H NMR (600 MHz, DMSO-d6) δ 12.94 (s, 1H), 9.11 (d, J = 7.4 Hz, 1H), 8.20 (d, J = 8.0 Hz, 1H), 8.05 (d, J = 6.6 Hz, 1H), 7.89 - 7.84 (m, 1H), 7.73 (d, J = 8.2 Hz, 1H), 7.66 (t, J = 8.9 Hz, 1H), 7.60 (d, J = 8.2 Hz, 1H), 7.51 (t, J = 7.8 Hz, 1H), 7.45 (t, J = 7.8 Hz, 1H), 7.35 (s, 2H), 7.32 (d, J = 7.0 Hz, 1H), 7.15 (d, J = 7.8 Hz, 1H), 7.06 (t, J = 7.6 Hz, 1H), 5.65 (d, J = 7.4 Hz, 1H), 4.28 - 4.22 (m, 2H), 3.91 (s, 3H), 3.39 (d, J = 5.3 Hz, 2H), 2.87 (t, J = 5.9 Hz, 2H). ¹³C NMR (151 MHz, DMSO-*d*₆) δ 171.87, 168.79, 159.91 (d, *J* = 254.0 Hz), 140.90, 140.85, 134.86, 133.55, 132.73, 131.51, 130.43, 129.98, 128.94 (d, *J* = 8.6 Hz), 128.80, 126.64, 126.12, 125.96, 124.84, 123.54, 121.09 (d, *J =* 18.4 Hz), 120.70, 118.80, 118.48, 118.45, 118.12 (d, *J =* 22.6 Hz), 110.89, 108.90, 56.64, 47.29, 43.47, 29.04, 27.69. LC-MS (ESI): m/z = 604.1 [M-1]⁻, t_{R} = 4.64 min. Purity > 95% (UV).

**2-(2-((3-cyanophenyl)sulfonyl)-1,2,3,4-tetrahydroisoquinolin-7-yl)-2-(9-methyl-9H-carbazole-4-carboxamido)acetic acid (K43).** The reaction was conducted following the general procedure **J,** using **K40e** (14 mg, 0.03 mmol), Et₃N (9 mg, 13 µL, 0.09 mmol), 3-cyano-benzenesulfonyl chloride (6 mg, 0.03 mmol), 2 mL of DCM. The intermediate was obtained. LC-MS (ESI): m/z = 607.3 [M+1]⁺, t_{R} = 4.79 min. The subsequent reaction was conducted in the initial flask according to general procedure **L,** using intermediate (assumed as 0.03 mmol.), ethanol (0.5 mL) and water (0.25 mL), 6 M NaOH (10 µL, 0.06 mmol). White solid (3.36 mg, 0.005 mmol, 20%). ¹H NMR (600 MHz, DMSO-d6) δ 12.96 - 12.93 (m, 1H), 9.11 (d, J = 7.2 Hz, 1H), 8.32 (s, 1H), 8.20 (d, J = 8.0 Hz, 1H), 8.15 (dd, J = 17.3, 7.9 Hz, 2H), 7.82 (t, J = 8.0 Hz, 1H), 7.73 (d, J = 8.2 Hz, 1H), 7.60 (d, J = 8.3 Hz, 1H), 7.51 (t, J = 7.8 Hz, 1H), 7.45 (t, J = 7.8 Hz, 1H), 7.34 (q, J = 7.8, 7.3 Hz, 3H), 7.15 (d, J = 7.8 Hz, 1H), 7.06 (t, J = 7.5 Hz, 1H), 5.64 (d, J = 7.2 Hz, 1H), 4.26 (s, 2H), 3.91 (s, 3H), 3.39 (m, 2H), 2.88 (d, J = 6.1 Hz, 2H). ¹³C NMR (151 MHz, DMSO-*d*₆) δ 171.84, 168.77, 140.89, 140.84, 137.33, 136.75, 134.86, 132.72, 131.81, 131.46, 131.01, 130.79, 130.42, 128.79, 126.64, 126.09, 125.96, 124.83, 123.54, 120.69, 118.79, 118.47, 118.44, 117.48, 112.80, 110.88, 108.90, 56.65, 47.25, 43.46, 29.04, 27.73. LC-MS (ESI): m/z = 579.1 [M+1]⁺, t_{R} = 4.31 min.

**2-(2-((3-bromophenyl)sulfonyl)-1,2,3,4-tetrahydroisoquinolin-7-yl)-2-(9-methyl-9H-carbazole-4-carboxamido)acetic acid (K44).** The reaction was conducted following the general procedure **J,** using **K40e** (16 mg, 0.02 mmol), Et₃N (11 mg, 16 µL, 0.11 mmol), 3-bromobenzenesulfonyl chloride (10 mg, 0.036 mmol) in 2 mL of DCM. Intermediate was obtained. LC-MS (ESI): t_{R} = 5.09 min. The subsequent reaction was conducted in the initial flask according to general procedure **L,** using crude intermediate (assumed as 0.02 mmol), ethanol (0.5 mL) and water (0.25 mL) 6M NaOH (12 µL, 0.08 mmol). White solid (10 mg, 0.016 mmol, 44%). ¹H NMR (600 MHz, DMSO-d6) δ 12.94 (s, 1H), 9.11 (d, J = 7.4 Hz, 1H), 8.20 (d, J = 7.9 Hz, 1H), 7.96 (d, J = 1.9 Hz, 1H), 7.91 (dd, J = 8.0, 2.0 Hz, 1H), 7.84 (d, J = 7.8 Hz, 1H), 7.73 (d, J = 8.2 Hz, 1H), 7.62 - 7.55 (m, 2H), 7.51 (t, J = 7.8 Hz, 1H), 7.46 (t, J = 7.6 Hz, 1H), 7.36 (d, J = 9.3 Hz, 2H), 7.33 (d, J = 7.2 Hz, 1H), 7.15 (d, J = 7.8 Hz, 1H), 7.06 (t, J = 7.5 Hz, 1H), 5.65 (d, J = 7.3 Hz, 1H), 4.25 (d, J = 3.4 Hz, 2H), 3.91 (s, 3H), 3.36 (q, J = 6.4 Hz, 2H), 2.87 (t, J = 6.1 Hz, 2H). ¹³C NMR (151 MHz, DMSO-*d*₆) δ 171.86, 168.79, 140.89, 140.84, 138.07, 136.09, 134.78, 132.77, 131.64, 131.50, 130.42, 129.58, 128.80, 126.64, 126.46, 126.13, 125.97, 124.83, 123.54, 122.42, 120.69, 118.79, 118.47, 118.46, 110.88, 108.90, 56.62, 47.27, 43.48, 29.04, 27.72. LC-MS (ESI): m/z = 632.1 [M-1]⁻, t_{R} = 4.60 min. Purity > 95% (UV).

**7-bromo-2-(phenylsulfonyl)-1,2,3,4-tetrahydroisoquinoline (K45a).** The reaction was conducted following the general procedure **A,** using 7-bromo-1,2,3,4-tetrahydroisoquinoline (500 mg, 2.36 mmol), Et₃N (0.98 mL, 7.08 mmol), benzenesulfonyl chloride (0.3 mL, 2.36 mmol). White solid (0.78 g, 2.22 mmol, quantitative). ¹H NMR (400 MHz, DMSO-d6) δ 7.86 - 7.79 (m, 2H), 7.76 - 7.67 (m, 1H), 7.67 - 7.59 (m, 2H), 7.42 (d, J = 2.1 Hz, 1H), 7.32 (dd, J = 8.2, 2.1 Hz, 1H), 7.07 (d, J = 8.2 Hz, 1H), 4.24 (s, 2H), 3.35 - 3.28 (m, 2H), 2.79 (t, J = 6.0 Hz, 2H). LC-MS (ESI): m/z = 352.0 [M+1]⁺, t_{R} = 4.83 min.

**Methyl (E)-3-(2-(phenylsulfonyl)-1,2,3,4-tetrahydroisoquinolin-7-yl)acrylate (K45b).** To a solution of **K45a** (783 mg, 2.2 mmol, 1.0 equiv.) in DMF (1 mL) was added methyl acrylate (764 mg, 8.8 mmol, 4.0 equiv.), tri-o-tolyphosphine (202 mg, 0.67 mmol, 0.30 equiv.) and DIPEA (1150 mg, 8.8 mmol, 4.0 equiv.). The mixture was degassed with nitrogen for 20 min, followed by the addition of Pd(OAc)₂ (11.2 mg, 0.11 mmol, 0.05 equiv.) in sealed tube. The reaction mixture was stirred at 100 °C for 3 h. The reaction mixture was then cooled to 0°C, quenched with cold water, extracted with EtOAc. The organic layer was washed with brine, dried under MgSO₄ and filtered. The filtrate was evaporated and was purified by silica column chromatography (heptane: ethyl acetate) to afford the title compound **K45b** (133 mg, 0.372 mmol, 16%).¹H NMR (600 MHz, DMSO-d6) δ 7.83 (dd, J = 7.3, 1.6 Hz, 2H), 7.74 - 7.68 (m, 1H), 7.67 - 7.61 (m, 2H), 7.58 (d, J = 16.0 Hz, 1H), 7.54 (d, J = 1.8 Hz, 1H), 7.51 (dd, J = 7.9, 1.8 Hz, 1H), 7.17 (d, J = 7.9 Hz, 1H), 6.58 (d, J = 16.0 Hz, 1H), 4.23 (s, 2H), 3.72 (s, 3H), 3.33 (t, J = 5.8 Hz, 2H), 2.87 (t, J = 6.0 Hz, 2H). LC-MS (ESI): m/z = 358.1 [M+1]⁺, t_{R} = 4.59 min.

**Methyl 4-amino-3-(2-(phenylsulfonyl)-1,2,3,4-tetrahydroisoquinolin-7-yl)butanoate (K45c).** The solution of **K45b** (133 mg, 0.372 mmol, 1.0 equiv.), nitromethane (250 mg, 220 µL, 4.09 mmol, 11 equiv.) and tetrabutylammonium fluoride (0.483 mL, 0.483 mmol, 1.3 equiv.) in 1 mL of toluene was mixed and stirred at 50°C overnight. Upon reaction completion, the mixture was poured into 10 mL of 1 M HCl. The aqueous layer was extracted with toluene (2 × 15 mL). The organic phase was combined, then dried with Na₂SO₄, filtered by celite and concentrated *in vacuo* to afford crude intermediate (136 mg, assumed as 0.32 mmol, 86%). The intermediate was treated as a starting material in the next step without further purification. LC-MS (ESI): m/z = 419.1 [M+1]⁺, t_{R} = 4.51 min. The reaction was conducted by using the intermediate product, using intermediate (136 mg, assumed as 0.32 mmol, 1.0 equiv), methanol (4.32 mL), 1 M HCl (3.2 mL) and zinc dust (63 mg, 0.96 mmol, 3.0 equiv.). The zinc dust was added portion wise during 1 h. The reaction was stirred overnight. Upon reaction completion, the mixture was filtered through celite and washed several times with methanol. The filtrate was concentrated, and the compound the title compound **K45c** was obtained (52 mg, 0.133 mmol, 41%) and used as a starting material in the next step without further purification. LC-MS (ESI): m/z = 389.2 [M+1]⁺, t_{R} = 3.20 min.

**4-(9-oxo-9H-fluorene-4-carboxamido)-3-(2-(phenylsulfonyl)-1,2,3,4-tetrahydroisoquinolin-7-yl)butanoic acid (K45).** The reaction was conducted following the general procedure **H,** using EDCI (38 mg, 0.2 mmol), HOBt (27 mg, 0.200 mmol), **K45c** (52 mg, 0.133 mmol), 9-oxo-9H-fluorene-4-carboxylic acid (45 mg, 0.200 mmol) and DIPEA (51 µL, 38 mg, 0.292 mmol). Intermediate was obtained as colorless oil (73 mg, 0.12 mmol, 92%) and treated as a starting material in the next step without further purification. LC-MS (ESI): m/z = 595.1 [M+1]⁺, t_{R} = 4.52 min. The subsequent reaction was conducted following the general procedure **L,** using intermediate (assumed as 0.12 mmol), ethanol (0.14 mL), water (0.07 mL) and 6 M solution NaOH (4 µL, 0.24 mmol). Yellow solid (27 mg, 0.046 mmol, 35%). ¹H NMR (600 MHz, DMSO-d6) δ 12.05 (s, 1H), 8.71 (t, J = 5.7 Hz, 1H), 7.82 - 7.77 (m, 2H), 7.71 (t, J = 7.4 Hz, 1H), 7.66 (dd, J = 7.1, 1.4 Hz, 1H), 7.65 - 7.59 (m, 3H), 7.46 - 7.42 (m, 2H), 7.41 - 7.33 (m, 3H), 7.13 - 7.09 (m, 2H), 7.05 (d, J = 7.8 Hz, 1H), 4.14 (d, J = 15.3 Hz, 1H), 4.01 (d, J = 15.3 Hz, 1H), 3.60 - 3.45 (m, 2H), 3.37 (dq, J = 14.6, 7.1, 6.6 Hz, 1H), 3.23 (ddt, J = 81.6, 12.0, 6.0 Hz, 2H), 2.81 (t, J = 6.0 Hz, 2H), 2.76 - 2.53 (m, 2H). ¹³C NMR (151 MHz, DMSO-d6) δ 192.32, 172.92, 167.36, 142.52, 140.05, 140.00, 135.47, 135.19, 133.76, 133.24, 132.73, 131.10, 129.52, 129.38, 129.16, 128.56, 127.38, 126.27, 125.83, 124.54, 123.80, 123.77, 47.40, 44.35, 43.63, 38.02, 27.69. LC-MS (ESI): m/z = 581.1 [M+1]⁺, t_{R} = 4.16 min. Purity > 95% (UV).

**Methyl 3-amino-3-(2-(phenylsulfonyl)-1,2,3,4-tetrahydroisoquinolin-7-yl)propanoate (K46a).** To the solution of **K45b** (121 mg, 0.34 mmol, 1.0 equiv.) in 0.88 mL of dioxane was added hydroxylamine hydrochloride (70 mg, 1.01 mmol, 3. 0 equiv.) and anhydrous sodium acetate (80.83 mg, 1.01 mmol, 3.0 equiv.) followed by adding tetrabutylammonium hydrogen sulfate (catalyst amount, 0.002 equiv.). The reaction mixture was stirred at 99 °C for overnight. The reaction mixture was extracted with ethyl acetate (3 × 5 mL). Organic layer was separated and washed with water, brine, dried over MgSO₄ and concentrated. Dried to give oil. Intermediate was obtained (114 mg, 0.30 mmol). LC-MS (ESI): m/z = 391.2 [M+1]⁺, t_{R} = 4.00 and 4.16 min.The reaction was conducted following the general procedure **G,** using intermediate (114 mg, 0.30 mmol), 88% formic acid in methanol (0.57 mL formic acid, 0.30 mL water, 0.60 mL methanol) and zinc dust (60 mg, 0.9 mmol). Crude intermediate was obtained (37 mg, 0.09 mmol, 29%) LC-MS (ESI): m/z = 375.2 [M+1]⁺, t_{R} = 3.15 min.

**3-(9-oxo-9H-fluorene-4-carboxamido)-3-(2-(phenylsulfonyl)-1,2,3,4-tetrahydroisoquinolin-7-yl)propanoic acid (K46).** The reaction was conducted following the general procedure **H,** using EDCI (28 mg, 0.15 mmol) and HOBt (20 mg, 0.15 mmol), **K46a** (36 mg, 0.1 mmol), 9-oxo-9H-fluorene-4-carboxylic acid (59 mg, 0.15 mmol) and DIPEA (38 µL, 28 mg, 0.22 mmol). The crude intermediatewas obtained (17 mg, assumed 0.03 mmol). LC-MS (ESI): m/z = 581.1 [M+1]⁺, t_{R} = 4.50 min. The subsequent reaction was conducted following the general procedure **L,** using the intermediate (17mg, assumed as 0.03 mmol), ethanol (0.1 mL), water (0.05 mL). Yellow solid (5 mg, 0.009 mmol, 3%, over four steps). ¹H NMR (600 MHz, DMSO-d6) δ 12.35 (s, 1H), 9.21 (d, J = 8.2 Hz, 1H), 7.86 - 7.81 (m, 2H), 7.76 - 7.67 (m, 2H), 7.67 - 7.60 (m, 3H), 7.49 (dd, J = 7.7, 1.4 Hz, 1H), 7.45 (t, J = 7.4 Hz, 1H), 7.42 - 7.36 (m, 1H), 7.33 (tt, J = 7.4, 5.9 Hz, 2H), 7.26 - 7.20 (m, 2H), 7.13 (d, J = 7.9 Hz, 1H), 5.43 (td, J = 8.6, 6.1 Hz, 1H), 4.18 (q, J = 15.4 Hz, 2H), 3.32 (t, J = 6.0 Hz, 2H), 2.87 (t, J = 6.0 Hz, 2H), 2.85 - 2.72 (m, 2H). ¹³C NMR (151 MHz, DMSO-d6) δ 192.35, 171.64, 166.54, 142.47, 140.01, 135.71, 135.10, 133.76, 133.65, 133.26, 133.22, 132.73, 132.00, 131.47, 129.59, 129.41, 129.30, 128.82, 127.39, 125.12, 124.70, 124.57, 123.83, 123.79, 49.67, 47.41, 43.60, 40.26, 27.76. LC-MS (ESI): m/z = 567.1 [M+1]⁺, t_{R} = 4.18 min. Purity > 95% (UV).

**2-(benzylsulfonyl)-7-bromo-1,2,3,4-tetrahydroisoquinoline (K47a).** The reaction was conducted following the general procedure A, using 7-bromo-1,2,3,4-tetrahydroisoquinoline (125 mg, 0.59 mmol), Et₃N (0.245 mL, 1.77 mmol), DCM (1.25 mL), phenylmethanesulfonyl chloride (0.3 mL, 2.36 mmol, 1.00 equiv.) and following by 1 M HCl (2 × 20 mL). White solid (158 mg, 0.43 mmol, 73%). ¹H NMR (400 MHz, DMSO-d6) δ 7.37 (tt, J = 6.7, 3.9 Hz, 7H), 7.12 (d, J = 8.7 Hz, 1H), 4.49 (s, 2H), 4.37 (s, 2H), 3.38 (t, J = 5.9 Hz, 2H), 2.75 (t, J = 5.9 Hz, 2H). LC-MS (ESI): m/z = 366.0 [M+1]⁺, t_{R} = 4.79 min.

**Methyl (E)-3-(2-(benzylsulfonyl)-1,2,3,4-tetrahydroisoquinolin-7-yl)acrylate (K47b).** To a solution of **K47a** (158 mg, 0.43 mmol, 1.00 equiv.) in DMF (0.2 mL) was added methyl acrylate (150 mg, 1.72 mmol, 4.00 equiv.), tri-o-tolyphosphine (39 mg, 0.13 mmol, 0.30 equiv.) and DIPEA (222.3 mg, 1.72 mol, 4.00 equiv.). The mixture was degassed with nitrogen for 20 min, followed by the addition of Pd(OAc)₂ (4.86 mg, 0.02 mmol, 0.05 equiv.) in sealed tube. The reaction mixture was stirred at 100 °C for 3 h. The reaction mixture was then cooled to 0°C, quenched with cold water, extracted with EtOAc. The organic layer was washed with brine, dried under MgSO₄ and filtered. The filtrate was evaporated and was purified by silica column chromatography (heptane and ethyl acetate) to afford the title compound **K47b** (133mg, 0.372 mmol, 16%).¹H NMR (400 MHz, Chloroform-d) δ 7.53 (d, J = 16.0 Hz, 1H), 7.32 - 7.16 (m, 6H), 7.07 - 6.98 (m, 2H), 6.29 (d, J = 16.0 Hz, 1H), 4.21 (d, J = 5.4 Hz, 5H), 3.73 (d, J = 5.6 Hz, 3H), 3.31 (q, J = 5.8 Hz, 2H), 2.76 - 2.65 (m, 2H). LC-MS (ESI): m/z = 372.1 [M+1]⁺, t_{R} = 4.56 min.

**Methyl 4-amino-3-(2-(benzylsulfonyl)-1,2,3,4-tetrahydroisoquinolin-7-yl)butanoate (K47c).** The solution of **K47b** (32 mg, 0.086 mmol; 1.00 equiv.), nitromethane (60 mg, 52 µL, 0.95 mmol, 11.00 equiv.) and tetrabutylammonium fluoride (0.118 mL, 0.176 mmol, 1.30 equiv.) in 1 mL of toluene was mixed and stirred at 50°C overnight. Upon reaction completion, the mixture was poured into 10 mLof 1 M HCl. Separate the phases. And the aqueous layer was extracted with toluene (2 × 15 mL). The organic phase was combined, then dried with Na₂SO₄, filtered by celite and concentrated *in vacuo* to afford crude intermediate. The intermediate was treated as starting material in the next step without further purification. LC-MS (ESI): no ionization, t_{R} = 4.53 min. The reaction was conducted by using the intermediate product (26 mg, assumed 0.06 mmol, 1.0 equiv.), 0.78 mL methanol, 0.6 mL of 1M HCl and zinc dust (12 mg, 0.18 mmol, 3.0 equiv.). The zinc dust was added portion wise during 1 h. The reaction was stirred overnight. Upon reaction completion, the mixture was filtered through celite and washed several times with methanol. The filtrate was concentrated, and the compound **K47c** (5 mg) was used as starting material in the next steps without further purification. LC-MS (ESI): m/z = 403.1 [M+1]⁺, t_{R} = 3.21 min.

**3-(2-(benzylsulfonyl)-1,2,3,4-tetrahydroisoquinolin-7-yl)-4-(9-oxo-9H-fluorene-4-carboxamido)butanoic acid (K47).** The reaction was conducted following the general procedure **H,** using EDCI (3.4 mg, 0.02 mmol), HOBt (2.4 mg, 0.020 mmol), **K47c** (5 mg, assumed as 0.012 mmol), 9-oxo-9H-fluorene-4-carboxylic acid (3 mg, 0.012 mmol) and DIPEA (5 µL, 3.4 mg, 0.02 mmol), the desired compound was obtained and detected on LC-MS (ESI): m/z = 608.1 [M+1]⁺, t_{R} = 4.53 min could be found. The subsequent reaction was conducted in the initial flask according to general procedure **L,** using ethanol (14 µL), water (7 µL) and 6 M NaOH (5 µL, 0.03 mmol). White solid (1.1 mg, 0.002 mmol, 2%, over four steps). ¹H NMR (600 MHz, DMSO-d6) δ 12.08 (s, 1H), 8.75 (t, J = 5.7 Hz, 1H), 7.67 (dd, J = 5.8, 2.7 Hz, 1H), 7.63 (d, J = 7.3 Hz, 1H), 7.53 - 7.46 (m, 2H), 7.44 - 7.32 (m, 8H), 7.15 (dd, J = 7.9, 1.8 Hz, 1H), 7.10 (d, J = 7.9 Hz, 1H), 7.06 (d, J = 1.8 Hz, 1H), 4.46 (s, 2H), 4.34 - 4.22 (m, 2H), 3.65 - 3.45 (m, 2H), 3.30 (dd, J = 12.5, 6.1 Hz, 2H), 2.76 (dd, J = 5.8, 2.4 Hz, 2H), 2.77 - 2.54 (m, 2H). ¹³C NMR (151 MHz, DMSO-d6) δ 192.38, 172.97, 167.43, 142.59, 140.10, 140.02, 135.27, 133.78, 133.30, 132.76, 132.01, 131.64, 130.85, 129.64, 129.58, 129.44, 129.21, 128.76, 128.33, 128.17, 126.18, 125.53, 124.56, 123.85, 123.81, 54.89, 46.84, 44.40, 43.25, 40.84, 38.06, 28.17. LC-MS (ESI): m/z = 595.1 [M+1]⁺, t_{R} = 4.17 min. Purity > 95% (UV).

**Methyl 3-amino-3-(2-(benzylsulfonyl)-1,2,3,4-tetrahydroisoquinolin-7-yl)propanoate (K48a).** To the solution of **K47b** (97 mg, 0.27 mmol, 1.0 equiv.) in 0.18 mL of dioxane was added hydroxylamine hydrochloride (54 mg, 0.78 mmol, 3.0 equiv.) and anhydrous sodium acetate (62 mg, 0.78 mmol, 3.0 equiv.) followed by adding tetrabutylammonium hydrogen sulfate (catalyst amount, 0.002 equiv.). The reaction mixture was stirred at room temperature for 48 hours. The reaction mixture was extracted with ethyl acetate (3 × 5 mL). Organic layer was separated and washed with water, brine, dried over MgSO₄ and concentrated to give dryness. The intermediate was obtained as oil and used for next reaction without further purification. LC-MS (ESI): m/z = 403.1 [M+1]⁺, t_{R} = 4.02 min and 4.16 min. The reaction was conducted following the general procedure G, using the intermediate (119 mg, assumed as 0.30 mmol), 88% formic acid in methanol (0.57 mL formic acid, 0.30 mL water, 0.60 mL methanol) and zinc dust (60 mg, 0.9 mmol). Compound **K48a** (6 mg) was used as a starting material in the next step without further purification. LC-MS (ESI): m/z = 389.2 [M+1]⁺, t_{R} = 3.05 min.

**3-(2-(benzylsulfonyl)-1,2,3,4-tetrahydroisoquinolin-7-yl)-3-(9-oxo-9H-fluorene-4-carboxamido)propanoic acid (K48).** The reaction was conducted following the general procedure **H,** using EDCI (4.3 mg, 0.02 mmol), HOBt (3 mg, 0.02 mmol.), **K48a** (6 mg, 0.015 mmol), 9-oxo-9H-fluorene-4-carboxylic acid (5 mg, 0.022 mmol) and DIPEA (6 µL, 4.4 mg, 0.03 mmol). The crude intermediate was obtained (10 mg) and treated as a starting material in next step without further purification. LC-MS (ESI): m/z = 595.1 [M+1]⁺, t_{R} = 4.57 min. The reaction was conducted following the general procedure **L,** using the intermediate (10mg, assumed as 0.017 mmol), methanol (0.1 mL), water (0.05 mL) and 6 M NaOH (3 µL, 0.034 mmol). Yellow solid (1 mg, 0.002 mmol, 0.6%, over four steps). ¹H NMR (600 MHz, DMSO-*d*₆) δ 12.36 (s, 1H), 9.25 (d, *J=* 8.2 Hz, 1H), 7.70 (dd, *J* = 7.1, 1.4 Hz, 1H), 7.66 - 7.61 (m, 1H), 7.52 - 7.40 (m, 3H), 7.39 (dtd, *J* = 14.2, 5.0, 2.7 Hz, 4H), 7.38 - 7.33 (m, 3H), 7.27 (dd, *J* = 7.8, 1.8 Hz, 1H), 7.17 (d, *J =* 8.3 Hz, 2H), 5.46 (td, *J* = 8.7, 6.1 Hz, 1H), 4.51 (s, 2H), 4.38 - 4.30 (m, 2H), 3.42 - 3.37 (m, 1H), 2.88 -2.80 (m, 3H), 2.77 (dd, *J=* 16.0, 6.0 Hz, 1H).¹³C NMR (151 MHz, DMSO-d6) δ 192.39, 171.69, 166.58, 157.56, 142.51, 140.07, 135.20, 133.80, 133.67, 133.29, 132.75, 132.46, 132.28, 130.88, 129.65, 129.49, 129.32, 129.01, 128.35, 128.19, 124.61, 124.36, 123.90, 123.83, 54.93, 49.72, 49.70, 46.87, 43.24, 40.41, 28.30. LC-MS (ESI): m/z = 581.1 [M+1]⁺, t_{R} = 4.18 min. Purity > 95% (UV).

**2-(2-(benzylsulfonyl)-1,2,3,4-tetrahydroisoquinolin-7-yl)-2-(9-oxo-9H-fluorene-4-carboxamido)acetic acid (K49).** The reaction was conducted following the general procedure **H,** using from **K1j** (29 mg, 0.065 mmol), Et₃N (20 mg, 30 µL, 0.195 mmol), phenylmethanesulfonyl chloride (12 mg, 0.065 mmol), 2 mL of dry DCM. The crude intermediate was obtained. The subsequent reaction was conducted according to general procedure **L,** using intermediate (assumed as 0.65 mmol), methanol (74 µL), water (37 µL) then and 6M NaOH (21 µL, 0.130 mmol). White solid (7.9 mg, 0.014 mmol, 21.4 %). ¹H NMR (600 MHz, DMSO-d6) δ 13.04 (s, 1H), 9.45 (d, J = 7.2 Hz, 1H), 7.75 - 7.68 (m, 2H), 7.65 (d, J = 7.3 Hz, 1H), 7.57 (dd, J = 7.7, 1.2 Hz, 1H), 7.50 - 7.41 (m, 2H), 7.43 - 7.36 (m, 3H), 7.38 - 7.31 (m, 4H), 7.25 (d, J = 1.9 Hz, 1H), 7.19 (d, J = 7.9 Hz, 1H), 5.58 (d, J = 7.1 Hz, 1H), 4.51 (s, 2H), 4.39 - 4.29 (m, 2H), 3.43 - 3.33 (m, 2H), 2.81 (t, J = 6.0 Hz, 2H). ¹³C NMR (151 MHz, DMSO-d6) δ 192.43, 171.64, 167.44, 142.67, 140.39, 135.12, 134.20, 134.18, 133.73, 133.51, 133.33, 132.45, 132.09, 130.86 (2C), 129.67, 129.46, 129.14, 129.06, 128.34 (2C), 128.19, 126.45, 125.86, 124.73, 124.14, 123.79, 56.71, 54.98, 46.74, 43.13, 28.29. LC-MS (ESI): m/z = 567.2 [M+1]⁺, t_{R} = 4.21 min. Purity > 95% (UV).

**2,2,2-trifluoro-1-(isoindolin-2-yl)ethan-1-one (K50a).** The reaction was conducted following the general procedure **A.** Starting from isoindoline (2.48 g, 20.8 mmol), trifluoroacetic anhydride (5.25 g, 3.47 mL, 25 mmol) and Et₃N (5.80 mL, 41.6 mmol). Brown solid (7g, quantitative) *in vacuo.* LC-MS (ESI): m/z = 216.1 [M+1]⁺, 435.2 [2M+1]⁺, t_{R}= 4.26 min.

**1-(5-acetylisoindolin-2-yl)-2,2,2-trifluoroethan-1-one (K50b).** The reaction was conducted following the general procedure **B,** using **K50a** (20 mmol), aluminum chloride (16 g, 120 mmol) and acetyl chloride (4.7 g , 60 mmol). White solid (2g, 7.7 mmol, 39%). ¹H NMR (400 MHz, Chloroform-d) δ 7.91 - 7.80 (m, 2H), 7.34 (dd, J = 21.4, 8.0 Hz, 1H), 5.02 (s, 2H), 4.90 (s, 2H), 2.56 (d, J = 2.7 Hz, 3H). LC-MS (ESI): m/z = 258.1 [M+1]⁺ , t_{R}= 3.93 min.

**2-oxo-2-(2-(2,2,2-trifluoroacetyl)isoindolin-5-yl)acetic acid (K50c).** The reaction was conducted following the general procedure **D,** using **K50b** (1.145 g, 4.45 mmol), selenium dioxide (594 mg, 5.35 mmol), 18 mL of pyridine. Orange solid (570 mg, 1.98 mmol, 45%). ¹H NMR (600 MHz, DMSO-d6) δ 14.76 (s, 1H), 8.01 - 7.97 (m, 1H), 7.92 (dt, J = 8.0, 1.7 Hz, 1H), 7.64 (dd, J = 13.7, 8.0 Hz, 1H), 5.14 (d, J = 9.4 Hz, 2H), 4.93 (d, J = 9.4 Hz, 2H). ¹H NMR (600 MHz, Chloroform-d) δ 8.33 (S, 1H), 7.45 (d, J = 8.0 Hz, 1H), 7.40 (d, J = 8.0 Hz, 1H), 5.07 - 5.04 (m, 4H). LC-MS (ESI): m/z = 288.0 [M+1]⁺, t_{R} = 2.81 min.

**Ethyl 2-oxo-2-(2-(2,2,2-trifluoroacetyl)isoindolin-5-yl)acetate (K50d).** The reaction was conducted following the general procedure **E,** using **K50c** (570 mg, 1.98 mmol), TMS-Cl (430mg, 0.5 mL, 4.00 mmol.) and 2 mL ethanol. Orange oil (280mg, 0.85 mmol). LC-MS (ESI): m/z = 316.1 [M+1]⁺, t_{R} = 4.35 min.

**Ethyl 2-(hydroxyimino)-2-(2-(2,2,2-trifluoroacetyl)isoindolin-5-yl)acetate (K50e).** The reaction was conducted following the general procedure **F,** using **K50d** (about 1.98 mmol), anhydrous sodium acetate (412 mg, 2.38 mmol), hydroxylamine hydrochloride (220 mg, 3.17 mmol) and methanol (15 mL). Colorless oil (350 mg, 1.06 mmol, 53% over two steps). LC-MS (ESI): m/z = 331.1 [M+1]⁺, t_{R} = 3.85 and 4.04 min.

**Ethyl 2-amino-2-(2-(2,2,2-trifluoroacetyl)isoindolin-5-yl)acetate (K50f).** The reaction was conducted following the general procedure **G,** using **K50e** (330 mg, 1.00 mmol, 1.00 equiv), 88% formic acid in methanol (5.7 mL formic acid, 3 mL water, 6 mL methanol) and zinc dust (196 mg, 3 mmol). Brown oil (280 mg) and used as starting material in the next steps without further purification. LC-MS (ESI): m/z = 317.2 [M+1]⁺, t_{R} = 2.74 min.

**Ethyl 2-(9-oxo-9H-fluorene-4-carboxamido)-2-(2-(2,2,2-trifluoroacetyl)isoindolin-5-yl)acetate (K50g).** The reaction was conducted following the general procedure **H,** using EDCI (253 mg, 1.32 mmol, 1.5 equiv), HOBt (178 mg, 1.32 mmol, 1.5 equiv.), **K50f** (280 mg, 0.885 mmol, 1.0 equiv.), 9-oxo-9H-fluorene-4-carboxylic acid (200 mg, 0.885 mmol, 1.0 equiv.) and DIPEA (0.34 mL, 251 mg, 1.95 mmol, 2.2 equiv). Colorless oil (1.07g, 1.99 mmol, 67.7%). LC-MS (ESI): m/z = 523.2 [M+1]⁺, t_{R} = 4.60 min.

**2-(isoindolin-5-yl)-2-(9-oxo-9H-fluorene-4-carboxamido)acetic acid (K50h).** The reaction was conducted following the general procedure **I,** using **K50g** (crude product from last step), 3 mL ethanol and 6 M NaOH 0.92 mL. Brown solid (132 mg, 0.33 mmol, 37 % over 3 steps). ¹H NMR (400 MHz, DMSO-d6) δ 13.20 (s, 1H), 9.57 (d, J = 7.3 Hz, 1H), 9.49 (br s, 1H), 7.71 (dd, J = 9.7, 7.3 Hz, 2H), 7.66 (d, J = 7.2 Hz, 1H), 7.62 - 7.36 (m, 7H), 5.71 (d, J = 7.3 Hz, 1H), 4.63 - 4.45 (m, 4H). LC-MS (ESI): m/z = 399.1 [M+1]⁺, t_{R} = 2.74 min.

**2-(2-((3-cyano-4-ethoxyphenyl)sulfonyl)isoindolin-5-yl)-2-(9-oxo-9H-fluorene-4-carboxamido)acetic acid (K50).** The reaction was conducted following the general procedure **K,** using **K50h** (23 mg, 0.058 mmol), 3-cyano-4-fluorobenzenesulfonyl chloride (13 mg, 0.058 mmol). The solvent was evaporated and subsequent reaction was conducted following general procedure **L,** using the intermediate, 100 µL of ethanol, 50 µL of water, and 116 µL of 1M NaOH. Yellow solid (1.0 mg, 0.002 mmol, 3 %) ¹H NMR (600 MHz, DMSO-*d*₆) δ 13.08 (s, 1H), 9.47 (d, J = 7.2 Hz, 1H), 8.23 (d, J = 2.4 Hz, 1H), 8.12 (dd, J = 9.0, 2.4 Hz, 1H), 7.70 (d, J = 7.3 Hz, 1H), 7.64 (t, J = 6.4 Hz, 2H), 7.54 (d, J = 7.7 Hz, 1H), 7.45 - 7.35 (m, 6H), 7.28 (d, J = 7.9 Hz, 1H), 5.61 (d, J = 7.1 Hz, 1H), 4.68 - 4.54 (m, 4H), 4.26 (q, J = 7.0 Hz, 2H), 1.36 (t, J = 7.0 Hz, 3H).¹³C NMR (151 MHz, DMSO-d6) δ 192.40, 171.47, 167.37, 163.19, 142.63, 140.35, 136.15, 136.11, 135.80, 135.05, 134.34, 134.15, 133.72, 133.46, 133.32, 132.04, 129.63, 129.14, 128.14, 127.72, 124.75, 124.08, 123.78, 122.94, 122.55, 114.99, 113.81, 101.60, 65.58, 56.73, 53.39, 53.30, 14.13. LC-MS (ESI): m/z = 608.1 [M+1]⁺, t_{R} = 4.27 min. Purity > 95% (UV).

**2-(2-((4-methoxyphenyl)sulfonyl)isoindolin-5-yl)-2-(9-oxo-9H-fluorene-4-carboxamido)acetic acid (K51).** The reaction was conducted following the general procedure **K,** using **K50h** (38.6 mg, 0.097 mmol), 4-methoxybenzenesulfonyl chloride (20 mg, 0.097 mmol). Yellow solid. (8.0 mg, 0.014 mmol, 15%) ¹H NMR (600 MHz, DMSO-*d*₆) δ 13.03 (s, 1H), 9.45 - 9.41 (m, 1H), 7.82 (d, *J* = 8.4 Hz, 2H), 7.69 (d, *J* = 7.3 Hz, 1H), 7.63 (q, *J* = 4.5 Hz, 2H), 7.54 (d, *J* = 7.7 Hz, 1H), 7.46 - 7.33 (m, 5H), 7.28 (d, *J =* 8.0 Hz, 1H), 7.12 (d, *J* = 8.4 Hz, 2H), 5.58 (d, *J* = 6.8 Hz, 1H), 4.62 - 4.48 (m, 4H), 3.81 (s, 3H). ¹³C NMR (151 MHz, DMSO-d6) δ 191.97, 165.84, 163.07, 151.89, 141.99, 141.18, 135.73, 135.65, 135.15, 134.35, 133.83, 133.62, 133.39, 132.29, 130.68, 129.28, 128.10, 127.42, 127.18, 126.46, 124.04, 123.99, 123.78, 123.66, 115.00, 113.62, 101.47, 65.59, 58.03 (2C), 47.39. LC-MS (ESI): m/z = 569.1 [M+1]⁺, t_{R} = 4.12 min. Purity > 95% (UV).

**3-(o-tolyl)picolinic acid (K52a).** Pd(OAc)₂ (23 mg, 5.0 mol %), dppf (67 mg, 6.0 mol %), methyl 3-bromopicolinate (432 mg, 2.00 mmol, 1.00 equiv.), o-tolylboronic acid (543 mg, 4.00 mmol, 2.00 equiv.), and K₃PO₄ (3.0 equiv.) was added in an oven-dried resealable Schlenk tube. The vessel changed with nitrogen in three cycles. 5 mL of DME was then injected into the Schlenk TUBE. The mixture was stirred vigorously at 80 °C for 12 h. The reaction mixture was then cooled to room temperature, and concentrated under reduced pressure to give a residue which was purified by reverse phase on C18 column. The title compound was obtained as white solid (269 mg, 1.3 mmol, 63%).¹H NMR (600 MHz, DMSO-d6) δ 8.33 (dd, J = 4.8, 1.7 Hz, 1H), 7.38 (dd, J = 7.6, 1.7 Hz, 1H), 7.20 - 7.08 (m, 5H), 2.11 (s, 3H). LC-MS (ESI): m/z = 214.1 [M+1]⁺, t_{R} = 2.94 min.

**5-methyl-9H-indeno[2,1-b]pyridin-9-one (K52b). K52a** (216 mg, 1.01 mmol) was dissolved in thionyl chloride (4 mL). The resulting mixture was stirred at RT for 6 hours. The excess thionyl chloride was removed via rotavapor DCM (10 mL) was added to redissolve the remaining purple solid. Aluminium trichloride (245 mg, 3.03 mmol, 3.00 equiv.) was added carefully, observe a deep purple colouration. The reaction mixture was stirred at RT overnight. 5 mL of water was added into the mixture. The mixture was filtered with celite. The resultant was extracted with a further portion of DCM (3 × 10 mL). The organic extracts were combined, dried over MgSO₄. The excess solvent was removed under reduced pressure to obtain title compound K52b as brown oil (133 mg, 0.68 mmol, 67%). The crude product was treated as a starting materials in next step without further purification. LC-MS (ESI): m/z = 196.1 [M+1]⁺, t_{R} = 3.84 min.

**9-oxo-9H-indeno[2,1-b]pyridine-5-carboxylic acid (K52c).** A suspension of **K52b** (130 mg, 0.66 mmol, 1.00 equiv.) and potassium permanganate (526 mg, 3.32 mmol, 3.00 equiv.) in a 1:1 mixture of pyridine (3 mL) and water (3 mL) was heated to reflux overnight. The warm solution was filtered through celite. The filtrate was then concentrated under reduced pressure. The resulting solid was re-dissolved by using MeCN: H₂O (1:1) and purified by reverse-phase system on C18 column. 9 mg (0.040 mmol, 4% over two steps.) yellow solid was obtained. ¹H NMR (600 MHz, DMSO-*d*₆) δ 8.93 (dd, *J* = 7.9, 1.5 Hz, 1H), 8.53 (dd, *J* = 4.8, 1.5 Hz, 1H), 7.82 (dd, *J* = 7.8, 1.3 Hz, 1H), 7.59 (dd, *J* = 7.3, 1.2 Hz, 1H), 7.46 (dd, *J* = 7.8, 4.8 Hz, 1H), 7.37 (t, *J=* 7.6 Hz, 1H). LC-MS (ESI): m/z = 226.0 [M+1]⁺, t_{R} = 3.07 min.

**2-(9-oxo-9H-indeno[2,1-b]pyridine-5-carboxamido)-2-(2-(2,2,2-trifluoroacetyl)-1,2,3,4-tetrahydroisoquinolin-7-yl)acetic acid (K52d).** The reaction was conducted following the general procedure **M,** using EDCI (11 mg, 0.057 mmol) and HOBt (7.7 mg, 0.057 mmol), **K1g** (12 mg, 0.038 mmol), **K52c** (8.7 mg, 0.038 mmol) and DIPEA (14 µL, 10 mg, 0.0836 mmol). Subsequently 6 M NaOH (8 µL, 0.076 mmol) was added. The title compound **K52d** was obtained (9 mg, 0.022 mmol, 57%). LC-MS (ESI): m/z = 414.2 [M+1]⁺, t_{R} = 2.22 min.

**Ethyl 2-(9-oxo-9H-indeno[2,1-b]pyridine-5-carboxamido)-2-(1,2,3,4-tetrahydroisoquinolin-7-yl)acetate (K52e).** The reaction was conducted following the general **E,** using **K52d** (9 mg, 0.022 mmol), TMS-Cl (8 µL, 0.044 mmol), ethanol (500 µL).Yellow solid (10 mg, 0.038 mmol). LC-MS (ESI): m/z = 442.2 [M+1]⁺, t_{R} = 2.85 min.

**2-(2-((3-cyano-4-ethoxyphenyl)sulfonyl)-1,2,3,4-tetrahydroisoquinolin-7-yl)-2-(9-oxo-9H-indeno[2,1-b]pyridine-5-carboxamido)acetic acid (K52).** The first reaction was conducted following the general procedure **J,** using **K52e** (10 mg, 0.038 mmol, 1.00 equiv.), Et₃N (11 mg, 16 µL, 0.114 mmol), 3-cyano-4-fluorobenzenesulfonyl chloride (8 mg, 0.038 mmol). The subsequent reaction was conducted according to general procedure **L,** using crude intermediate, ethanol (464 µL), water (232 µL), 6 M NaOH (13 µL, 0.076 mmol). Yellow solid (2 mg, 0.003 mmol, 14%). ¹H NMR (600 MHz, DMSO-d6) δ 13.05 (s, 1H), δ 9.42 (d, J = 6.7 Hz, 1H), 8.58 (d, J = 4.9 Hz, 1H), 8.20 (d, J = 2.3 Hz, 1H), 8.12 (d, J = 7.9 Hz, 1H), 8.06 (dd, J = 9.0, 2.4 Hz, 1H), 7.76 (d, J = 7.3 Hz, 1H), 7.67 (d, J = 7.7 Hz, 1H), 7.51 (t, J = 7.5 Hz, 1H), 7.48 - 7.37 (m, 2H), 7.30 (d, J = 7.2 Hz, 2H), 7.16 (d, J = 7.9 Hz, 1H), 5.52 (d, J = 6.9 Hz, 1H), 4.29 (q, J = 6.9 Hz, 2H), 4.21 (s, 2H), 3.32 - 3.27 (m, 2H), 2.87 (t, J = 6.1 Hz, 2H), 1.38 (t, J = 7.0 Hz, 3H). ¹³C NMR (151 MHz, DMSO-d6) δ 191.52, 171.64, 167.02, 163.16, 152.20, 150.32, 138.99, 138.19, 134.88, 134.38 (2C), 133.41, 133.04, 132.41, 132.02, 131.74, 131.63, 129.90, 128.90, 128.03, 127.45, 126.57, 126.15 (2C), 125.15, 115.03, 113.71, 101.52, 65.64, 56.98, 47.35, 43.45, 27.74, 14.15. LC-MS (ESI): m/z = 623.2 [M+1]⁺, t_{R} = 3.98 min. Purity > 95% (UV).

**Methyl 2-(2-((3-cyano-4-ethoxyphenyl)sulfonyl)-1,2,3,4-tetrahydroisoquinolin-7-yl)-2-(9-oxo-9H-fluorene-4-carboxamido)acetate (K53).** The reaction was conducted following the general procedure **E,** using **K26** (around 5mg, 0.009 mmol), methanol (45 µL), TMS-Cl (3 µL, 0.018 mmol).The crude product is purified by revered phase on C18 column with standard solution A (95% water, 5% ACN and 0.1% TFA) and solution B (95% ACN, 5% water and 0.1% TFA). Yellow solid (1.5 mg, 0.002 mmol, 17%). ¹H NMR (600 MHz, DMSO-*d*₆) δ 9.58 (dd, *J* = 20.3, 6.9 Hz, 1H), 8.19 (d, *J =* 2.5 Hz, 1H), 8.06 (dd, *J* = 9.0, 2.4 Hz, 1H), 7.71 (d, *J* = 7.4 Hz, 1H), 7.68 (d, *J =* 7.6 Hz, 1H), 7.65 (d, *J =* 7.3 Hz, 1H), 7.56 (d, *J* = 7.7 Hz, 1H), 7.49 (t, *J* = 7.4 Hz, 1H), 7.44 (q, *J* = 8.6, 8.0 Hz, 2H), 7.39 (t, *J =* 7.5 Hz, 1H), 7.30 (d, *J =* 9.7 Hz, 2H), 7.19 (dd, J = 19.3, 7.9 Hz, 1H), 5.66 (d, *J* = 6.6 Hz, 1H), 4.29 (q, *J* = 7.0 Hz, 2H), 4.26 - 4.16 (m, 2H), 3.70 (d, *J = 4.4* Hz, 3H), 2.88 (t, *J =* 6.1 Hz, 2H), 1.38 (t, *J =* 7.0 Hz, 3H). ¹³C NMR (151 MHz, DMSO-d6) δ 192.37, 170.72, 167.58, 163.15, 142.61, 140.36, 135.21, 134.38, 134.19, 133.75, 133.43, 133.41, 133.37, 133.33, 131.89, 131.83, 129.70, 129.19, 129.04, 128.00, 126.65, 126.19, 124.84, 124.01, 123.84, 115.00, 113.70, 101.53, 65.63, 56.66, 52.44, 47.30, 43.42, 27.74, 14.14. LC-MS (ESI): m/z = 636.2 [M + H]⁺, t_{R} = 4.65 min. Purity> 95% (UV).

**Methyl 2-(9-oxo-9H-fluorene-4-carboxamido)-2-(1,2,3,4-tetrahydroisoquinolin-7-yl)acetate (K54i).** The reaction was conducted following the general procedure **E,** using **K1i** (around 12 mg, 0.029 mmol), methanol (150 µL), TMS-Cl (70 µL, 0.06 mmol). Yellow solid (12 mg, quantitative). LC-MS (ESI): m/z = 427.2 [M + H]⁺, t_{R} = 3.57 min.

**Methyl 2-(2-((3-cyano-4-fluorophenyl)sulfonyl)-1,2,3,4-tetrahydroisoquinolin-7-yl)-2-(9-oxo-9H-fluorene-4-carboxamido)acetate (K54).** The reaction was conducted following the general procedure **J,** using **K54i** (assumed as 0.029 mmol), 3-cyano-4-methylbenzenesulfonyl chloride (7 mg, 0.029 mmol), Et₃N (9 mg, 12 µL, 0.087 mmol.). The crude product is purified by revered phase on C18 column with standard solution A (95% water, 5% ACN and 0.1% TFA) and solution B (95% ACN, 5% water and 0.1% TFA). Yellow solid (11 mg, 0.018 mmol, 62 %). ¹H NMR (600 MHz, DMSO-d6) δ 9.57 (d, J = 6.9 Hz, 1H), 8.46 (d, J = 5.9 Hz, 1H), 8.21 (d, J = 6.4 Hz, 1H), 7.76 (t, J = 8.9 Hz, 1H), 7.70 (t, J = 9.3 Hz, 2H), 7.65 (d, J = 7.3 Hz, 1H), 7.57 (d, J = 7.7 Hz, 1H), 7.50 (t, J = 7.6 Hz, 1H), 7.45 (t, J = 7.5 Hz, 1H), 7.40 (t, J = 7.4 Hz, 1H), 7.33 - 7.27 (m, 2H), 7.17 (d, J = 7.8 Hz, 1H), 5.67 (d, J = 6.7 Hz, 1H), 4.27 (s, 2H), 3.71 (s, 3H), 3.38 (dq, J = 29.1, 6.4 Hz, 2H), 2.88 (d, J = 6.4 Hz, 2H). ¹³C NMR (151 MHz, DMSO-d6) δ 192.37, 170.73, 167.59, 164.77 (d, J = 262.7 Hz), 142.61, 140.38, 135.30, 135.22, 134.19, 133.76 (2C), 133.50 (d, J = 2.9 Hz), 133.48, 133.47, 133.33, 131.82, 131.77, 129.71, 129.18, 129.07, 126.71, 126.15, 124.85, 124.02, 123.84, 117.98 (d, J = 21.0 Hz), 112.78, 101.94 (d, J = 16.5 Hz), 56.66, 52.44, 47.22, 43.39, 27.66. LC-MS (ESI): m/z = 610.2 [M+1]⁺, t_{R} = 5.13 min. Purity > 95% (UV).

### Conclusion

The compounds according to the present disclosure were prepared and characterized.

### Example 2: Expression and purification of human and mouse Keap1 Kelch domains

The recombinant His-tagged human Keap1 Kelch domain (residue 321-609, UniProt Q14145) and the recombinant His-tagged mouse Keap1 Kelch domain (residue 322-624, UniProt Q9Z2X8; 97% identical to the human sequence) were cloned into a pRSET A vector and expressed in Escherichia coli BL21 (DE3) pLysS. Keap1 was grown in a preculture of 50 mL LB media supplemented with 1% glucose and 100 µg/mL ampicillin overnight (ON) at 37 °C to an OD600 of ~1.0. The preculture was transferred to 1 L of LB medium supplemented with 1% glucose and 100 µg/mL ampicillin and grown at 37 °C/180 rpm to an OD600 of ~0.5, before induction with isopropyl β-d-1 thiogalactopyranoside (final concentration, 0.5-1 mM) ON at 15 °C/180 rpm. Cells were harvested by centrifugation at 4000 g for 30 min. The cells were resuspended in lysis buffer (50mM HEPES pH 7.5, cOmplete Protease Inhibitor Cocktail (1 tablet/50 mL of buffer), 25 µg/mL DNase, 40 mM Mg₂SO₄, 150 mM NaCl, 5 mM imidazole, 5% glycerol, 0.5% Triton X-100, 3 mM DTT, 1 mg/mL lysozyme) and lysed using a cell disruptor at 26 KPsi in 4 °C. The cell lysate was spun down at 35 000g for 1 h at 4 °C. The supernatant was filtered on a 0.45 µm filter and loaded onto a 5 mL HisTrap HP column (GE Healthcare). The column was washed with five column volumes of HisTrap binding buffer (50 mM HEPES pH 7.5, 150 mM NaCl, 10 mM imidazole, 3 mM DTT) followed by eluting the protein using a gradient of HisTrap elution buffer (50 mM HEPES pH 7.5, 150 mM NaCl, 1 M imidazole, 3 mM DTT). The human Keap1 Kelch was eluted between 5 and 10% elution buffer and concentrated to 5 mL. The protein was then loaded onto a Superdex 75 16/ 600 column (GE Healthcare), equilibrated with SEC buffer (25 mM HEPES pH 7.5, 150mMNaCl, 1 mM TCEP) with a flow rate at 1 mL/ min, and was eluted at 65 mL. The pure human Keap1 Kelch protein was concentrated to 12 mg/mL and stored at -80 °C.

The mouse Keap1 Kelch domain was eluted between 10 and 15% elution buffer from the HisTrap column. The His-tag of mouse Keap1 Kelch domain was cleaved by adding 500 µL of 1 mg/mL His-tagged human rhinovirus-3C protease to approx. 50 mg of protein and incubated overnight at 4 °C in HisTrap binding buffer. After 16 h, the cleaved mouse Keap1 Kelch domain was purified using "reverse purification" on the HisTrap HP column and concentrated to 5 mL. The cleaved protein was loaded onto a Superdex 75 16/600 column (GE Healthcare), equilibrated with SEC buffer (20 mM Tris-HCl pH 8.3, 20 mM DTT and 10 mM benzamidine) with a flow rate of 1 mL/min, and was eluted at 65 mL. Protein was concentrated to 18 mg/mL for crystallography analysis and stored at -80 °C.

The human and mouse Keap1 Kelch domains were analyzed using sodium dodecyl sulfate polyacrylamide gel electrophoresis for purity, and the concentrations were measured by absorbance (Nanodrop) using molar extinction coefficients calculated based on the amino acid sequence. The exact molecular weights of purified human and mouse Keap1 Kelch were confirmed by ESI-LC-MS.

### Example 3: Keap1-Nrf2 inhibition by the fluorescence polarization (FP) assay

### Aim

To characterize the inhibitory effect of the compounds of the present disclosure on Keap1-Nrf2.

### Materials and Methods

The binding affinities between the fluorescent peptide probe Cy5-Nrf2 (SEQ: Cy5-LDEETGEFL-NH₂) and the human Keap1 Kelch domain were determined as *K*_{d} values by saturation binding experiments, where increasing concentrations of Keap1 (0-300 nM) were added to a fixed concentration of peptide probe (3 nM). The assay was performed in a 1 × HBSTET buffer (10 mM HEPES, 150 mM NaCl, 0.005% Tween20, 3 mM ethylenediaminetetraacetic acid (EDTA), 1 mM TCEP, pH = 7.4) using black flat-bottom 384-well plates (Corning Life Sciences, NY), a volume of 30 µL/well, and a final DMSO concentration of 4% or 8%. The assay plate was spun down to ascertain proper mixing and removal of potential air bubbles and incubated for 10-15 min at room temperature before measuring the FP levels on a Safire2 plate reader (Tecan, Männedorf, Switzerland). The g-factor was adjusted at each experiment so that a series of three blank wells containing probe but no Keap1 Kelch defined the baseline FP value. The Cy5-Nrf2 probe was measured at excitation/emission values of 635:670 nm. The FP values were fitted to the one-site specific binding equation: Y = Bₘₐₓ × X/(*K*_{d} + X), with Bₘₐₓ being the maximal FP value, X the Keap1 Kelch concentration, and Y the variable FP values. The *K*_{d} values were derived from the resulting binding saturation curve as being equal to the Keap1 Kelch concentration, where the curve is half-saturated.

The affinities between nonfluorescent small molecule Keap1 inhibitors and Keap1 Kelch were determined as *K*ᵢ values in a heterologous competition FP binding assay. This was done by adding increasing concentration of test compound (2-fold serial dilutions, 12 concentrations tested per inhibition curve as duplicates) to a fixed concentration of Keap1 Kelch (14 nM) and the Cy5-Nrf2 peptide probe (3 nM) in the same HBSTET buffer and conditions as described above (final DMSO concentrations were 4%). FP values were fitted to the equation Y = Bottom + (Top- Bottom)/[1 + (10^{HillSlope×(log IC50-X)})], where X is the logarithmic value of compound concentration. Hereby, the IC₅₀ value was obtained, which together with the *K*_{d} value and probe and Keap1 Kelch concentrations was used to calculate the theoretical competitive inhibition constant, the *K*ᵢ value.⁴³ *K*ᵢ values are shown as mean ± SEM (standard error of mean) if based on two or more individual measurements.

### Results

Table 1 shows the results of theoretical competitive inhibition constant (*K*ᵢ) for the compounds according to the present disclosure.

**Table 1. Kᵢ values (µM) of compounds.**

| **ID** | **(*K*ᵢ/µM)^{a}** | **ID** | **(*K*ᵢ/µM)^{a}** |
|---|---|---|---|
| K1 | 0.36 ± 0.07 | K27 | 0.035 ± 0.005 |
| K2 | 0.12 ± 0.01 | K28 | 0.017 ± 0.003 |
| K3 | 0.18 ± 0.02 | K29 | 0.014 ± 0.002 |
| K4 | 2.93 ± 0.70 | K30 | 0.024 ± 0.004 |
| K5 | 0.37 ± 0.07 | K31 | 4.0 ± 0.6 |
| K6 | 0.27 ± 0.04 | K32 | 0.59 ± 0.10 |
| K7 | 1.2 ± 0.1 | K33 | 0.065 ± 0.008 |
| K8 | 0.13 ± 0.02 | K34 | 0.016 ± 0.002 |
| K9 | 0.13 ± 0.03 | K35 | 0.091 ± 0.008 |
| K10 | 0.24 ± 0.04 | K36 | 0.036 ± 0.005 |
| K11 | 0.11 ± 0.03 | K37 | 0.13 ± 0.01 |
| K12 | 0.35 ± 0.08 | K38 | 0.17 ± 0.04 |
| K13 | 0.18 ± 0.04 | K39 | 0.18 ± 0.02 |
| K14 | 0.19 ± 0.04 | K40 | 0.013 ± 0.002 |
| K15 | 0.027 ± 0.01 | K41 | 0.014 ± 0.001 |
| K16 | 0.37 ± 0.06 | K42 | 0.032 ± 0.004 |
| K17 | 0.14 ± 0.02 | K43 | 0.015 ± 0.004 |
| K18 | 0.17 ± 0.03 | K44 | 0.043 ± 0.003 |
| K19 | 0.081 ± 0.001 | K45 | 3.6 ± 0.8 |
| K20 | 0.056 ± 0.013 | K46 | 0.93 ± 0.21 |
| K21 | 0.089 ± 0.03 | K47 | 13 ± 2 |
| K22 | 0.13 ± 0.01 | K48 | 13 ± 5 |
| K23 | 16 ± 3 | K49 | 1.3 ± 0.3 |
| K24 | 0.069 ± 0.017 | K50 | 3.6 ± 0.4 |
| K25 | 0.041 ± 0.003 | K51 | 9.4 ± 1 |
| K26 | 0.015 ± 0.002 | K52 | 0.15 ± 0.01 |

### a: The values represent mean ± SEM of at least 3 individual measurements (n ≥3).

### Conclusion

The compounds according to the present disclosure inhibit the Keap1-Nrf2 interaction.

### Example 4: X-ray crystallography

### Aim

To show direct target binding to the Keap1 Kelch domain and determine the binding mode of compounds.

### Materials and Methods

Crystals of the apo mouse Keap1 Kelch domain were obtained within 4 days through crystallization trials in a sitting drop with 100 nL Crystal Screen HT condition - F3 (Hampton Research, USA) and 100 nL protein solution (12.93 mg/mL) using Crystal Gryphon Robot (ARI, USA) in a Swissci 3 Well 96-Midi Crystallization Plate (Hampton Research, USA) at 293 K. Best apo crystals were grown within 2 days by vapor-diffusion with 1 uL of screening solution (modified Crystal Screen HT condition - F3: 0.1 M sodium citrate pH 5.6, 0.5 M lithium sulfate, and 0.7-0.9 M ammonium sulfate) and 1 uL of protein solution (12.93 mg/mL) using the hanging drop method at 293 K. For obtaining protein-compound complexes, crystals of mouse Keap1 Kelch domain were soaked for 2-16 hours (0.5-10 mM; 10% DMSO) with compounds, in a solution containing 0.1 M Na citrate pH 5.6, 0.5-0.6 M ammonium sulphate, 0.9-1.1 M lithium sulphate, and then harvested in liquid nitrogen for X-ray diffraction. Data for mouse Keap1 Kelch domain in complex with compounds were collected from synchrotron beamline facilities (Diamond Light Source, Oxfordshire and BioMAX at MAXIV, Lund, respectively). Diffraction images were integrated, scaled, and merged using autoprocessed beamline tools, while in some cases the data were reprocessed and scaled using XDS. The structures were solved using PHASER with PDB ID 6ZF4 as the search model for molecular replacement. Restraints for the compounds were prepared using the AceDRG followed by Model building and refinement using COOT and Phenix.refine.³⁷ Figure were prepared using PyMOL (The PyMOL Molecular Graphics System, Version 2.3.4 Schrödinger, LLC).

### Results

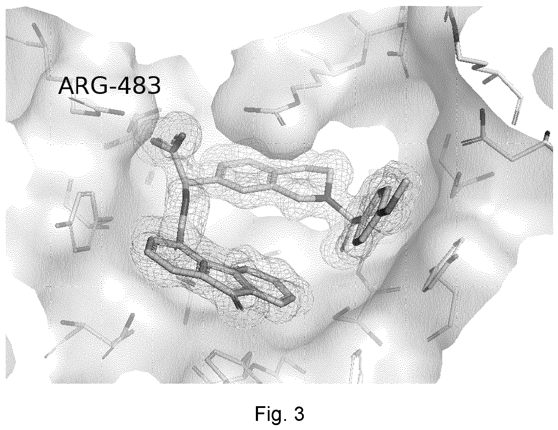

Prior to proceeding with further optimization, an X-ray co-crystal structure of THIQ compound K2 was obtained **(****Figure 3****).** It verified that the conformational restriction design gave THIQ compounds that bind directly into the Keap1 Kelch binding pocket. The carboxylic acid moiety binds Arg483 in P1, the phenyl ring is located in P3, the fluorenone moiety occupies P4/P5, and the phenylsulfonamide moiety is found in the P2/P3/P5 region.

### Conclusion

Compound **K2** was verified to bind in the Neh2-binding pocket of the Keap1 Kelch domain, as also shown by the FP data. Hence, analogues of K2 are likewise anticipated to bind the Keap1 Kelch domain and in a similar binding mode.

### References

1. Bach, A., Targeting Oxidative Stress in Stroke. In Neuroprotective Therapy for Stroke and Ischemic Disease, Lapchak, P. A.; Zhang, J. H., Eds. Springer: 2017; pp 203-250.
2. Casas, A. I.; Nogales, C.; Mucke, H. A. M.; Petraina, A.; Cuadrado, A.; Rojo, A. I.; Ghezzi, P.; Jaquet, V.; Augsburger, F.; Dufrasne, F.; Soubhye, J.; Deshwal, S.; Di Sante, M.; Kaludercic, N.; Di Lisa, F.; Schmidt, H., On the Clinical Pharmacology of Reactive Oxygen Species. Pharmacol Rev 2020, 72 (4), 801-828.
3. Baird, L.; Yamamoto, M., The Molecular Mechanisms Regulating the KEAP1-NRF2 Pathway. Mol Cell Biol 2020, 40 (13).
4. Pallesen, J. S.; Tran, K. T.; Bach, A., Non-covalent Small-Molecule Kelch-like ECH-Associated Protein 1-Nuclear Factor Erythroid 2-Related Factor 2 (Keap1-Nrf2) Inhibitors and Their Potential for Targeting Central Nervous System Diseases. J Med Chem 2018, 61 (18), 8088-8103.
5. Horie, Y.; Suzuki, T.; Inoue, J.; Iso, T.; Wells, G.; Moore, T. W.; Mizushima, T.; Dinkova-Kostova, A. T.; Kasai, T.; Kamei, T.; Koshiba, S.; Yamamoto, M., Molecular basis for the disruption of Keap1-Nrf2 interaction via Hinge & Latch mechanism. Commun Biol 2021, 4 (1), 576.
6. Cuadrado, A.; Rojo, A. I.; Wells, G.; Hayes, J. D.; Cousin, S. P.; Rumsey, W. L.; Attucks, O. C.; Franklin, S.; Levonen, A. L.; Kensler, T. W.; Dinkova-Kostova, A. T., Therapeutic targeting of the NRF2 and KEAP1 partnership in chronic diseases. Nat Rev Drug Discov 2019, 18 (4), 295-317.
7. Barreca, M.; Qin, Y.; Cadot, M. E. H.; Barraja, P.; Bach, A., Advances in developing noncovalent small molecules targeting Keap1. Drug Discov Today 2023, 28 (12), 103800.
8. Magesh, S.; Chen, Y.; Hu, L., Small molecule modulators of Keap1-Nrf2-ARE pathway as potential preventive and therapeutic agents. Med Res Rev 2012, 32 (4), 687-726.
9. Lu, M. C.; Ji, J. A.; Jiang, Z. Y.; You, Q. D., The Keap1-Nrf2-ARE Pathway As a Potential Preventive and Therapeutic Target: An Update. Med Res Rev 2016, 36 (5), 924-963.
10. Tu, J.; Zhang, X.; Zhu, Y.; Dai, Y.; Li, N.; Yang, F.; Zhang, Q.; Brann, D. W.; Wang, R., Cell-Permeable Peptide Targeting the Nrf2-Keap1 Interaction: A Potential Novel Therapy for Global Cerebral Ischemia. J Neurosci 2015, 35 (44), 14727-39.
11. Zhao, X.; Sun, G.; Zhang, J.; Ting, S. M.; Gonzales, N.; Aronowski, J., Dimethyl Fumarate Protects Brain From Damage Produced by Intracerebral Hemorrhage by Mechanism Involving Nrf2. Stroke 2015, 46 (7), 1923-1928.
12. Shih, A. Y.; Li, P.; Murphy, T. H., A small-molecule-inducible Nrf2-mediated antioxidant response provides effective prophylaxis against cerebral ischemia in vivo. Journal of Neuroscience 2005, 25 (44), 10321-10335.
13. Bach, A., Targeting Oxidative Stress in Stroke. In Neuroprotective Therapy for Stroke and Ischemic Disease, Lapchak, P. Z. L., Ed. Springer Series in Translational Stroke Research, 2017.
14. Alfieri, A.; Srivastava, S.; Siow, R. C.; Modo, M.; Fraser, P. A.; Mann, G. E., Targeting the Nrf2-Keap1 antioxidant defence pathway for neurovascular protection in stroke. J Physiol 2011, 589 (17), 4125-36.
15. Zhao, J.; Redell, J. B.; Moore, A. N.; Dash, P. K., A novel strategy to activate cytoprotective genes in the injured brain. Biochemical and Biophysical Research Communications 2011, 407 (3), 501-506.
16. Hui, Q. Y.; Karlstetter, M.; Xu, Z. H.; Yang, J.; Zhou, L. L.; Eilken, H. M.; Terjung, C.; Cho, H.; Gong, J. S.; Lai, M. J.; Nassar, K.; Duh, E. J., Inhibition of the Keap1-Nrf2 protein-protein interaction protects retinal cells and ameliorates retinal ischemia-reperfusion injury. Free Radical Bio Med 2020, 146, 181-188.
17. Davies, D. A.; Adlimoghaddam, A.; Albensi, B. C., Role of Nrf2 in Synaptic Plasticity and Memory in Alzheimer's Disease. Cells 2021, 10 (8).
18. Sun, Y.; Huang, J. X.; Chen, Y. F.; Shang, H.; Zhang, W. N.; Yu, J. Q.; He, L.; Xing, C. G.; Zhuang, C. L., Direct inhibition of Keap1-Nrf2 Protein-Protein interaction as a potential therapeutic strategy for Alzheimer's disease. Bioorg Chem 2020, 103*.*
19. Johnson, J. A.; Johnson, D. A.; Kraft, A. D.; Calkins, M. J.; Jakel, R. J.; Vargas, M. R.; Chen, P. C., The Nrf2-ARE pathway: an indicator and modulator of oxidative stress in neurodegeneration. Ann N Y Acad Sci 2008, 1147, 61-9.
20. Davies, T. G.; Wixted, W. E.; Coyle, J. E.; Griffiths-Jones, C.; Hearn, K.; McMenamin, R.; Norton, D.; Rich, S. J.; Richardson, C.; Saxty, G.; Willems, H. M.; Woolford, A. J.; Cottom, J. E.; Kou, J. P.; Yonchuk, J. G.; Feldser, H. G.; Sanchez, Y.; Foley, J. P.; Bolognese, B. J.; Logan, G.; Podolin, P. L.; Yan, H.; Callahan, J. F.; Heightman, T. D.; Kerns, J. K., Monoacidic Inhibitors of the Kelch-like ECH-Associated Protein 1: Nuclear Factor Erythroid 2-Related Factor 2 (KEAP1:NRF2) Protein-Protein Interaction with High Cell Potency Identified by Fragment-Based Discovery. J Med Chem 2016**.**
21. Olagnier, D.; Farahani, E.; Thyrsted, J.; Blay-Cadanet, J.; Herengt, A.; Idorn, M.; Hait, A.; Hernaez, B.; Knudsen, A.; Iversen, M. B.; Schilling, M.; Jorgensen, S. E.; Thomsen, M.; Reinert, L. S.; Lappe, M.; Hoang, H. D.; Gilchrist, V. H.; Hansen, A. L.; Ottosen, R.; Nielsen, C. G.; Moller, C.; van der Horst, D.; Peri, S.; Balachandran, S.; Huang, J.; Jakobsen, M.; Svenningsen, E. B.; Poulsen, T. B.; Bartsch, L.; Thielke, A. L.; Luo, Y.; Alain, T.; Rehwinkel, J.; Alcami, A.; Hiscott, J.; Mogensen, T. H.; Paludan, S. R.; Holm, C. K., SARS-CoV2-mediated suppression of NRF2-signaling reveals potent antiviral and anti-inflammatory activity of 4-octyl-itaconate and dimethyl fumarate. Nat Commun 2020, 11 (1), 4938.
22. Yan, J.; Li, Y.; Ding, L.; Hou, R.; Xing, C.; Jiang, C.; Miao, Z.; Zhuang, C., Fragment-Based Discovery of Azocyclic Alkyl Naphthalenesulfonamides as Keap1-Nrf2 Inhibitors for Acute Lung Injury Treatment. J Med Chem 2023, 66 (12), 8267-8280.
23. Seedorf, K.; Weber, C.; Vinson, C.; Berger, S.; Vuillard, L. M.; Kiss, A.; Creusot, S.; Broux, O.; Geant, A.; Ilic, C.; Lemaitre, K.; Richard, J.; Hammoutene, A.; Mahieux, J.; Martiny, V.; Durand, D.; Melchiore, F.; Nyerges, M.; Paradis, V.; Provost, N.; Duvivier, V.; Delerive, P., Selective disruption of NRF2-KEAP1 interaction leads to NASH resolution and reduction of liver fibrosis in mice. JHEP Rep 2023, 5 (4), 100651.
24. Hammoutene, A.; Laouirem, S.; Albuquerque, M.; Colnot, N.; Brzustowski, A.; Valla, D.; Provost, N.; Delerive, P.; Paradis, V., A new NRF2 activator for the treatment of human metabolic dysfunction associated fatty liver disease. JHEP Reports 2023**.**
25. Hassanein, E. H. M.; Ibrahim, I. M.; Abd-alhameed, E. K.; Sharawi, Z. W.; Jaber, F. A.; Althagafy, H. S., Nrf2/HO-1 as a therapeutic target in renal fibrosis. Life Sciences 2023, 334, 122209.
26. Kaseda, S.; Sannomiya, Y.; Horizono, J.; Kuwazuru, J.; Suico, M. A.; Ogi, S.; Sasaki, R.; Sunamoto, H.; Fukiya, H.; Nishiyama, H.; Kamura, M.; Niinou, S.; Koyama, Y.; Nara, F.; Shuto, T.; Onuma, K.; Kai, H., Novel Keap1-Nrf2 Protein-Protein Interaction Inhibitor UBE-1099 Ameliorates Progressive Phenotype in Alport Syndrome Mouse Model. Kidney360 2022, 3 (4), 687-699.
27. Ogawa, T.; Ishitsuka, Y., The Role of KEAP1-NRF2 System in Atopic Dermatitis and Psoriasis. Antioxidants (Basel) 2022, 11 (7).
28. Manda, G.; Milanesi, E.; Gene, S.; Niculite, C. M.; Neagoe, I. V.; Tastan, B.; Dragnea, E. M.; Cuadrado, A., Pros and cons of NRF2 activation as adjunctive therapy in rheumatoid arthritis. Free Radic Biol Med 2022, 190, 179-201.
29. Lu, M. C.; Ji, J. A.; Jiang, Y. L.; Chen, Z. Y.; Yuan, Z. W.; You, Q. D.; Jiang, Z. Y., An inhibitor of the Keap1-Nrf2 protein-protein interaction protects NCM460 colonic cells and alleviates experimental colitis. Sci Rep-Uk 2016, 6.
30. Lu, M. C.; Zhang, X.; Wu, F.; Tan, S. J.; Zhao, J.; You, Q. D.; Jiang, Z. Y., Discovery of a Potent Kelch-Like ECH-Associated Protein 1-Nuclear Factor Erythroid 2-Related Factor 2 (Keap1-Nrf2) Protein-Protein Interaction Inhibitor with Natural Proline Structure as a Cytoprotective Agent against Acetaminophen-Induced Hepatotoxicity. J Med Chem 2019, 62 (14), 6796-6813.
31. Linker, R. A.; Lee, D. H.; Ryan, S.; van Dam, A. M.; Conrad, R.; Bista, P.; Zeng, W.; Hronowsky, X.; Buko, A.; Chollate, S.; Ellrichmann, G.; Bruck, W.; Dawson, K.; Goelz, S.; Wiese, S.; Scannevin, R. H.; Lukashev, M.; Gold, R., Fumaric acid esters exert neuroprotective effects in neuroinflammation via activation of the Nrf2 antioxidant pathway. Brain 2011, 134, 678-692.
32. Prosperini, L.; Pontecorvo, S., Dimethyl fumarate in the management of multiple sclerosis: appropriate patient selection and special considerations. Therapeutics and Clinical Risk Management 2016, 12, 339-350.
33. Blewett, M. M.; Xie, J. J.; Zaro, B. W.; Backus, K. M.; Altman, A.; Teijaro, J. R.; Cravatt, B. F., Chemical proteomic map of dimethyl fumarate-sensitive cysteines in primary human T cells. Science Signaling 2016, 9 (445).
34. Zhou, H.; Wang, Y.; You, Q.; Jiang, Z., Recent progress in the development of small molecule Nrf2 activators: a patent review (2017-present). Expert opinion on therapeutic patents 2020, 30 (3), 209-225.
35. Tran, K. T.; Pallesen, J. S.; Solbak, S. M. QJ.; Narayanan, D.; Baig, A.; Zang, J.; Aguayo-Orozco, A.; Carmona, R. M. C.; Garcia, A. D.; Bach, A., A Comparative Assessment Study of Known Small-Molecule Keap1-Nrf2 Protein-Protein Interaction Inhibitors: Chemical Synthesis, Binding Properties, and Cellular Activity. J Med Chem 2019, 62 (17), 8028-8052.
36. Pallesen, J. S.; Narayanan, D.; Tran, K. T.; Solbak, S. M. QJ.; Marseglia, G.; Sorensen, L. M. E.; Hoj, L. J.; Munafo, F.; Carmona, R. M. C.; Garcia, A. D.; Desu, H. L.; Brambilla, R.; Johansen, T. N.; Popowicz, G. M.; Sattler, M.; Gajhede, M.; Bach, A., Deconstructing Noncovalent Kelch-like ECH-Associated Protein 1 (Keap1) Inhibitors into Fragments to Reconstruct New Potent Compounds. J Med Chem 2021, 64 (8), 4623-4661.
37. Narayanan, D.; Tran, K. T.; Pallesen, J. S.; Solbak, S. M. QJ.; Qin, Y.; Mukminova, E.; Luchini, M.; Vasilyeva, K. O.; Gonzalez Chichon, D.; Goutsiou, G.; Poulsen, C.; Haapanen, N.; Popowicz, G. M.; Sattler, M.; Olagnier, D.; Gajhede, M.; Bach, A., Development of Noncovalent Small-Molecule Keap1-Nrf2 Inhibitors by Fragment-Based Drug Discovery. J Med Chem 2022, 65 (21), 14481-14526.
38. Heightman, T. D.; Callahan, J. F.; Chiarparin, E.; Coyle, J. E.; Griffiths-Jones, C.; Lakdawala, A. S.; McMenamin, R.; Mortenson, P. N.; Norton, D.; Peakman, T. M.; Rich, S. J.; Richardson, C.; Rumsey, W. L.; Sanchez, Y.; Saxty, G.; Willems, H. M. G.; Wolfe, L., 3rd; Woolford, A. J.; Wu, Z.; Yan, H.; Kerns, J. K.; Davies, T. G., Structure-Activity and Structure-Conformation Relationships of Aryl Propionic Acid Inhibitors of the Kelch-like ECH-Associated Protein 1/Nuclear Factor Erythroid 2-Related Factor 2 (KEAP1/NRF2) Protein-Protein Interaction. J Med Chem 2019, 62 (9), 4683-4702.
39. Erlanson, D. A.; Fesik, S. W.; Hubbard, R. E.; Jahnke, W.; Jhoti, H., Twenty years on: the impact of fragments on drug discovery. Nat Rev Drug Discov 2016, 15 (9), 605-19.
40. Kirsch, P.; Hartman, A. M.; Hirsch, A. K. H.; Empting, M., Concepts and Core Principles of Fragment-Based Drug Design. Molecules 2019, 24 (23).
41. Norton, D.; Bonnette, W. G.; Callahan, J. F.; Carr, M. G.; Griffiths-Jones, C. M.; Heightman, T. D.; Kerns, J. K.; Nie, H.; Rich, S. J.; Richardson, C.; Rumsey, W.; Sanchez, Y.; Verdonk, M. L.; Willems, H. M. G.; Wixted, W. E.; Wolfe, L., 3rd; Woolford, A. J.; Wu, Z.; Davies, T. G., Fragment-Guided Discovery of Pyrazole Carboxylic Acid Inhibitors of the Kelch-like ECH-Associated Protein 1: Nuclear Factor Erythroid 2 Related Factor 2 (KEAP1:NRF2) Protein-Protein Interaction. J Med Chem 2021, 64 (21), 15949-15972.
42. Rees, D. C.; Congreve, M.; Murray, C. W.; Carr, R., Fragment-based lead discovery. Nat Rev Drug Discov 2004, 3 (8), 660-72.
43. Lu, M.; Zhang, X.; Zhao, J.; You, Q.; Jiang, Z., A hydrogen peroxide responsive prodrug of Keap1-Nrf2 inhibitor for improving oral absorption and selective activation in inflammatory conditions. Redox Biol 2020, 34, 101565.
44. Nikolovska-Coleska, Z.; Wang, R.; Fang, X.; Pan, H.; Tomita, Y.; Li, P.; Roller, P. P.; Krajewski, K.; Saito, N. G.; Stuckey, J. A.; Wang, S., Development and optimization of a binding assay for the XIAP BIR3 domain using fluorescence polarization. Anal Biochem 2004, 332 (2), 261-273.

## Claims

1. A compound according to formula (I): 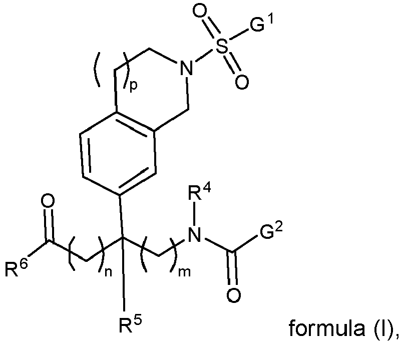 or a pharmaceutically acceptable salt thereof, wherein
G¹ is according to 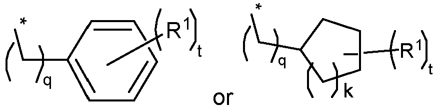
R¹ is in each instance individually selected from -H, -F, -Cl, -Br, -I, -CN, -O-(C₁-C₃ alkyl), -CF₃, -OCF₃, -N(R^{1a})(R^{1b}),
C₁-C₃ alkyl, -C(=O)O-(C₁-C₃ alkyl), or two R¹ groups are linked together to form a carbocycle or heterocycle;
R^{1a} and R^{1b} are each independently a C₁-C₃ alkyl;
G² is according to 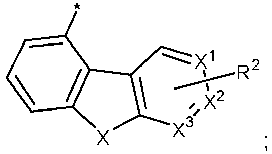
R² is H, -F, -Cl, -CH₃, or -O-CH₃;
X¹, X², X³ are each individually selected from CH, or N; provided that at most only one of X¹, X² and X³ is N;
X is O, S, 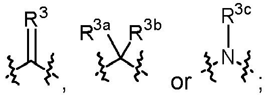
R³ is (=O), or (=S);
R^{3a} is H, halogen, -O-(C₁-C₃ alkyl), -O-CF₃, -S-(C₁ alkyl) or -C₁-C₃ alkyl; R^{3b} is H, -OH, -O-(C₁-C₃ alkyl), -O-CF₃, halogen, -S-(C₁ alkyl), or -C₁-C₃ alkyl; or R^{3a} and R^{3b} are linked together to form a carbocycle or heterocycle;
R^{3c} is H, C₁-C₃ alkyl, or -CF₃;
R⁴ is H, or C₁ alkyl;
R⁵ is H, or F;
R⁶ is -OH, -O-C₁-C₁₀ alkyl, -O-C₃-C₁₀ cycloalkyl, a heterocycle, or a -O-C₅-C₁₂ aryl;
n, m, p, and q are integers each individually and independently selected from 0 or 1;
k is an integer selected from 0 to 2
t is an integer selected from 0 to 4.

2. The compound according claim 1, wherein R⁴ is H.

3. The compound according to claim 1, wherein R⁵ is H.

4. The compound according to any one of claims 1 to 3, wherein X is 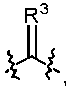 wherein R³ is (=O), or (=S).

5. The compound according any one of claims 1 to 3, wherein X is wherein X is 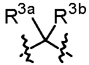 wherein R^{3a} is H, halogen, -O-(C₁-C₃ alkyl), -O-CF₃, -S-(C₁ alkyl) or - C₁-C₃ alkyl; and R^{3b} is H, -OH, -O-(C₁-C₃ alkyl), -O-CF₃, halogen, -S-(C₁ alkyl), or -C₁-C₃ alkyl.

6. The compound according any one of claims 1 to 3, wherein X is wherein X is 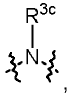 wherein R^{3c} is H, C₁-C₃ alkyl, or -CF₃.

7. The compound according to any one of the preceding claims, wherein X is selected from any one of the group consisting of:

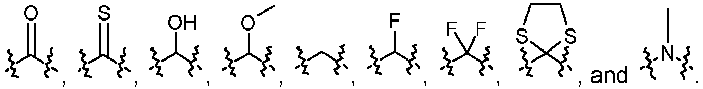

8. The compound according to any one of claims 1 to 7, wherein n is 0, and/or m is 0.

9. The compound according to any one of claims 1 to 7, wherein q is 0.

10. The compound according to any one of claims 1 to 7, wherein p is 1.

11. The compound according to any one of the preceding claims, wherein G¹ is according to any one of: 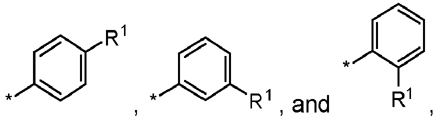 wherein
R¹ is selected from -H, -F, -Cl, -Br, -I, -CN, -O-(C₁-C₃ alkyl), -CF₃, -OCF₃, - N(R^{1a})(R^{1b}), C₁-C₃ alkyl, -C(=O)O-(C₁-C₃ alkyl);
R^{1a} and R^{1b} are each independently a C₁-C₃ alkyl.

12. The compound according to any one of the preceding claims, wherein G¹ is according to any one of: 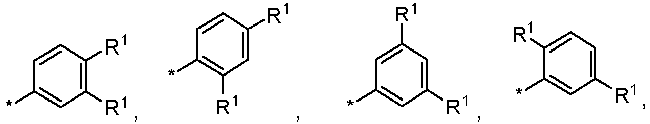 and 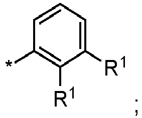 wherein each instance of R¹ is individually and independently selected from , -F, -Cl, -Br, -I, -CN, -O-(C₁-C₃ alkyl), -CF₃, -OCF₃, -N(R^{1a})(R^{1b}), C₁-C₃ alkyl, -C(=O)O-(C₁-C₃ alkyl), or two R¹ groups are linked together to form a carbocycle or heterocycle.

13. The compound according to any one of the preceding claims, wherein R⁶ is -OH.

14. A composition comprising the compound according to any one of the preceding claims, or a pharmaceutically acceptable salt thereof, and a and a pharmaceutically acceptable carrier or excipient.

15. A compound according to any one of claims 1 to 13, or a composition according to claim 14, for use for use in treating, ameliorating and/or preventing a disease where reactive oxygen species (ROS) and/or inflammation is involved.
